# EUROPEAN PATENT SPECIFICATION

(11) **EP 1 963 337 B1**
(45) Date of publication and mention of the grant of the patent: **01.09.2010**
(21) Application number: 06831522.5
(22) Date of filing: 19.12.2006
(51) Int. Cl.: C07D 495/04, A61K 31/4365, A61P 25/00

(54) **THIENO[2,3-b]PYRIDINE DERIVATIVES**
THIENO[2,3-b]PYRIDIN DERIVATE
COMPOSÉS DE THIENO[2,3-b]PYRIMIDINE

(30) Priority: 20.12.2005 HU 0501171; 18.12.2006 HU 0600920
(43) Date of publication of application: 03.09.2008
(73) Proprietor: Richter Gedeon Nyrt., 1103 Budapest (HU)
(72) Inventor: NÓGRÁDI, Katalin, H-1182 Budapest (HU); WÁGNER, Gábor, H-1107 Budapest (HU); KESERÜ, György, H-2089 Telki (HU); BIELIK, Attila, H-2377 Örkény (HU); GÁTI, Tamás, H-1114 Budapest (HU); HÁDA, Viktor, H-1108 Budapest (HU); KÓTI, János, H-4440 Tiszavasvári (HU); GÁL, Krisztina, H-1193 Budapest (HU); VASTAG, Mónika, H-1025 Budapest (HU); BOBOK, Amrita, Ágnes, H-1025 Budapest (HU)
(74) Representative: HOFFMANN EITLE
(86) International application number: PCT/HU2006/000123
(87) International publication number: WO 2007/072095

(56) References cited:
- WO-A-01/32632
- WO-A-02/06288

## Description

### FIELD OF THE INVENTION

The present invention relates to new mGluR1 and mGluR5 receptor subtype preferring ligands of formula (I) and/or salts and/or hydrates and/or solvates thereof, to the processes for their preparation, to pharmaceutical compositions containing these compounds and to their use in therapy and/or prevention of a condition which requires modulation of mGluR1 and mGluR5 receptors.

### BACKGROUND OF THE INVENTION

A major excitatory neurotransmitter in the mammalian central nervous system (CNS) is the glutamate molecule, which binds to neurons, thereby activating cell surface receptors. These receptors can be divided into two major classes, ionotropic and metabotropic glutamate receptors, based on the structural features of the receptor proteins, the means by which the receptors transduce signals into the cell, and pharmacological profiles.

The metabotropic glutamate receptors (mGluRs) are G protein-coupled receptors that activate a variety of intracellular second messenger systems following the binding of glutamate. Activation of mGluRs in intact mammalian neurons elicits one or more of the following responses: activation of phospholipase C; increases in phosphoinositide (PI) hydrolysis; intracellular calcium release; activation of phospholipase D; activation or inhibition of adenyl cyclase; increases or decreases in the formation of cyclic adenosine monophosphate (cAMP); activation of guanylyl cyclase; increases in the formation of cyclic guanosine monophosphate (cGMP); activation of phospholipase A2; increases in arachidonic acid release; and increases or decreases in the activity of voltage- and ligand-gated ion channels. (Trends Pharmacol. Sci., 1993, 14, 13; Neurochem. Int., 1994, 24, 439; Neuropharmacology, 1995, 34, 1; Prog. Neurobiol., 1999, 59, 55; Berl. Psychopharmacology 2005, 179, 4).

Eight distinct mGluR subtypes, termed mGluRl through mGluR8, have been identified by molecular cloning (Neuron, 1994, 13, 1031; Neuropharmacology, 1995, 34, 1; J. Med. Chem., 1995, 38, 1417). Further receptor diversity occurs via expression of alternatively spliced forms of certain mGluR subtypes (PNAS, 1992, 89, 10331; BBRC, 1994, 199, 1136; J. Neurosci., 1995, 15, 3970).

Metabotropic glutamate receptor subtypes may be subdivided into three groups, Group I, Group II, and Group III mGluRs, based on amino acid sequence homology, the second messenger systems utilized by the receptors, and by their pharmacological characteristics. Group I mGluR comprises mGluRl, mGluR5 and their alternatively spliced variants.

Attempts at elucidating the physiological roles of Group I mGluRs suggest that activation of these receptors elicits neuronal excitation. Evidence indicates that this excitation is due to direct activation of postsynaptic mGluRs, but it also has been suggested that activation of presynaptic mGluRs occurs, resulting in increased neurotransmitter release (Trends Pharmacol. Sci., 1992, 15, 92; Neurochem. Int., 1994, 24, 439; Neuropharmacology, 1995, 34, 1; Trends Pharmacol. Sci., 1994, 15, 33).

Metabotropic glutamate receptors have been implicated in a number of normal processes in the mammalian CNS. Activation of mGluRs has been shown to be required for induction of hippocampal long-term potentiation and cerebellar long-term depression (Nature, 1993, 363, 347; Nature, 1994, 368, 740; Cell, 1994, 79, 365; Cell, 1994, 79, 377). A role for mGluR activation in nociception and analgesia also has been demonstrated (Neuroreport, 1993, 4, 879; Brain Res., 1999, 871, 223).

Group I metabotropic glutamate receptors and mGluR5 in particular, have been suggested to play roles in a variety of pathophysiological processes and disorders affecting the CNS. These include stroke, head trauma, anoxic and ischemic injuries, hypoglycemia, epilepsy, neurodegenerative disorders such as Alzheimer's disease, acute and chronic pain, substance abuse and withdrawal, obesity and gastroesophageal reflux disease (GERD) *(*Schlepp et al., Trends Pharmacol. Sci. 1993, 14:13*;* Cunningham et al., Life Sci. 1994, 54:135*;* Hollman et al., Ann. Rev. Neurosci. 1994, 17:31*;* Pin et al., Neuropharmacology 1995, 34:1*;* Knopfel et al., J. Med. Chem. 1995, 38:1417*;* Spooren et al., Trends Pharmacol. Sci. 2001, 22:331*;* Gasparini et al. Curr. Opin. Pharmacol. 2002, 2:43*;* Neugebauer Pain 2002, 98:1*,* Slassi et al., Curr Top Med Chem. 2005; 5(9):897-911*).* MGluR5-selective compounds such as 2-methyl-6-(phenylethynyl)-pyridine ("MPEP") are effective in animal models of mood disorders, including anxiety and depression *(*Spooren et al., J. Pharmacol. Exp. Ther. 2000, 295:1267*;* Tatarczynska et al.. Br. J. Pharmacol. 2001, 132:1423*;* Klodzynska et al., Pol., J. Pharmacol, 2001, 132:1423*).* Much of the pathology in these conditions is thought to be due to excessive glutamate-induced excitation of CNS neurons. As Group I mGluRs appear to increase glutamate-mediated neuronal excitation via postsynaptic mechanisms and enhanced presynaptic glutamate release, their activation probably contributes to the pathology. Therefore, selective antagonists of Group I mGluR receptors could be therapeutically beneficial, especially as neuroprotective agents, analgesics or anticonvulsants.

Much of the pathology in these conditions is thought to be due to excessive glutamate-induced excitation of CNS neurons. As Group I mGluRs (mGluR1 and mGluRs5) appear to increase glutamate-mediated neuronal excitation via postsynaptic mechanisms and enhanced presynaptic glutamate release, their activation probably contributes to the pathology. Accordingly, selective antagonists of Group I mGluR receptors could be therapeutically beneficial, specifically as neuroprotective agents, analgesics or anticonvulsants.

International Patent Application WO 9739000 describes novel thienopyridinesulfonamide derivatives useful for inhibiting the binding of an endothelin peptide to ETA or ETB and their use for the treatment of hypertension, cardiovascular disease, asthma, ocular disease, renal failure, wound healing, endotoxic shock, immediate type hypersensitivity and hemorrhagic shock.

Japanese Patent JP 07076586 describes furopyridines and thienopyridines as bone absorption inhibitors for the treatment of osteoporosis.

Thienopyridine derivatives are useful as hematinics, antitumor agents and immunostimulants, as described in JP 07053562 patent application.

According to E. Zeinab et al. (Arch. Pharm, 1992, 325(5), 301) thienopyridine and thienopyrimidine derivatives were synthesized and their mycotoxin inhibitor activities were evaluated. Some of the compounds inhibit the production of mycotoxins and fungal growth.

International patent application WO 02/087584 describes thienopyridin sulfone derivative as aldose reductase inhibitors in formulation also containing cyclooxygenase-2 inhibitors.

WO 01/32632 discloses thieno[2,3-d]pyrimidine derivatives useful for treating and/or preventing conditions requiring the modulation of mGlu receptors.

The compounds mentioned in the above publications are not declared or even not suggested having activity on the mGluR receptors.

### SUMMARY OF THE INVENTION

The present invention relates to new mGluRs1 and mGluR5 receptor subtype preferring ligands of formula (I): 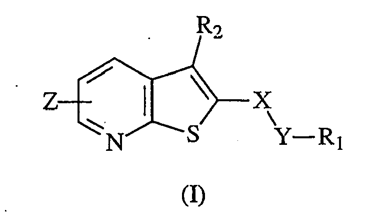 wherein
X represents a group selected from SO, SO₂;
Y represents a group selected from (CH₂)ₙ, NH, NHCH₂;
n is an integer of 0 to 1;
Z is H or monosubstituted by alkyl, nitro, halogen, alkoxy, trifluoromethyl, cyano, amino, alkylamino, dialkylamino, aminomethyl, alkylaminomethyl, dialkylaminomethyl, hydroxyl, alkylsulfonylamino;
R₁ is an optionally substituted alkyl, cycloalkyl, phenyl, biphenyl, heterocyclyl;
R₂ is an optionally substituted phenyl, heterocyclyl, or
NR₃R₄ group wherein R₃ and R₄ are independently selected from the group of hydrogen and an optionally substituted alkyl, or R₃ and R₄ together with the N atom to which they are attached form an optionally substituted C₅₋₇ heterocyclyl group, containing one or more heteroatom(s), or
NH-CO-NR₅R₆ group, wherein R₅ and R₆ are independently selected from the group of hydrogen and an optionally substituted alkyl, or R₅ and R₆ together with the N atom to which they are attached form an optionally substituted C₅₋₇ heterocyclyl group, containing one or more heteroatom(s); and/or hydrates and/or solvates and/or pharmaceutically acceptable salts thereof formed with acids or bases.

Another aspect of the present invention provides processes for the synthesis of compounds of formula (I).

A further aspect of the present invention relates to the intermediates of the preparation process.

A further aspect of the present invention provides pharmaceutical compositions containing a therapeutically effective amount of a compound of formula (I) and/or enantiomers and/or racemates and/or diastereomers and/or salts and/or hydrates or solvates thereof as active ingredient and pharmaceutically acceptable diluents, excipients and/or inert carriers.

A further aspect of the present invention provides the compounds of formula (I) for the prevention and/or treatment of mgluR5 receptor mediated disorders, particularly neurological disorders, psychiatric disorders, acute and chronic pain and neuromuscular dysfunction of the lower urinary tract and gastrointestinal disorders.

A further aspect of the present invention provides the use of a compound of formula (I) for the manufacture of a medicament for the prevention and/or treatment of mGluR5 receptor-mediated disorders, particularly neurological disorders, psychiatric disorders, acute and chronic pain and neuromuscular dysfunction of the lower urinary tract and gastrointestinal disorders.

### DETAILED DESCRIPTION OF THE INVENTION

The present invention relates to new mGluRs1 and mGluR5 receptor subtype preferring ligands of formula (I): 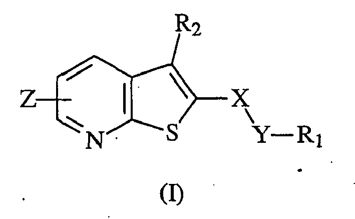 wherein
X represents a group selected from SO, SO₂;
Y represents a group selected from (CH₂)ₙ, NH, NHCH₂;
n is an integer of 0 to 1;
Z is H or monosubstituted by alkyl, nitro, halogen, *alk*oxy, trifluoromethyl, cyano, amino, alkylamino, dialkylamino, aminomethyl, alkylaminomethyl, dialkylaminomethyl, hydroxyl, alkylsulfonylamino;
R₁ is an optionally substituted alkyl, cycloalkyl, phenyl, biphenyl, heterocyclyl;
R₂ is an optionally substituted phenyl, heterocyclyl, or
NR₃R₄ group wherein R₃ and R₄ are independently selected from the group of hydrogen and an optionally substituted alkyl, or R₃ and R₄ together with the N atom to which they are attached form an optionally substituted C₅₋₇ heterocyclyl group, containing one or more heteroatom(s), or
NH-CO-NR₅R₆ group, wherein R₅ and R₆ are independently selected from the group of hydrogen and an optionally substituted alkyl, or R₅ and R₆ together with the N atom to which they are attached form an optionally substituted C₅₋₇ heterocyclyl group, containing one or more heteroatom(s) and/or hydrates and/or solvates and/or pharmaceutically acceptable salts thereof formed with acids or bases.

A more preferred embodiment of the invention is a compound of formula (I) 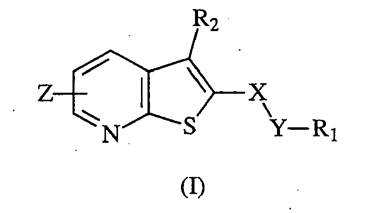 wherein
X represents a group selected from SO, SO₂;
Y represents a group selected from (CH₂)ₙ, NH, NHCH₂;
n is an integer of 0 to 1;
Z is H or monosubstituted by alkyl, nitro, halogen, *alk*oxy, trifluoromethyl, cyano, amino, alkylamino, dialkylamino, aminomethyl, alkylaminomethyl, dialkylaminomethyl, hydroxyl, alkylsulfonylamino;
R₁ is alkyl or C₃₋₁₀ cycloalkyl group optionally substituted with one or more substituent(s) selected from alkoxy, trifluoromethyl, amino, alkylamino, dialkylamino, aminomethyl, alkylaminomethyl, dialkylaminomethyl, acylamino, cyano, halogen, or
phenyl or biphenyl optionally substituted with one or more substituent(s) selected from alkyl, methoxy, trifluoromethyl, amino, alkylamino, dialkylamino, aminomethyl, alkylaminomethyl, dialkylaminomethyl, acylamino, alkylsulfonyl, alkylsulfonylamino, cyano, halogen, or
saturated or unsaturated monocyclic or bicyclic heterocyclyl, containing 1-4 heteroatom(s) selected from O, N or S, such as pyridyl, quinolinyl, thiazolyl, piperidinyl, morpholyl, tetrahydroquinolinyl, oxazolyl, isoxazolyl, furyl thiophen, triazolyl, pyrrolidinyl ring optionally substituted with one or more hydroxy, alkylhydroxy, alkyl, alkoxy, trifluoromethyl, amino, alkylamino, dialkylamino, aminomethyl, alkylaminomethyl, dialkylaminomethyl, acylamino, cyano, halogen or oxo group;
R₂ is phenyl optionally substituted with one or more substituent(s) selected from alkyl, methoxy; trifluoromethyl, amino, alkylamino, dialkylamino, aminomethyl, alkylaminomethyl, dialkylaminomethyl, acylamino, alkylsulfonyl, alkylsulfonylamino, cyano, halogen, or
saturated or unsaturated monocyclic or bicyclic C₅₋₇ heterocyclyl group, containing 1-4 heteroatom(s) selected from O, N or S, such as pyridyl, quinolinyl, thiazolyl, piperidinyl, morpholyl, tetrahydroquinolinyl, oxazolyl, isoxazolyl, furyl thiophen, triazolyl, pyrrolidinyl ring optionally substituted with one or more hydroxy, alkylhydroxy, alkyl, alkoxy, trifluoromethyl, amino, alkylamino, dialkylamino, aminomethyl, alkylaminomethyl, dialkylaminomethyl, acylamino, cyano, halogen or oxo group, or
NR₃R₄ group wherein R₃ and R₄ are independently selected from the group of hydrogen and alkyl group optionally substituted with one or more substituent(s) selected from alkoxy, trifluoromethyl, amino, alkylamino, dialkylamino, aminomethyl, alkylaminomethyl, dialkylaminomethyl, acylamino, cyano, halogen, or R₃ and R₄ together with the N atom to which they are attached form a C₅₋₇ heterocyclyl group, containing one or more heteroatom(s), selected from O, N or S, optionally substituted with one or more hydroxy, alkylhydroxy, alkyl, alkoxy, trifluoromethyl, amino, alkylamino, dialkylamino, aminomethyl, alkylaminomethyl, dialkylaminomethyl, acylamino, cyano, halogen or oxo group, or
NH-CO-NR₅R₆ group, wherein R₅ and R₆ are independently selected from the group hydrogen and alkyl group optionally substituted with one or more substituent(s) selected from alkoxy, trifluoromethyl, amino, alkylamino, dialkylamino, aminomethyl, alkylaminomethyl, dialkylaminomethyl, acylamino, cyano, halogen, or R₅ and R₆ together with the N atom to which they are attached form a C₅₋₇ heterocyclyl group, containing one or more heteroatom(s), selected from O, N or S, optionally substituted with one or more hydroxy, alkylhydroxy, alkyl, alkoxy, trifluoromethyl, amino, alkylamino, dialkylamino, aminomethyl, alkylaminomethyl, dialkylaminomethyl, acylamino, cyano, halogen or oxo group and/or hydrates and/or solvates and/or pharmaceutically acceptable salts thereof formed with acids or bases.

With respect to Z and the substituents of R₁, R₂, R₃, R₄, R₅ and R₆ the term alkyl means an alkyl group containing 1 to 4 carbon atom(s) with straight or branched chain, excepting when R₁ represents cycloalkyl, wherein the cyvloalkyl moiety contains 3 to 10 carbon atoms.

When R₁ and/or R₃ and/or R₄ and/or R₅ and/or R₆ the alkyl group may be optionally substituted with one or more substituent(s) selected from alkoxy, trifluoromethyl, amino, alkylamino, dialkylamino, aminomethyl, alkylaminomethyl, dialkylaminomethyl, acylamino, cyano, fluoro, chloro, bromo.

When R₁ represents cycloalkyl, the cycloalkyl moiety contains 3 to 10 carbon atoms and may be a mono-, bi-, or tricyclic group, such as cyclohexyl or adamantyl, and the cycloalkyl group may be optionally substituted with one or more substituent(s) selected from alkyl, methoxy, trifluoromethyl, amino, alkylamino, dialkylamino, aminomethyl, alkylaminomethyl, dialkylaminomethyl, acylamino, cyano, fluoro, chloro, bromo.

When R₁ and/or R₂ represents phenyl or R₁ represents biphenyl, the phenyl or biphenyl group may be optionally substituted with one or more substituent(s) selected from alkyl, methoxy, trifluoromethyl, amino, alkylamino, dialkylamino, aminomethyl, alkylaminomethyl, dialkylaminomethyl, acylamino, alkylsulfonyl, alkylsulfonylamino, cyano, fluoro, chloro, bromo.

When R₁ and/or R₂ represents C₅₋₇ heterocyclyl, the heterocyclic ring may be saturated or unsaturated monocyclic or bicyclic ring containing 1-4 heteroatom(s) selected from O, N or S, such as pyridyl, quinolinyl, thiazolyl, piperidinyl, morpholyl, tetrahydroquinolinyl, oxazolyl, isoxazolyl, furyl thiophen, triazolyl, pyrrolidinyl ring. When the heteoatom containing ring for R2 has no aromatic character, it must contain at least one basic nitrogen atom by which the heterocyclic group is connected with the thienopyridine moiety. The heterocyclyl group may be optionally substituted one or more hydroxy, alkylhydroxy, alkyl, methoxy, trifluoromethyl, amino, alkylamino, dialkylamino, aminomethyl, alkylaminomethyl, dialkylaminomethyl, acylamino, cyano, fluoro, chloro, bromo *or oxo group.*

Compounds of formula (I) may form salts with acids. The invention relates also to the salts of compounds of formula (I) formed with acids, especially the salts formed with pharmaceutically acceptable acids. The meaning of compound of formula (I) is either the free base or the salt even if it is not referred separately.

Both organic and inorganic acids can be used for the formation of acid addition salts. Suitable inorganic acids can be for example hydrochloric acid, sulfuric acid, nitric acid and phosphoric acid. Representatives of monovalent organic acids can be for example formic acid, acetic acid, propionic acid, and different butyric acids, valeric acids and capric acids. Representatives of bivalent organic acids can be for example oxalic acid, malonic acid, maleic acid, fumaric acid and succinic acid. Other organic acids can also be used, such as hydroxy acids for example citric acid, tartaric acid, or aromatic carboxylic acids for example benzoic acid or salicylic acid, as well as aliphatic and aromatic sulfonic acids for example methanesulfonic acid, naphthalenesulfonic acid and p-toluenesulfonic acid. Especially valuable group of the acid addition salts is in which the acid component itself is physiologically acceptable and does not have therapeutical effect in the applied dose or it does not have unfavourable influence on the effect of the active ingredient. These acid addition salts are pharmaceutically acceptable acid addition salts. The reason why acid addition salts, which do not belong to the pharmaceutically acceptable acid addition salts belong to the present invention is, that in given case they can be advantageous in the purification and isolation of the desired compounds.

Solvates and/or hydrates of compounds of formula (I) are also included within the scope of the invention.

Especially important compounds of formula (I) of the present invention are the following:
2-(4-chloro-benzenesulfonyl)-3-(4-chloro-phenyl)-thieno[2,3-*b*]pyridine,
2-(4-chloro-benzenesulfinyl)-3-(4-chloro-phenyl)-thieno[2,3-*b*]pyridine,
3-(4-chloro-phenyl)-2-(4-fluoro-benzenesulfonyl)-thieno[2,3-*b*]pyridine,
3-(4-chloro-phenyl)-2-(toluene-4-sulfonyl)-thieno[2,3-*b*]pyridine,
4-[3-(4-chloro-phenyl)-thieno[2,3-*b*]pyridine-2-sulfonyl]-benzonitrile,
2-benzenesulfonyl-3-(4-chloro-phenyl)-thieno[2,3-*b*]pyridine,
3-[3-(4-chloro-phenyl)-thieno[2,3-*b*]pyridine-2-sulfonyl]-benzonitrile,
3-(4-chloro-phenyl)-2-(pyridine-3-sulfonyl)-thieno[2,3-*b*]pyridine,
2-(butane-1-sulfonyl)-3-(4-chloro-phenyl)-thieno[2,3-*b*]pyridine,
3-(4-chloro-phenyl)-2-(2,4-dimethyl-thiazole-5-sulfonyl)-thieno[2,3-*b*]pyridine,
3-(4-chloro-phenyl)-2-(thiophene-2-sulfonyl)-thieno[2,3-*b*]pyridine,
3-(4-chloro-phenyl)-thieno[2,3-*b*]pyridine-2-sulfonic acid (4-chloro-phenyl)-amide,
3-(4-chloro-phenyl)-2-phenylmethanesulfonyl-thieno[2,3-*b*]pyridine,
2-(3-chloro-benzenesulfonyl)-3-(4-chloro-phenyl)-thieno[2,3-*b*]pyridine,
2-(4-chloro-benzenesulfonyl)-3-(4-chloro-phenyl)-6-chloro-thieno[2,3-*b*]pyridin,
2-(3-fluoro-benzenesulfonyl)-3-(4-chloro-phenyl)- thieno[2,3-*b*]pyridin,
2-(3-cyano-benzenesulfonyl)-3-(3-fluoro-phenyl)-thieno[2,3-*b*]pyridine,
2-(4-chloro-benzenesulfonyl)-3-(4-chloro-phenyl)-6-fluoro-thieno[2,3-*b*]pyridin,
2-(4-cyano-benzenesulfonyl)-3-(3-chloro-phenyl)-thieno[2,3-*b*]pyridine,
2-(3-cyano-benzenesulfonyl)-3-(3-methyl-piperidinyl)-thieno[2,3-*b*]pyridine,
2-(3-cyano-benzenesulfonyl)-3-(4-methyl-piperidinyl)-thieno[2,3-*b*]pyridine,
2-(3-cyano-benzenesulfonyl)-3-(4-tolyl)-thieno[2,3-*b*]pyridine,
2-(3-cyano-benzenesulfonyl)-3-(3-methoxy-phenyl)-thieno[2,3-*b*]pyridine,
2-(3-methoxy-benzenesulfonyl)-3-(4-chloro-phenyl)-thieno[2,3-*b*]pyridine,
2-(3-cyano-5-fluoro-benzenesulfonyl)-3-(4-chloro-phenyl)-thieno[2,3-*b*]pyridine,
2-(3-fluoro-4-methyl-benzenesulfonyl)-3-(4-chloro-phenyl)-thieno[2,3-*b*]pyridine,
2-(3,4-dimethyl-benzenesulfonyl)-3-(4-fluoro-phenyl)-thieno[2,3-*b*]pyridine,
2-(3-cyano-benzenesulfonyl)-3-(4-fluoro-phenyl)-6-chloro-thieno[2,3-*b*]pyridine,
2-(3-cyano-5-fluoro-benzenesulfonyl)-3-(4-fluoro-phenyl)-thieno[2,3-*b*]pyridine,
2-(3-cyano-benzenesulfonyl)-3-(4-fluoro-phenyl)-6-fluoro-thieno[2,3-*b*]pyridine,
2-(4-bromo-benzenesulfonyl)-3-(4-chloro-phenyl)-thieno[2,3-*b*]pyridin,
5-amino-2-(4-chloro-benzenesulfonyl)-3-(4-chloro-phenyl)-thieno[2,3-*b*]pyridin,
2-(3,4-dichloro-benzenesulfonyl)-3-(4-chloro-phenyl)-thieno[2,3-*b*]pyridin,
2-(3-fluoro-4-methyl-benzenesulfonyl)-3-(4-fluoro-phenyl)-thieno[2,3-*b*]pyridine,
2-(3-cyano-benzenesulfonyl)-3-(2-chloro-phenyl)-thieno[2,3-*b*]pyridine,
2-(3-cyano-5-fluoro-benzenesulfonyl)-3-(4-chloro-phenyl)-6-chloro-thieno[2,3-*b*]pyridine,
2-(3-fluoro-4-methoxy-benzenesulfonyl)-3-(4-chloro-phenyl)-thieno[2,3-*b*]pyridine,
2-(4-chloro-benzenesulfonyl)-3-(4-methyl-piperidinyl)-thieno[2,3-*b*]pyridine,
2-(3-ciano-5-fluoro-benzenesulfonyl)-3-(4-methyl-piperidinyl)-thieno[2,3-*b*]pyridine,
2-(4-chloro-benzenesulfonyl)-3-(4-chloro-phenyl)-5-fluoro-thieno[2,3-*b*]pyridin,
2-(4-chloro-benzenesulfonyl)-3-(4-chloro-phenyl)-5-chloro-thieno[2,3-*b*]pyridin,
2-(4-chloro-benzenesulfonyl)-3-(4-chloro-phenyl)-4-chloro-thieno[2,3-*b*]pyridin,
2-(4-chloro-benzenesulfonyl)-3-(4-chloro-phenyl)-4-fluoro-thieno[2,3-*b*]pyridin,
5-amino-2-(4-chloro-benzenesulfonyl)-3-(4-methyl-piperidinyl)-thieno[2,3-*b*]pyridin,
2-(3-cyano-5-fluoro-benzenesulfonyl)-3-(3-fluoro-phenyl)-thieno[2,3-*b*]pyridine,
5-amino-2-(4-chloro-benzenesulfonyl)-3-(3-fluoro-phenyl)-thieno[2,3-*b*]pyridin,
2-(4-chloro-benzenesulfonyl)-3-(4-methyl-piperidinyl)-6-chloro-thieno[2,3-*b*]pyridin,
2-(3-fluoro-4-methoxy-benzenesulfonyl)-3-(4-fluoro-phenyl)-thieno[2,3-*b*]pyridine,
2-(3-fluoro-4-methoxy-benzenesulfonyl)-3-(3-fluoro-phenyl)-thieno[2,3-*b*]pyridine,
2-(3-cyano-5-fluoro-benzenesulfonyl)-3-(2-fluoro-phenyl)-thieno[2,3-*b*]pyridine,
2-(3-cyano-5-fluoro-benzenesulfonyl)-3-(3-chloro-phenyl)-thieno[2,3-*b*]pyridine.

### Pharmaceutical formulations

The invention also relates to the pharmaceutical compositions containing the compounds of formula (I) and/or physiologically acceptable salts and/or hydrates and/or solvates thereof as active ingredient and one or more physiologically acceptable carriers.

The compounds of formula (I) and/or physiologically acceptable salts and/or hydrates and/or solvates thereof may be administered by any convenient method, for example by oral, parenteral (including subcutaneous, intramuscular, and intravenous), buccal, sublingual, nasal, rectal or transdermal administration and the pharmaceutical compositions adapted accordingly.

The compounds of formula (I) and/or physiologically acceptable salts and/or hydrates and/or solvates thereof which are active when given orally can be formulated as liquids or solids, for example syrups, suspensions or emulsions, tablets, capsules and lozenges.

A liquid formulation of the compounds of formula (I) and/or physiologically acceptable salts and/or hydrates and/or solvates thereof generally consist of a suspension or solution of the compound of formula (I) and/or physiologically acceptable salts and/or hydrates and/or solvates thereof in a suitable liquid carrier(s) for example an aqueous solvent, such as water and ethanol or glycerine, or a non-aqueous solvent, such as polyethylene glycol or an oil. The formulation may also contain a suspending agent, preservative, flavouring or colouring agent.

A composition in the solid form of a tablet can be prepared using any suitable pharmaceutical carrier(s) routinely used for preparing solid formulations. Examples of solid carriers include lactose, terra alba, sucrose, talc, gelatine, agar, pectin, acacia, magnesium stearate, stearic acid etc. Optionally, tablets may be coated by standard aqueous or nonaqueous techniques.

A composition in the solid form of a capsule can be prepared using routine encapsulation procedures. For example, pellets containing the active ingredient can be prepared using standard carriers and then these are filled into a hard gelatine capsule; alternatively, a dispersion or suspension can be prepared using any suitable pharmaceutical carrier(s), for example aqueous gums, celluloses, silicates or oils and the dispersion or suspension then is filled into a soft gelatine capsule.

Typical parenteral compositions consist of a solution or suspension of the compound of formula (I) and/or physiologically acceptable salts and/or hydrates and/or solvates thereof in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions of the present invention for nasal administration containing a compound of formula (I) and/or physiologically acceptable salts and/or hydrates and/or solvates thereof may conveniently be formulated as aerosols, drops, gels and powders. Aerosol formulations of the present invention typically comprise a solution or fine suspension of the compound of formula (I) and/or physiologically acceptable salts and/or hydrates and/or solvates in a physiologically acceptable aqueous or non-aqueous solvent and are usually presented in a single or multidose quantities in sterile form in a sealed container, which can take the form of a cartridge or refill for use with an atomizing device. Alternatively, the sealed container may be a unitary dispensing device, such as a single dose nasal inhaler or an aerosol dispenser fitted with a metering valve which is intended for disposal once the contents of the container have been exhausted. If the dosage form comprises an aerosol dispenser, it will contain a propellant which can be a compressed gas, such as compressed air or an organic propellant, such as a fluorochlorohydrocarbon. The aerosol dosages form can also take the form of a pump-atomiser.

Compositions of the present invention containing a compound of formula (I) and/or physiologically acceptable salts and/or hydrates and/or solvates are suitable for buccal or sublingual administration including tablets, lozenges and pastilles, wherein the active ingredient is formulated with a carrier, such as sugar and acacia, tragacanth, or gelatine, glycerin etc.

Compositions of the present invention containing a compound of formula (I) and/or physiologically acceptable salts and/or hydrates and/or solvates thereof for rectal administration are conveniently in the form of suppositories containing a conventional suppository base, such as cocoa butter and other materials commonly used in the art. The suppositories may be conveniently formed by first admixing the composition with the softened or melted carrier(s) followed by chilling and shaping in moulds.

Compositions of the present invention containing a compound of formula (I) and/or physiologically acceptable salts and/or hydrates and/or solvates thereof for transdermal administration include ointments, gels and patches.

The compositions of the present invention containing a compound of formula (I) and/or physiologically acceptable salts and/or hydrates and/or solvates thereof is preferably in the unit dose form, such as tablet, capsule or ampoule.

Each dosage unit of the present invention for oral administration contains preferably from 0.1 to 500 mg of a compound of formula (I) and/or physiologically acceptable salts and/or hydrates and/or solvates thereof calculated as a free base.

Each dosage unit of the present invention for parenteral administration contains preferably from 0.1 to 500 mg of a compound of formula (I) and/or physiologically acceptable salts and/or hydrates and/or solvates thereof calculated as a free base.

The compounds of formula (I) and/or physiologically acceptable salts and/or hydrates and/or solvates thereof can normally be administered in a daily dosage regimen. In the treatment of mGluR1 and mGluR5 mediated disorders, such as schizophrenia, anxiety, depression, panic, bipolar disorders, and circadian disorders or chronic and acute pain disorders the dosage levels from about 0,01 mg/kg to about 140 mg/kg of body weight per day are useful or alternatively about 0.5 mg to about 7 g per patient per day.

The amount of active ingredient that may be combined with the carrier materials to produce a single dosage form will vary depending upon the host treated and the particular mode of administration. For example, a formulation intended for the oral administration to humans may conveniently contain from about 0.5 mg to about 5 g of active agent, compounded with an appropriate and convenient amount of carrier material which may vary from about 5 to about 95 percent of the total composition. Unit dosage forms will generally contain between from about 1 mg to about 1000 mg of the active ingredient, typically 25 mg, 50 mg, 100 mg, 200 mg, 250-300 mg, 400 mg, 500 mg, 600 mg, 800 mg or 1000 mg.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

### Medical use

The compounds of formula (I) and/or physiologically acceptable salts and/or hydrates and/or solvates of the present invention have been found to exhibit biological activity at mGluR1 and mGluR5 receptors and are expected to be useful in the treatment of mGluR1 and mGluR5 mediated disorders.

It has been found that the compounds according to the present invention or salts thereof, exhibit a high degree of potency and selectivity for individual metabotropic glutamate receptor (mGluR) subtypes. In particular there are compounds according to the present invention that are potent and selective for mGluR1 and mGluR5 receptors. Accordingly, the compounds of the present invention are expected to be useful in the prevention and/or treatment of conditions associated with excitatory activation of mGluR1 and mGluR5 receptor and for inhibiting neuronal damage caused by excitatory activation of mGluR1 and mGluR5 receptor. The compounds may be used to produce an inhibitory effect of mGluR1 and mGluR5, in mammals, including human.

Thus, it is expected that the compounds of the invention are well suited for the prevention and/or treatment of mGluR1 and mGluR5 receptor-mediated disorders such as acute and chronic neurological and psychiatric disorders, chronic and acute pain disorders and neuromuscular dysfunction of the lower urinary tract and gastrointestinal disorders.

The dose required for the therapeutic or preventive treatment of a particular disorder will necessarily be varied depending on the host treated and the route of administration.

The invention relates to compounds of formula (I) as defined hereinbefore, for use in therapy.

The invention relates to compounds of formula (I) as defined hereinbefore, for use in prevention and/or treatment of mGluR1 and mGluR5 receptor-mediated disorders.

The invention relates to compounds of formula (I) as defined hereinbefore, for use in prevention and/or treatment of neurological disorders.

The invention relates to compounds of formula (I) as defined hereinbefore, for use in prevention and/or treatment of psychiatric disorders.

The invention relates to compounds of formula (I) as defined hereinbefore, for use in prevention and/or treatment of chronic and acute pain disorders.

The invention relates to compounds of formula (I) as defined hereinbefore, for use in prevention and/or treatment of neuromuscular dysfunction of the lower urinary tract and gastrointestinal disorders.

The invention relates to compounds of formula (I) as defined hereinbefore, for use in prevention and/or treatment of pain related to migraine, inflammatory pain, neuropathic pain disorders such as diabetic neuropathies; arthritis and rheumatoid diseases, low back pain, post-operative pain and pain associated with various conditions including angina, in renal or biliary colic, menstruation, migraine and gout.

The invention relates to compounds of formula (I) as defined hereinbefore, for use in prevention and/or treatment of Alzheimer's disease senile dementia, AIDS-induced dementia Parkinson's disease, amyotrophic lateral sclerosis, Huntington's Chorea, migraine, epilepsy, schizophrenia, depression, anxiety, acute anxiety, obesity, obsessive compulsive disorder, ophthalmological disorders such as retinopathies, diabetic retinopathies, glaucoma, auditory neuropathic disorders such as tinnitus, chemotherapy induced neuropathies, post-herpetic neuralgia and trigeminal neuralgia, tolerance, dependency, Fragile X, autism, mental retardation, schizophrenia and Down's Syndrome.

The invention relates to compounds of formula (I) as defined hereinbefore, for use in prevention and/or treatment of stroke, head trauma, anoxic and ischemic injuries, hypoglycemia, cardiovascular diseases and epilepsy.

The compounds are also well suited for the treatment of neuromuscular dysfunction of the lower urinary tract, such as urinary urgency, overactive bladder, greater urinary frequency, reduced urinary compliance, cystitis, incontinence, enuresis and dysuria.

Furthermore, the compounds are also well suited for the treatment of gastrointestinal disorders, such as transient lower esophageal sphincter relaxation (TLESR), gastrointestinal reflux disease and irritable bowel syndrome.

The present invention relates also to the use of a compound of formula (I) as defined hereinbefore, in the manufacture of a medicament for the prevention and/or treatment of mGluRs1 and mGluR5 receptor-mediated disorders and any disorder listed above.

The invention also provides a method of treatment and/or prevention of mGluR1 and mGluR5 receptor mediated disorders and any disorder listed above, in a patient suffering from, or at risk of, said condition, which comprises administering to the patient an effective amount of a compound of formula (I), as hereinbefore defined.

In the context of the present specification, the term "therapy" includes treatment as well as prevention, unless there are specific indications to the contrary. The terms "therapeutic" and "therapeutically" should be construed accordingly.

In this specification, unless stated otherwise, the term "antagonist" means a compound that by any means, partly or completely blocks the transduction pathway leading to the production of a response by the ligand.

The term "disorder", unless stated otherwise, means any condition and disease associated with metabotropic glutamate receptor activity.

### Methods of preparation

### Abbreviations

The abbreviations used herein have the following tabulated meaning. Abbreviations not tabulated below have their meanings as commonly used unless specifically stated otherwise.

| | |
|---|---|
| DMF | *N*,*N*-dimethylformamide |
| TEA | triethylamine |
| THF | tetrahydrofuran |
| DMSO | dimethyl sulphoxide |

According to the preset invention a process for the preparation of a compound of formula (Ia): 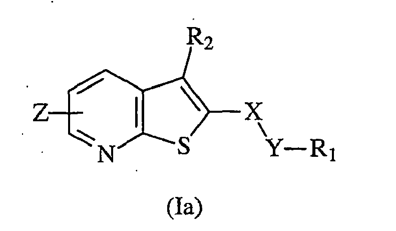 wherein
X represents a group selected from SO, SO₂;
Y represents a group selected from (CH₂)ₙ, NH, NHCH₂;
n is an integer of 0 to 1;
Z is H or monosubstituted by alkyl or nitro group,
R₁ is an optionally substituted alkyl, cycloalkyl, phenyl, biphenyl, heterocyclyl;
R₂ is phenyl, optionally substituted with alkyl or halogen;
by reacting a compound of formula (III): 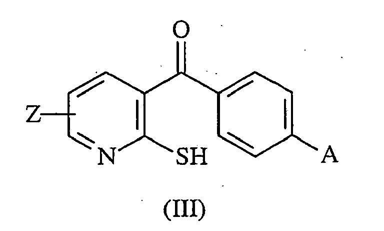
wherein A is a substituent as defined R₂ in formula (Ia) and Z is H or monosubstituted by alkyl or nitro group with a compound of formula (VII):

HlgCH₂SOYR₁ (VII)

wherein Hlg is chloro or bromo, Y and R₁ are as described above for the formula (Ia), or with a compound of formula (VIII):

HlgCH₂SO₂YR₁ (VIII)

wherein Hlg is chloro or bromo, Y and R₁ are as described above for the formula (Ia), in the presence of sodium methoxide in dimethylformamide as solvent to obtain a compound of formula (Ia), and optionally thereafter forming salts and/or hydrates and/or solvates of compounds of formula (Ia) (Scheme 1). 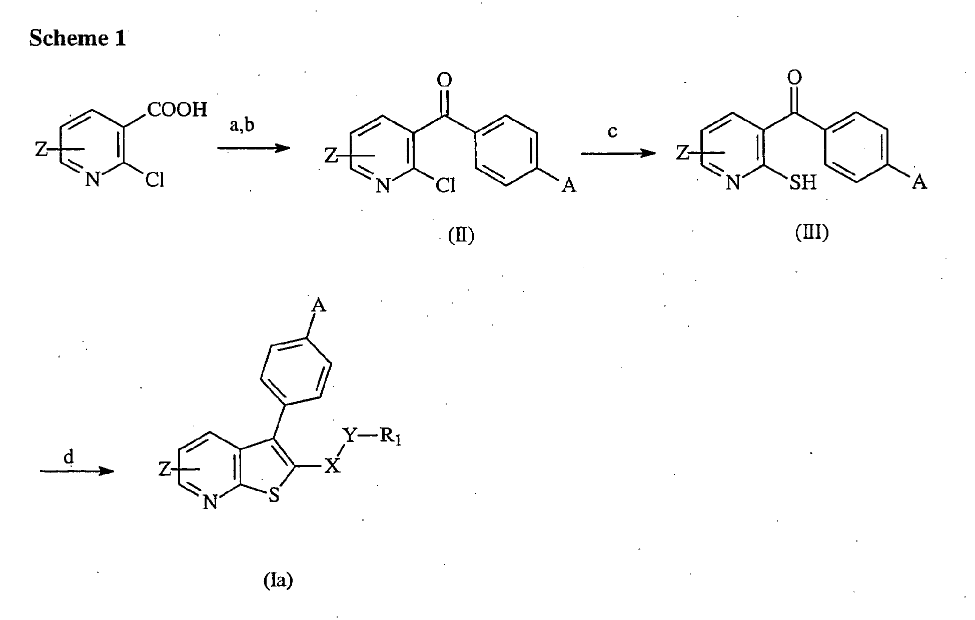
a. SOCl₂, Ph-A (compound of formula (VI), wherein A is a substituent as defined R₂ in formula (Ia)), catalytic amount of DMF, 80-130 °C, 2-3 hours;
b. AlCl₃, 80-130 °C, 5-8 hours;
c. thiourea, water/ethanol, reflux, 20-24 hours;
d. HlgCH₂SOYR₁ or HlgCH₂SO₂YR₁ (compound of formula (VII) and formula (VIII) respectively, wherein Hlg is chloro or bromo), NaOCH₃, DMF, 70-150°C, 2-6 hours.

Acid chloride was prepared from the appropriate 2-chloro-nicotinic acid or 5-nitro-2-chloronicotinic acid by the reaction of thionylchloride with the appropriate 4-monosubstituted benzene derivative in the presence of AlCl₃. The reaction may be carried out by well known methods suitable for Friedel-Crafts reactions using the appropriate benzene derivative as solvent.

The product (II) was purified by crystallization and reacted with thiourea in a mixture of water and ethanol under reflux according to the method of J. Katritzky (see: J. Chem. Soc., 1958, 3610). The resulted compounds of formula (III) are in crystalline form.

The S-alkylation and the ring closure were carried out by the method of F.Guerrera (Farmaco Ed. Sci., 1976, 31, 21).

Compounds of formula (III) were reacted with different halomethylsulfoxid, halomethylsulfone or halomethylsulfonamide derivatives in the presence of a base (e.g. NaOMe, Cs₂CO₃ or KOH).

The halomethylsulfoxide derivatives can be prepared from the appropriate halomethylsulfides with *m*-chloro-perbenzoic acid by the method of G. Letts et al. (J. Med. Chem., 2003, 465).

The halomethylsulfon derivatives are either commercially available or can be synthesized by known methods e.g. Y. Yinfa et al. (Synth. Commum., 2004, 34, 13, 2443).

The halomethylsulfonamido derivatives can be prepared e.g. by the method of K. Wojciechowski et al. (Tetrahedron, 2001, 57, 5009).

According to the present invention a process for the preparation of a compound of formula (Ib): 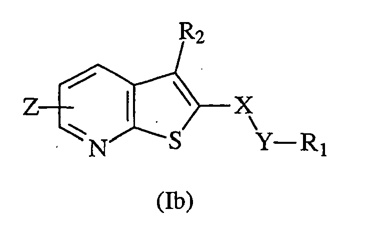 wherein
X represents SO, SO₂;
Y represents a group selected from (CH₂)ₙ, NH, NHCH₂;
n is an integer of 0 to 1;
Z is H or alkyl group;
R₁ is an optionally substituted alkyl, cycloalkyl, phenyl, biphenyl, heterocyclyl;
R₂ is phenyl, or aromatic heterocyclyl, optionally substituted with one or more substituent(s) selected from alkyl, alkoxy, trifluoromethyl, amino, alkylamino, dialkylamino, aminomethyl, alkylaminomethyl, dialkylaminomethyl, acylamino, cyano, fluoro, chloro, bromo, hydroxyl, methylsulfonyl,
by reacting a compound of formula (XI): 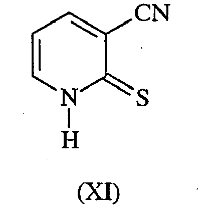 with a compound of formula (VII):

HlgCH₂SOYR₁ (VII)

wherein Hlg is chloro or bromo, Y and R₁ are as described above for the formula (Ib), or with a compound of formula (VIII):

HlgCH₂SO₂YR₁ (VIII)

wherein Hlg is chloro or bromo, Y and R₁ are as described above for the formula (Ib) in the presence of sodium methoxide in dimethylformamide as solvent and
reacting the obtained compound of formula (IV): 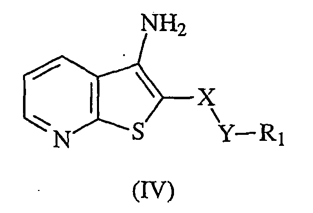 wherein R₁ are as described above for the formula (Ib) with copper(II) bromide and *tert*-butyl nitrite in acetonitrile; and reacting the obtained compound of formula (V): 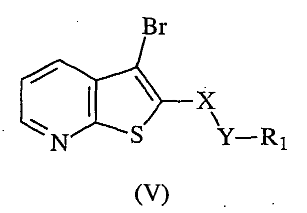 wherein R₁ are as described above for the formula (Ib) with a compound of formula (IX):

R₂-B (OH)₂ (IX)

wherein R₂ is as defined above for formula (Ib), in the presence of base and catalyst in a solvent to obtain a compound of formula (Ib), and optionally thereafter forming salts and/or hydrates and/or solvates of compounds of formula (Ib) (Scheme 2). 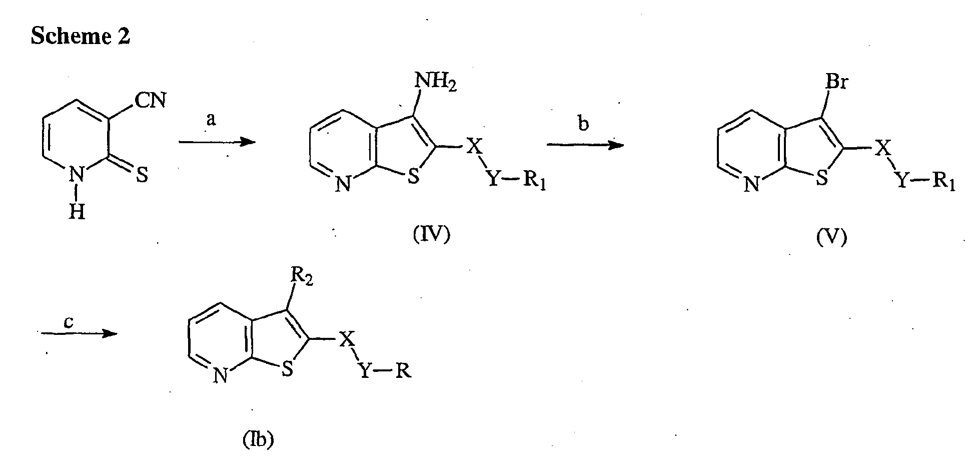
a. HlgCH₂SOYR₁ or HlgCH₂SO₂YR₁ (compound of formula (VII) and formula (VIII) respectively, wherein Hlg is chloro or bromo), DMF or butanol, 70-150 °C, 1-3 hours;
b. CuBr₂, tert-butyl nitrite, CH₃CN, 60-80 °C, 1-3 hours;
c. R₂-B(OH)₂ (compound of formula (IX), wherein R₂ is as defined above for formula (Ib), Na₂CO₃, ethanol-toluene, or dimethoxyethane, Pd(PPh₃)₄, 1-5 hours, 20-110 °C;

Compounds of formula (IV) were prepared from 2-mercapto-nicotinonitrile and the corresponding halomethyl-sulfone compound of formula (VIII) by the method of F.Guerrera (Farmaco Ed. Sci., 1976, 31, 21).

The obtained amino-thienopyridine derivatives of formula (IV) were converted to the appropriate bromine derivatives of formula (V) by analogous methods described in the literature H.C.Wals (e.g. Tetrahedron, 1988, 44, 5921). The reaction was carried out in acetonitrile using t-butylnitrite or i-amylnitrite and Cu(II) salt (e.g.CuBr₂) between 60-80°C temperature.

Compounds of formula (Ib) were synthesized by the well known methods of the Suzuki coupling reactions using the appropriate boronic acid-or boronic ester, base and palladium catalyst as described e.g. A. Suzuki & H. C. Brown: Organic Syntheses via Boranes Vol.1-3.

According to the present invention a process for the preparation of a compound of formula (Ic) 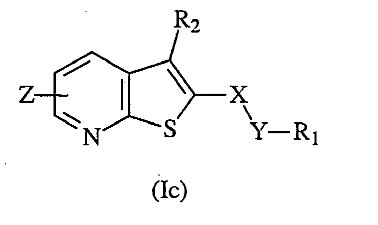 wherein
X represents SO₂;
Y represents a group selected from (CH₂)ₙ, NH, NHCH₂;
n is an integer of 0 to 1;
Z is H or alkyl group;
R₁ is an optionally substituted alkyl, cycloalkyl, phenyl, biphenyl, heterocyclyl;
R₂ is NR₃R₄ wherein R₃ and R₄ are independently selected from the group of hydrogen an optionally substituted alkyl group, or R₃ and R₄ together with the N atom to which they are attached form an optionally substituted C₅₋₇ heterocyclyl group, containing one or more heteroatom(s),
by reacting a compound of formula (V): 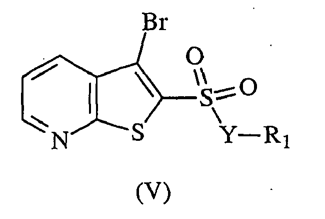 wherein R₁ are as described above for the formula (Ic), with a compound of formula (XVI):

HNR₃R₄ (XVI)

wherein R₃ and R₄ are as described above for formula (Ic) to obtain compound of formula (Ic), and optionally thereafter forming salts and/or hydrates and/or solvates of compounds of formula (Ic) (Scheme 3). 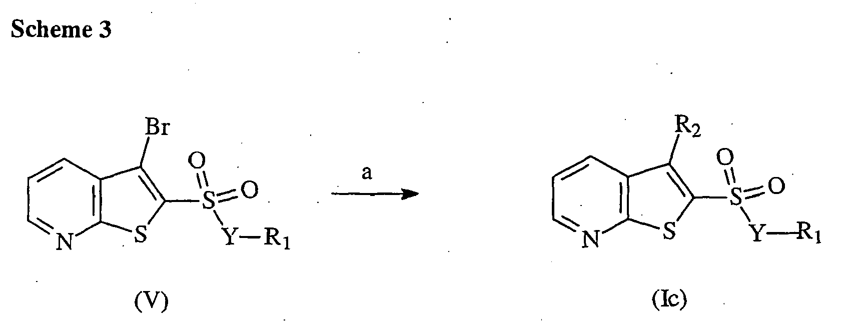
a. HNR₃R₄, 70-200 °C, 1-5 hours, acetonitrile or DMF or microwave assisted reaction.

The above described reaction was carried out by a simple method after Kirsch.G. (Tetrahedron 55, 21, 1999, 6511). The corresponding bromo compound (V) was heated with an appropriate amine (HNR₃R₄) in an appropriate solvent (acetonitrile, DMF or water) advantageously between 70-200 °C temperature. In special cases a sailed tube or microwave assisted reaction and higher temperature was used for the preparation.

According to the present invention a process for the preparation of a compound of formula (Id): 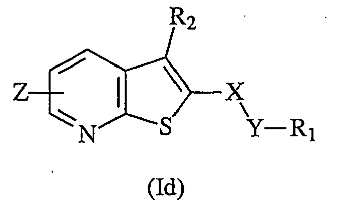 wherein
X represents SO₂;
Y represents a group selected from (CH₂)ₙ, NH, NHCH₂;
n is an integer of 0 to 1;
Z is H or alkyl group;
R₁ is an optionally substituted alkyl, cycloalkyl, phenyl, biphenyl, heterocyclyl;
R₂ is NH-CO-NR₃R₄ group, wherein R₅ and R₆ are independently selected from the group of hydrogen or optionally substituted alkyl group, or R₃ and R₄ together with the N atom to which they are attached form an optionally substituted C₅₋₇ heterocyclyl group, containing one or more heteroatom(s),
by reacting a compound of formula (IV): 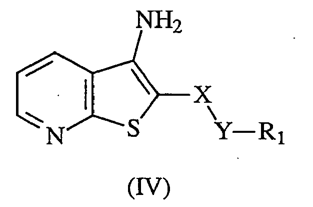 wherein R₁ are as described above for the formula (Id), with a compound of formula (XIII):

HNR₃R₄ (XIII)

wherein R₃ and R₄ are as described above for formula (Id), in the presence of phosgene or triphosgene and base to obtain a compound of formula (Id), and optionally thereafter forming salts and/or hydrates and/or solvates of compounds of formula (Id) (Scheme 4). 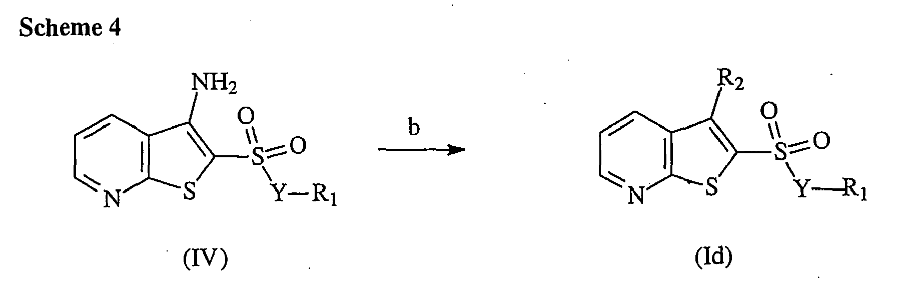
b. phosgene or triphosgene, NHR₃R₄ (compounds of formula (XIII)), base, (-)5°C-RT

The process for preparing compounds of formula (Id) is transforming in situ an amine of formula (IV) to its isocyanate derivative and reacting the latter with an amine of formula (XIII). The above reaction may be carried out by known methods. The transformation of amine (IV) to its isocyanate derivative was prepared in situ in an aprotic solvent (e.g. tetrahydrofurane, chlorinated hydrocarbons) by using an appropriate carbonic acid derivate (e.g. phosgene or triphosgene) in the presence of a base (e.g. triethylamine), advantageously between -5°C and room temperature.

According to the present invention a process for the preparation of a compound of formula (Ie): 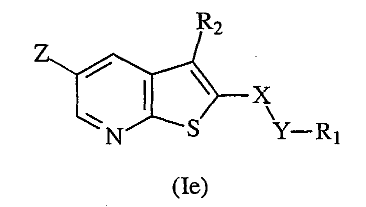 wherein
X represents a group selected from SO, SO₂;
Y represents a group selected from (CH₂)ₙ, NH, NHCH₂;
n is an integer of 0 to 1;
Z is amino, alkylsulfonylamino, halogen;
R₁ is an optionally substituted alkyl, cycloalkyl, phenyl, biphenyl, heterocyclyl;
R₂ is phenyl, optionally substituted with alkyl or halogen;
by reacting a compound of formula (Ia): 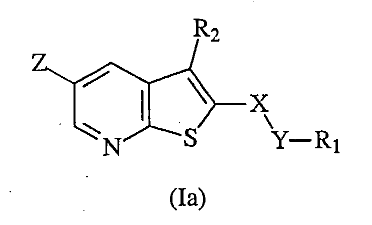 wherein
X represents a group selected from SO, SO₂;
Y represents a group selected from (CH₂)ₙ, NH, NHCH₂;
n is an integer of 0 to 1;
Z is nitro;
R₁ is an optionally substituted alkyl, cycloalkyl, phenyl, biphenyl, heterocyclyl;
R₂ is phenyl, optionally substituted with alkyl or halogen,
with an appropriate reducing agent to obtain compound of formula (Ie) 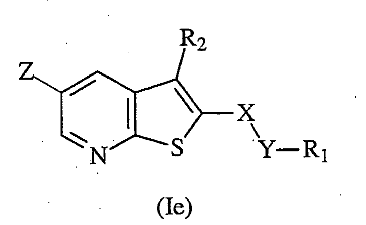 wherein Z is amino; X, Y, n, R₁ and R₂ are as described above for formula (Ie) and optionally thereafter forming salts and/or hydrates and/or solvates of the obtained amino derivatives, or
a. optionally reacting the obtained amino derivatives of formula (Ie), wherein Z is amino; X, Y, n, R₁ and R₂ are as described above for formula (Ie) with sodium nitrite in the presence of hydrochloric acid and copper(I) iodide or copper(I) chloride or copper(I) bromide or sodium tetrafluoroborate to obtain a compound of formula (Ie), ), wherein Z is halogen; X, Y, n, R₁ and R₂ are as described above for formula (Ie), and optionally thereafter forming salts and/or hydrates and/or solvates of the obtained halogen derivatives of formula Ie or
b. optionally reacting the obtained amino derivatives of formula (Ie), wherein Z is amino; X, Y, n, R₁ and R₂ are as described above for formula (Ie) with alkylsulfonyl chloride derivatives to obtain compounds of formula (Ie), wherein Z is alkylsulfonylamino; X, Y, n, R₁ and R₂ are as described above for formula (Ie), and optionally thereafter forming salts and/or hydrates and/or solvates of the obtained alkylsulfonylamino derivatives of formula (Ie) (Scheme 5).

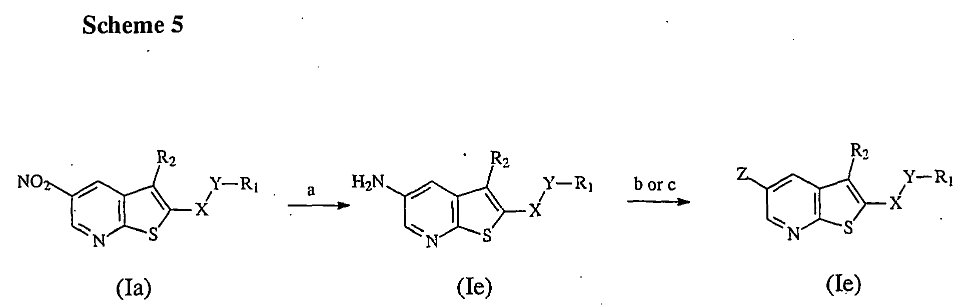

a. SnCl₂ or Fe powder/ HCl
b. NaNO₂, cc. HCl, Cu(I)I or Cu(I)Cl or Cu(I)Br or NaBF₄;
c. AlkylSO₂Cl, base, 0-80°C;

Compounds of formula (Ia) (wherein Z is nitro; X, Y, n, R₁ and R₂ are as described above for formula (Ie)) was used as starting material for the preparation of the corresponding 5-substituted thienopyridines of formula (Ie).

The appropriate nitro compounds as showed in Scheme 5 was selectively reduced e.g. with stannous chloride in ethylacetate under heating using the method of F.D. Bellamy (Tetrahedron Lett., 25, 83, 1984 ) or with iron used in combination with HCl by the method of Org. Synth Coll, 5, 346, 1973**.** This reaction gave a compounds of formula (Ie) (wherein Z is amino; X, Y, n, R₁ and R₂ are as described above for formula (Ie)), which was optionally diazoted by the well known Sandmeyer chemistry techniques after Ito,S. et al. (Bull. Chem. Soc. Jpn., 49, 1920, 1976). The reaction was carried out with sodium nitrite in conc. hydrochloric acid in the presence of copper(I) iodide or copper(I) chloride or copper(I) bromide advantageously between (-)5-(+5)°C temperature. In the case of the preparation of the corresponding fluoro compound we used sodium tetrafluoroborate at the same temperature to obtain the aryldiazonium salt, which was decomposed by heating the salt to 160-200°C.

By this preparation process halogen derivatives of formula (Ie) (wherein Z is halogen; X, Y, n, R₁ and R₂ are as described above for formula (Ie)) were obtained.

Alternatively, compound of formula (Ie) (wherein Z is amino; X, Y, n, R₁ and R₂ are as described above for formula (Ie)), was converted to sulfonamide derivatives. The reaction was carried out by the use of the corresponding sulfonylchlorides in the presence of an appropriate base (e.g. pyridine or triethylamine) between 0-80°C temperature.

According to the present invention a process for the preparation of a compound of formula (If): 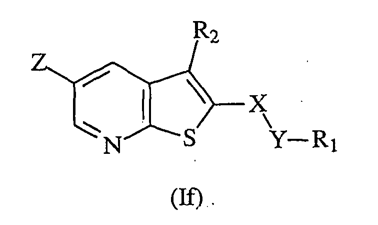 wherein
X represents SO₂;
Y represents a group selected from (CH₂)ₙ, NH, NHCH₂;
n is an integer of 0 to 1;
Z is amino, alkylsulfonylamino, halogen;
R₁ is an optionally substituted alkyl, cycloalkyl, phenyl, biphenyl, heterocyclyl;
R₂ is optionally substituted phenyl, heterocyclyl, or NR₃R₄ group wherein R₃ and R₄ are independently selected from the group of hydrogen or optionally substituted alkyl, or R₃ and R₄ together with the N atom to which they are attached form an optionally substituted C₅₋₇ heterocyclyl group, containing one or more heteroatom(s),
by reacting a compound of formula (V): 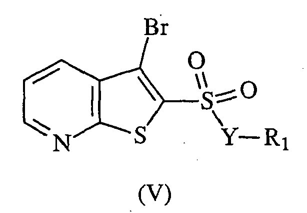 wherein R₁ and Y are as described above for the formula (If), with an appropriate oxidizing agent and reacting the obtained N-oxide derivative of formula (X): 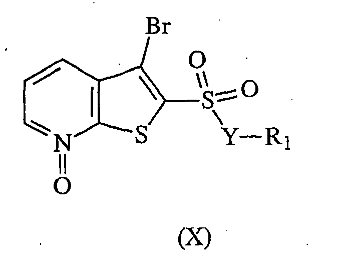 wherein R₁ and Y are as described above for the formula (If), with aquaeous nitric acid in acetic acid, thereafter reacting the obtained nitro derivative of formula (XI): 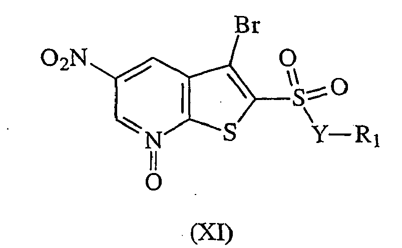 wherein R₁ and Y are as described above for the formula (If), with an appropriate reducing agent, and reacting the obtained amino derivative of formula (XII): 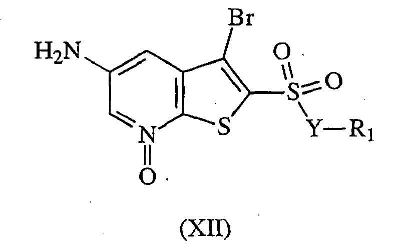 wherein R₁ and Y are as described above for the formula (If),
a. with a compound of formula (IX):

   R₂-B(OH)₂ (IX)

   wherein R₂ is optionally substituted phenyl, heterocyclyl, in the presence of base and catalyst in a solvent to obtain a compound of formula (If), wherein Z is amino; R₂ is optionally substituted phenyl, heterocyclyl, Y and R₁ are as described above for formula (If) and optionally thereafter forming salts and/or hydrates and/or solvates of the obtained amino derivatives of formula (If), and
   (i) optionally reacting the obtained amino derivatives of formula (If), wherein Z is amino; R₂ is optionally substituted phenyl, heterocyclyl, Y and R₁ are as described above for formula (If), with sodium nitrite in the presence of hydrochloric acid and copper(I) iodide or copper(I) chloride or copper(I) bromide or sodium tetrafluoroborate to obtain a compound of formula (If), wherein Z is halogen; R₂ is optionally substituted phenyl, heterocyclyl, Y and R₁ are as described above for formula (If), and optionally thereafter forming salts and/or hydrates and/or solvates of the obtained halogen derivatives of formula (If), or
   (ii) optionally reacting the obtained amino of formula (If), wherein Z is amino; R₂ is optionally substituted phenyl, heterocyclyl, Y and R₁ are as described above for formula (If), with alkylsulfonyl chloride derivatives to obtain a compound of formula (If), and optionally thereafter forming salts and/or hydrates and/or solvates of the obtained alkylsulfonylamino derivatives of formula (If), or
b. with a compound of (XIII):

   NHR₃R₄ (XIII)

   wherein R₃ and R₄ are as described above for formula (If), at 70-200°C to obtain a compound of formula (If), wherein Z is amino; Y, R₁ and R₂ which is NR₃R₄ group, are as described above for formula (If) and optionally thereafter forming salts and/or hydrates and/or solvates of the obtained amino derivatives of formula (If), or
   (i) optionally reacting the obtained amino derivatives of formula (If), wherein Z is amino; Y, R₁ and R₂ which is NR₃R₄ group, are as described above for formula (If) with sodium nitrite in the presence of hydrochloric acid and copper(I) iodide or copper(I) chloride or copper(I) bromide or sodium tetrafluoroborate to obtain a compound of formula (If), wherein Z is halogen Y, R₁ and R₂ which is NR₃R₄ group, are as described above for formula (If) and optionally thereafter forming salts and/or hydrates and/or solvates of the obtained halogen derivatives of formula (If), or
   (ii) optionally reacting the obtained amino derivatives wherein Z is amino; Y, R₁ and R₂ which is NR₃R₄ group, are as described above for formula (If) with alkylsulfonyl chloride derivatives a compound of formula (If), wherein Z alkylsulfonylamino; Y, R₁ and R₂ which is NR₃R₄ group, are as described above for formula (If), and optionally thereafter forming salts and/or hydrates and/or solvates of the obtained alkylsulfonylamino derivatives of formula (If) (Scheme 6).

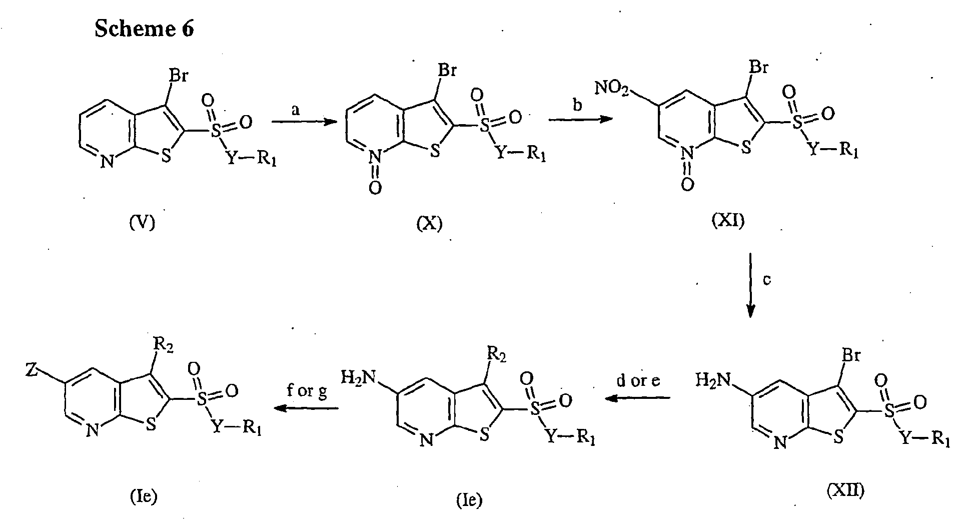

a. 3-chloro-peroxybenzoic acid, 0°C - RT, CHCl₃, 1-3 days,
b aq.HNO₃-acetic acid, 100-130°C, 2-10 hours,
c SnCl₂ or Fe powder, ethylacetate or cc.HCl,
d R₂-B(OH)₂ (compounds of formula (IX), wherein R₂ is optionally substituted phenyl, heterocyclyl); Na₂CO₃, ethanol, toluene or dimethoxyethane, Pd(PPh₃)₄, 1-5 hours, 20-110°C,
e. NHR₃R₄ (compounds of formula (XIII), wherein R₃ and R₄ are as described above for formula (If)), 70-200 °C, 1-5 hours, acetonitrile or DMF,
f NaNO₂, cc. HCl, Cu(I)I or Cu(I)Cl or Cu(I)Br or NaBF₄,
g AlkylSO₂Cl, base, 0-80°C

Starting from compound of formula (V) molecule of formula (X) was synthesized by the method described in the P0501166 Patent. The corresponding bromo derivative was transformed with m-chloroperoxybenzoic acid to the N-oxide of formula (X) in an aprotic solvent (e.g. chlorinated hydrocarbones) advantageously at low temperature, between 0°C - room temperature.

Compounds of formula (XI) were prepared from the corresponding N-oxide of formula (X) by a selective nitration reaction using aq. nitric acid. The reaction was carried out in the range of 100-130°C temperature in acetic acid by the method of Klemm,L.H. (J. Heterocycl. Chem., 7, 1970, 81). The conversion is about 20% and the unchanged starting material can be recovered.

The processes for the next reaction steps (c, d, e, f and g) were carried out as described above (see Scheme 2, 3 and 5).

According to the present invention a process for the preparation of a compound of formula (Ig): 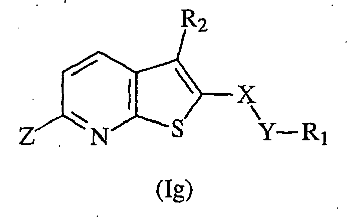 wherein
X represents SO₂;
Y represents a group selected from (CH₂)ₙ, NH, NHCH₂;
n is an integer of 0 to 1;
Z is amino, bromo, chloro, iodo, methoxy, mono- or dialkyamino;
R₁ is an optionally substituted alkyl, cycloalkyl, phenyl, biphenyl, heterocyclyl;
R₂ is an optionally substituted phenyl, heterocyclyl, or
NR₃R₄ group wherein R₃ and R₄ are independently selected from the group of hydrogen or an optionally substituted alkyl, or R₃ and R₄ together with the N atom to which they are attached form an optionally substituted C₅₋₇ heterocyclyl group, containing one or more heteroatom(s),
by reacting a compound of formula (X): 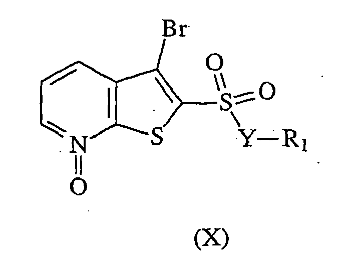 wherein R₁ and Y are as described above for the formula (Ig), with a compound of formula (IX):

R₂-B (OH)₂ (IX)

wherein R₂ is optionally substituted phenyl, heterocyclyl, in the presence of base and catalyst in a solvent, to obtain compounds of formula (XIV): 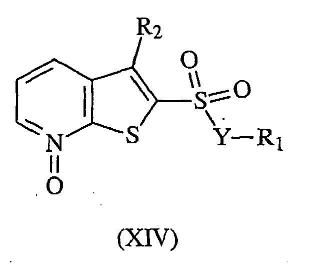 wherein R₂ is an optionally substituted phenyl, heterocyclyl, R₁ and Y are as described above for compounds of formula (Ig), or with a compound of (XIII):

NHR₃R₄ (XIII)

wherein R₃ and P₄ are as described above for formula (Ig) to obtain compounds of formula (XIV), wherein R₂ is NR₃R₄ group, wherein R₃ and R₄ are independently selected from the group of hydrogen or an optionally substituted alkyl, or R₃ and R₄ together with the N atom to which they are attached form an optionally substituted C₅₋₇ heterocyclyl group, containing one or more heteroatom(s); and
reacting compounds of formula (XIV), wherein R₁, R₂ and Y are as described above for formula (Ig), with trifluoroacetic anhydride in a solvent to obtain compounds of formula (XV): 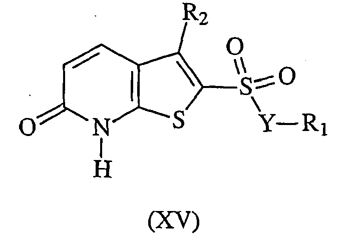 wherein R₁, R₂ and Y are as described above for formula (Ig); and reacting compounds of formula (XV) (wherein R₁, R₂ and Y are as described above for formula (Ig),
a. with phosphorous(III) oxybromide to obtain compounds of formula (Ig), wherein Z is bromo, X, Y, n, R₁ and R₂ are as described above for formula (Ig), and optionally thereafter forming salts and/or hydrates and/or solvates of the obtained bromo derivatives of formula (Ig); or
   (i) reacting the bromo derivatives of formula (Ig), wherein Z is bromo; X, Y, n, R₁ and R₂ are as described above for formula (Ig)) with potassium fluoride in DMSO to obtain compounds of formula (Ig), wherein Z is fluoro, X, Y, n, R₁ and R₂ are as described above for formula (Ig), and optionally thereafter forming salts and/or hydrates and/or solvates of the obtained fluoro derivatives of formula (Ig); or
b. with phosphorous(III) oxychloride to obtain compounds of formula (Ig), wherein Z is chloro, X, Y, n, R₁ and R₂ are as described above for formula (Ig), and optionally thereafter forming salts and/or hydrates and/or solvates of the obtained chloro deriivatives of formula (Ig); or
   (i) reacting the chloro derivatives of formula (Ig), wherein Z is chloro; X, Y, n, R₁ and R₂ are as described above for formula (Ig)) with potassium fluoride in DMSO to obtain compounds of formula (Ig), wherein Z is fluoro, X, Y, n, R₁ and R₂ are as described above for formula (Ig), and optionally thereafter forming salts and/or hydrates and/or solvates of the obtained fluoro derivatives of formula (Ig); or
c. with iodomethane in the presence of silver carbonate in a solvent to obtain compounds of formula (Ig), wherein Z is methoxy, X, Y, n, R₁ and R₂ are as described above for formula (Ig), and optionally thereafter forming salts and/or hydrates and/or solvates of the obtained methoxy derivatives of formula (Ig); or
d. with trifluoromethanesulfonic anhydride in a solvent and then with ammonia or mono- and dialkylamines to obtain compounds of formula (Ig), wherein Z is respectivelly amino or monoalkylamino or dialkylamino, X, Y, n, R₁ and R₂ are as described above for formula (Ig), and optionally thereafter forming salts and/or hydrates and/or solvates of the obtained methoxy derivatives of formula (Ig).(Scheme 7).

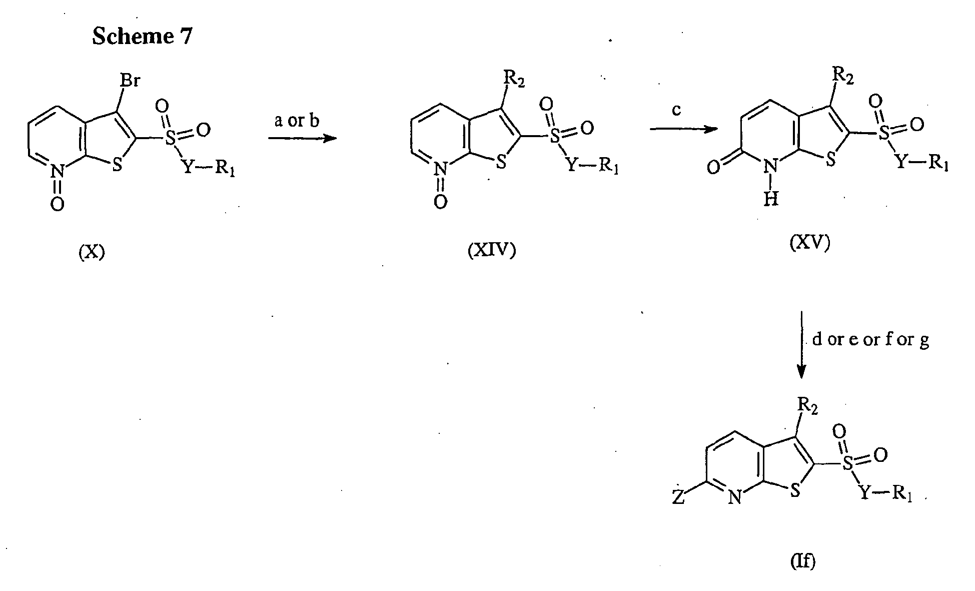

a. R₂-B(OH)₂ (compounds of formula (IX), wherein R₂ is optionally substituted phenyl, heterocyclyl); Na₂CO₃, ethanol, toluene or dimethoxyethane, Pd(PPh₃)₄, 1-5 hours, 20-110 °C;
b. NHR₃R₄ (compounds of formula (XIII), wherein R₃ and R₄ are as described above for formula (If)), 70-200 °C, 1-5 hours, acetonitrile or DMF;
c. Polonovsky type reaction (CF₃CO)₂O, DMF, 0°C-RT;
d. POBr₃, DMF, 80-130 °C and then optionally KF, DMSO, 100-150 °C;
e. POCl₃, DMF, 80-130 °C and then optionally KF, DMSO, 100-150 °C;
f. Iodomethane, AgCO₃, dichloromethane-DMF, 0°C-RT;
g.
   i. (CF₃SO₂)₂O, dichloromethane, pyridine; 0- 5 °C,
   ii. NH₃ or mono- or dialkylamine , 0°C-RT.

Compounds of formula (Ig) containing Z substituents (amino, dialkylamino, metoxy and halogen) are common synthesised by the conversion of a cyclic amide group of formula (XV). Synthesis of compounds of formula (XIV) and (XV) are described in the Hungarian patent Application number HU/P0501166.

Starting from an N-oxide of formula (X) - which was synthesized by the method shown earlier - compounds of formula (Ig) were obtained by a treating the compounds of formula (XIV) with acetic anhydride or trifluoracetic anhydride in dimethylformamide at 0-25 °C temperature by a Polonovsky type reaction using the method of Hartling, R. (J. Het. Chem., 13, 1976, 1197 ).

Compounds of formula (Ig) wherein Z is chloro or bromo may be prepared from the corresponding pyridons of formula (XV). Several methods are available in the art, involving a transformation of this reaction. We applied a reaction using phosphoroxychloride or bromide in a suitable solvent, advantageuosly DMF at 80-130 °C temperature using the method by Dennis W. (J. Org. Chem,; 60, 12 ,1995 ,3750*).*

Compounds of formula (Ig), wherein Z is fluoro may be synthesised from the corresponding chloro or bromo compound of formula (Ig) using the simplified method of Dolle F. (Bioorg. & Med., Chem.; 11, 2003, 5333*).* The reaction was carried out between 100-150°C temperature with potassium fluoride in DMSO.

In the case wherein Z is a methoxy group of formula (Ig) the corresponding compound of formula (XV) was reacted with iodomethane in a halogenated solvent e.g. dichloromethane or chloroform (sometimes 10-20% DMF was used to improve solubility) in the presence of silver carbonate at room temperature applied the process of Ned A. (J. Org. Chem. 26, 2004, 9215*).*

The 6-amino or 6-substituted amino compounds of formula (Ig) wherein Z is amino, or mono- or dialkylamino group was prepared from the compounds of formula (XV) via a two step process using the method of Hisayo I. et. al. (Bioorg. Med. Chem. Lett., 13 ,5, 2003, 913). First the amide function was converted to a triflate using trifluormethanesulfonic-anhydride in a dry halogenated solvent e.g. dichloromethane or chloroform in the presence of pyridine between 0- 5 °C temperature. The crude product was reacted with ammonia in methanol or DMSO or mono- or dialkylamine in DMSO between 0- 25 °C temperature.

According to the present invention a process for the preparation of a compound of formula (Ih): 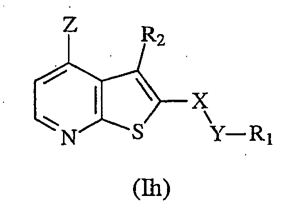 wherein
X represents SO₂;
Y represents a group selected from (CH₂)ₙ, NH, NHCH₂;
n is an integer of 0 to 1;
Z is amino, hydroxy, bromo, chloro, fluoro, methoxy, mono- or dialkyamino;
R₁ is an optionally substituted alkyl, cycloalkyl, phenyl, biphenyl, heterocyclyl;
R₂ is optionally substituted phenyl, heterocyclyl, or
NR₃R₄ group wherein R₃ and R₄ are independently selected from the group of hydrogen or an optionally substituted alkyl, or R₃ and R₄ together with the N atom to which they are attached form an optionally substituted C₅₋₇ heterocyclyl group, containing one or more heteroatom(s),
by reacting a compound of formula (XIV): 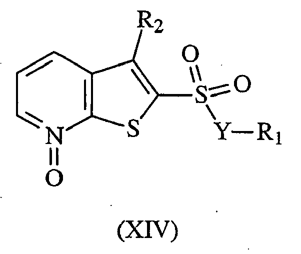 wherein R₁, R₂ and Y are as described above for formula (Ih),
a. with phosphorous(III) oxychloride or phosphorous pentachloride to obtain compounds of formula (Ih), wherein Z is chloro, X, Y, n, R₁ and R₂ are as described above for formula (Ih), or
b. with phosphorous(III) oxybromide to obtain compounds of formula (Ih), wherein Z is chloro, X, Y, n, R₁ and R₂ are as described above for formula (Ih), and optionally thereafter forming salts and/or hydrates and/or solvates of the obtained chloro or bromo derivatives of formula (Ih), wherein Z is chloro or bromo; X, Y, n, R₁ and R₂ are as described above for formula (Ih)); or
   (i) reacting the chloro or bromo derivatives of formula (Ih), wherein Z is chloro or bromo; X, Y, n, R₁ and R₂ are as described above for formula (Ih) with potassium fluoride in DMSO to obtain compounds of formula (Ih), wherein Z is fluoro, X, Y, n, R₁ and R₂ are as described above for formula (Ih), and optionally thereafter forming salts and/or hydrates and/or solvates of the obtained fluoro derivatives of formula (Ih); or
   (ii) reacting the chloro or bromo derivatives formula (Ih), wherein Z is chloro or bromo; X, Y, n, R₁ and R₂ are as described above for formula (Ih) with sodium methylate in methanol to obtain compounds of formula (Ih), wherein Z is methoxy, X, Y, n, R₁ and R₂ are as described above for formula (In), and optionally thereafter forming salts and/or hydrates and/or solvates of the obtained methoxy derivatives of formula (Ih), or
   (iii) reacting the chloro or bromo derivatives of formula (Ih), wherein Z is chloro or bromo; X, Y, n, R₁ and R₂ are as described above for formula (Ih)) with sodium acetate in acetic acid to obtain compounds of formula (Ih), wherein Z is hydroxy, X, Y, n, R₁ and R₂ are as described above for formula (Ih), and optionally thereafter forming salts and/or hydrates and/or solvates of the obtained hydroxy derivatives of formula (Ih), or
   (iv) reacting the chloro or bromo derivatives formula (Ih), wherein Z is chloro or bromo; X, Y, n, R₁ and R₂ are as described above for formula (Ih) with trifluoromethanesulfonic anhydride in a solvent and then with ammonia or mono- and dialkylamines to obtain compounds of formula (Ih), wherein Z is respectivelly amino or monoalkylamino or dialkylamino, X, Y, n, R₁ and R₂ are as described above for formula (Ih), and optionally thereafter forming salts and/or hydrates and/or solvates of the obtained methoxy derivatives of formula (Ih), wherein Z is respectivelly amino or monoalkylamino or dialkylamino; or
   (v) reacting the chloro or bromo derivatives of formula (Ih), wherein Z is chloro or bromo; X, Y, n, R₁ and R₂ are as described above for formula (Ih)) with sodium azide in DMF and water to obtain compounds of formula (Ih), wherein Z is azido, X, Y, n, R₁ and R₂ are as described above for formula (Ih), then reacting the obtained azido derivatives with sodium borohydride in a solvent to obtain compounds of formula (Ih), wherein Z is amino, X, Y, n, R₁ and R₂ are as described above for formula (Ih), and optionally thereafter forming salts and/or hydrates and/or solvates of the obtained amino derivatives of formula (Ih) (Scheme 8).

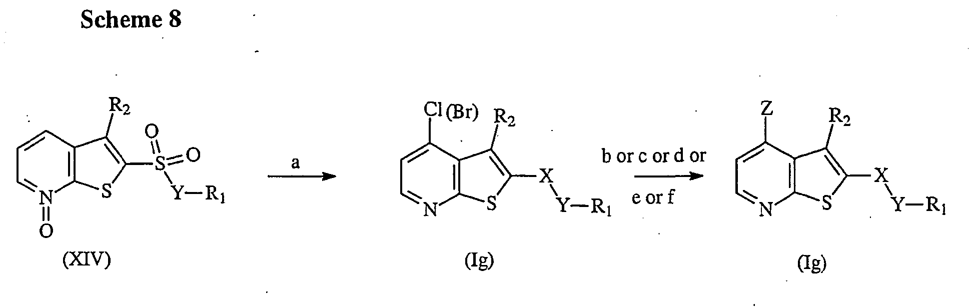

a. POCl₃, POBr₃, PCl₅, 50-150 °C, neat or in DMF;
b. KF, DMSO or DMF, 100-150 °C
c. NaOCH₃, methanol, 0-60°C
d. CH₃COONa, CH₃COOH, 80-100 °C
e. NH₃ or mono- or dialkylamine or DMF, 100-150°C, sealed tube or microwave assisted reaction
f.
   i. NaN₃, DMF-water, 80-100 °C,
   ii. NaBH₄, methanol 0-30°C

Compounds of formula (Ih) wherein Z is chloro or bromo was prepared from a N-oxide derivative of formula (XIV) by the method of Sakamoto et.al. (Chem. Pharm. Bull. 36; 1988; 2244). The reaction was carried out with an appropriate halogen containing phosphorus reagent neat or an suitable solvent e.g. DMF between 50-150 °C temperature. The procedure results two halogen isomers at the 4 and 6 position of the thienopyridin ring, which can be separated by crystallization or chromatography.

The 4-halogen (chloro or bromo) substituted compounds of formula (Ih) may be be transformed to 4-hydroxyl derivatives with application of the the procedure of Fujimoto etal.(Pharm Bull.; 2, 1954, 131*)* using acetic acid and sodium acetate between 80-100 °C temperature.

Compounds with 4-amino function of formula (Ih) was prepared via a two step process: first compounds of formula (Ih), wherein Z is chloro or bromo, was reacted with sodium azide producing azido derivatives of compounds of formula (Ih), wherein Z is an azide group, that is reduced to the corresponding amino derivatives with an appropriate borohydride e.g. with sodium borohydride in methanol or ethanol.

An alternative method - for the preparation of compound wherein Z is amino or momoalkyl, or dialkyl substituted amino of formula (Ih) - was that the corresponding 4-halogen derivatives of compounds of formula (Ih) was treated with ammonia, mono or disubstituted amine or dimethylformamide using the method of Gawley R. E. et.al. (Tetrahedron Lett., 45, 4, 2004, 757) and Prem. M. S. (Synth. Comm. 34, 16, 2004, 2925). The procedure was carried out in few cases in sealed tube or by a microwave assisted reaction between 100-150 oC temperature.

Compounds of formula (Ih) wherein Z is methoxy group may be prepared by the well known method described by Neumann K. (Chem. Ber.; 48, 1915, 961). Compound of formula (Ih) wherein Z is chloro or bromo was reacted with sodium methoxyde in methanol between 0-60°C temperature.

The fluoronation reaction starting from the corresponding chloro or bromo compound of formula (Ih) can be done with potassium fluoride in a suitable solvent e.g. DMF or DMSO between 100-150 °C temperature applied the simplified method of Hochberg R. B.;(J. Med. Chem.; 45, 2002, 5397) resulting the 4-fluoro compounds of formula (Ih).

All work-up of the reaction mixture can be carried out by different methods well known in the synthetic chemistry process. The products can be purified by crystallization or by column or flash cromatography.

### Biological test methods

### MGluR1 receptor binding test

MGluR1 receptor binding testes were performed according to modified method of Lavreysen et al. (Mol.Pharm., 2003, 63, 1082). Based on the high homology between the human and rat mGluR1 receptors, rat cerebellar membrane preaparation was used to determine the binding characteristics of reference compounds and novel compounds to the rat mGluR1. As radioligand [3H]R214127 (3 nM) was used and the nonspecific binding was determined in the presence of 1 µM of R214127.

IC-50 values were determined from displacement curves by nonlinear regression analysis and were converted by equation method of Cheng and Prusoff (Biochem. Pharmacol., 1973, 22, 3099) to Ki values.

### MGluR5 receptor binding tests

MGluR5 receptor binding was determined according to Gasparini et.al. (Bioorg. Med. Chem. Lett. 2000, 12:407-409) with modifications. Rat cerebro-cortical membrane preparation was used to determine the binding characteristics of reference compounds and novel compounds to the rat mGluR5. The A18 cell line expressing hmGluR5a (purchased from Euroscreen) was used to determine binding characteristics of the chemical compounds to the human mGluR5a receptor. As radioligand [3H]-M-MPEP (2 nM) was used. The nonspecific binding was determined in the presence of 10 µM M-MPEP.

### Assessment of functional activity

### Cell cultures for native rat mGluR5 and mGluR1 receptors

Functional potency at native rat mGluR5 and mGluR1 receptors was estimated using primary neocortical cell cultures derived from 17 day old Charles River rat embryos and primary cerebellar cell cultures derived from 4-day old Wistar rats, respectively (for the details on the preparation of neural cell cultures see Johnson, M.I.; Bunge, R.P. (**1992**): Primary cell cultures of peripheral and central neurons and glia. In: Protocols for Neural Cell Culture, eds: Fedoroff, S.; Richardson A., The Humana Press Inc., 51-77). After isolation the cells were plated onto standard 96-well microplates and the cultures were maintained in an atmosphere of 95% air-5% CO₂ at 37 °C. The neocortical and cerebellar cultures were used for the calcium measurements after 5-7 and 3-4 days in vitro, respectively.

### Cell cultures for recombinant human mGluR5a receptors

Chinese hamster ovary (CHO) cells stably expressing recombinant human mGluR5a (CHO-mGluR5a, purchased from Euroscreen) receptors were cultured in F12 medium containing 10% FCS, 1% antibiotic antimycotic solution, 400 µg/ml G418, 250 µg/ml zeocin, 5 µg/ml puromycin. Cells were kept at 37 °C in a humidified incubator in an atmosphere of 5% CO₂/95% air and were passaged three times a week. Cells were plated at 2.5-3.5×104 cell/well on standard 96-well microplates, receptor expression was induced by adding 600 ng/ml doxycycline on the next day. The calcium measurements were carried out 16-24 hours after the addition of the inducing agent.

### Fluorimetric measurement of cytosolic calcium concentration

Measurements of cytosolic calcium concentration ([Ca²⁺]ᵢ) were carried out on primary neocortical and cerebellar cultures, and on CHO-mGluR5a cells stably expressing human mGluR5a receptors. Cells were grown in standard 96-well microplates and before the measurement were loaded with a fluorescent Ca²⁺-sensitive dye, fluo-4/AM (2 µM): the neutral cultures were loaded in their growth medium, CHO-mGluR5a cells were loaded in assay buffer (145 mM NaCl, 5 mM KCl, 2 mM MgCl₂, 2 mM CaCl₂, 10 mM HEPES, 20 mM D-glucose, 2 mM probenecid, pH=7.4) supplemented with 2 mM Na-pyruvate and 30 µg/ml glutamate-pyruvate transaminase (in case of CHO-mGluR5a cells these supplements were also present during the course of the [Ca²⁺]ᵢ measurements). Loading was done by incubating the cells with 100 µl/well dye solution at 37 °C in a humidified incubator in an atmosphere of 5% CO2/95% air for 40-120 min. To stop dye loading cells were washed twice with assay buffer. After washing, various concentrations of the test compounds (diluted in assay buffer from a DMSO or a dimethylformamide (DMF) stock solution, final DMSO/DMF concentration was <0.1 %) or buffer were added to each well depending on the experimental setup. In the case of neocortical cultures the assay buffer also contained TTX (0.5 µM, to suppress spontaneous oscillations of [Ca2+]i, in the case of cerebellar cultures probenecid was substituted with sulfinpyrazone (0.25 mM).

After incubation at 37 °C for 10-20 min. baseline and agonist-evoked changes of [Ca2+]i were measured column by column with a plate reader fluorimeter (FlexStation II, Molecular Devices). Excitation and detection of emission was carried out from the bottom of the plate. The whole measurement process was performed at 37 °C and was controlled by custom software. Inhibitory potency of the test compounds was assessed by measuring the reduction in the agonist-evoked [Ca²⁺]ᵢ -elevation in the presence of different concentrations of the compounds. DHPG was used as agonist for all three cultures, the concentration was 20 and 100 µM for neocortical and cerebellar cultures, respectively. In the case of CHO-mGluR5a cells DHPG was applied at an EC80 concentration, the EC80-values were derived from daily determined dose-response curves. Fluorescence data were expressed as ΔF/F (fluorescence change normalized to baseline).

All treatments on a single plate were measured in multiple wells. Data from all wells with the same treatment were averaged and the average values were used for analysis. Inhibitory potency of a compound at a single concentration point was expressed as percent inhibition of the control agonist response. Sigmoidal concentration-inhibition curves were fitted to the data (derived from at least three independent experiments) and IC50-values were determined as the concentration that produces half of the maximal inhibition caused by the compound. Raw fluorescence data were analyzed using Soft Max Pro (Molecular Devices), curve fitting was done with GraphPad Prism.

### Results

Compounds of formula (I) of the present invention showed affinity for both rat and human mGluR1 and mGluR5 receptors and proved to be functional antagonists that are they inhibited functional responses elicited by stimulation of mGluR5 receptors.

**Table**

| **Comp. No.** | **Structure** | **(M+H)⁺ or M⁺** | **mGlu 5 Kᵢ (nM)** | **mGlu1 Kᵢ (nM)** | **¹H NMR data** |
|---|---|---|---|---|---|
| **1** | 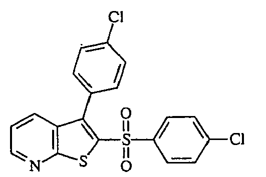 | 421,2 | * | *** | (300 MHz, DMSO-*d₆*, 30 °C): 8.82 (dd, *J* = 4.6, 1.7 Hz, 1H); 7.82 (dd, *J* = 8.3, 1.7 Hz, 1H); 7.63-7.50 (m, 7H); 7.32-7.23 (m, 2H). |
| **2** | 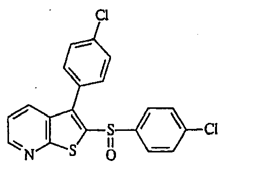 | 405,1 | * | * | (500 MHz, CDCl₃, 30 °C): 8.64 (dd, *J* = 4.6, 1.6 Hz, 1H); 7.89 (dd, *J* = 8.2, 1.6 Hz, 1H); 7.59-7.55 (m, 2H); 7.54-7.50 (m, 2H); 7.50-7.43 (m, 4H); 7.35 (dd, *J* = 8.2, 4.6 Hz, 1H). |
| **3** | 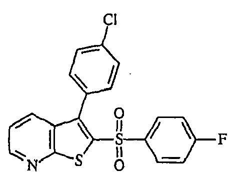 | 404,1 | * | *** | (300 MHz, CDCl₃, 30 °C): 8.72 (dd, *J* = 4.6, 1.7 Hz, 1H); 7.70 (dd, *J* = 8.3, 1.7 Hz, 1H); 7.59-7.50 (m, 2H); 7.48-7.41 (m, 2H); 7.34 (dd, *J* = 8.3, 4.6 Hz, 1H); 7.21-7.14 (m, 2H); 7.07-6.97 (m, 2H). |
| **4** | 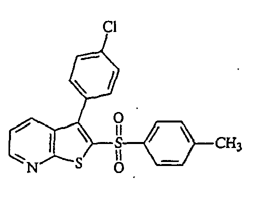 | 400,2 | * | ** | (300 MHz, DMSO-*d₆*, 30 °C): 8.79 (dd, *J* = 4.6, 1.6 Hz, 1H); 7.80 (dd, *J* = 8.3, 1.6 Hz, 1H): 7.60-7.52 (m, 3H); 7.47-7.41 (m, 2H); 7.36-7.29 (m, 2H); 7.28-7.22 (m, 2H); 2.36 (s, 3H). |
| **5** | 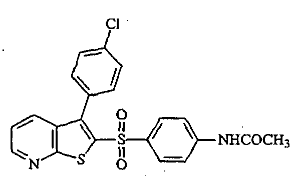 | 445.0 | *** | *** | (300 MHz, DMSO-*d₆*, 30 °C): 10.39 (s, 1H); 8.79 (dd, *J* = 4.6, 1.6 Hz, 1H); 7.80 (dd, *J* = 8.3, 1.6 Hz, 1H); 7.71-7.63 (m, 2H); 7.60-7.50 (m, 3H); 7.50-7.43 (m, 2H); 7.32-7.24 (m, 2H); 2.08 (s, 3H). |
| **6** | 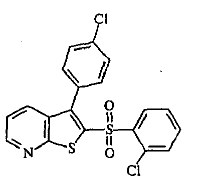 | 421,2 | ** | | (300 MHz, DMSO-*d₆*, 30 °C): 8.84 (dd, *J* = 4.6, 1.7 Hz, 1H); 7.84 (dd, *J* = 8.3, 1.7 Hz, 1H); 7.69-7.61 (m, 1H); 7.60-7.53 (m, 3H); 7.40-7.35 (m, 2H); 7.32 (ddd, J = 8.1, 7.4, 1.3 Hz, 1H); 7.17-7.09 (m, 2H). |
| **7** | 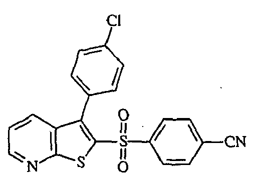 | 411,2 | * | *** | (300 MHz, CDCl₃, 30 °C): 8.75 (dd, *J* = 4.5, 1.6 Hz, 1H); 7.72 (dd, *J* = 8.2, 1.6 Hz, 1H); 7.67-7.61 (m, 4H); 7.50-7.43 (m, 2H); 7.36 (dd, *J* = 8.2, 4.5 Hz, 1H); 7.21-7.13 (m, 2H). |
| **8** | 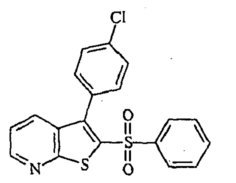 | 386,0 | * | *** | (300 MHz, CDCl₃, 30 °C): 8.71 (dd, *J* = 4.6, 1.7 Hz, 1H); 7.68 (dd, *J* = 8.2, 1.7 Hz, 1H); 7.58-7.48 (m, 3H); 7.45-7.39 (m, 2H); 7.38-7.29 (m, 3H); 7.18-7.12 (m, 2H). |
| **9** | 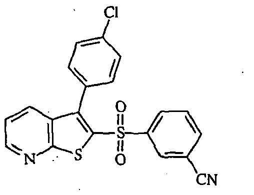 | 411,2 | * | * | (300 MHz, CDCl₃, 30 °C): 8.75 (dd, *J* = 4.6, 1.6 Hz, 1H); 7.80-7.75 (m, 2H); 7.72 (dd, *J* = 8.3, 1.6 Hz, 1H); 7.69-7.66 (m, 1H); 7.55-7.45 (m, 3H); 7.36 (dd, *J* = 8.3, 4.5 Hz, 1H); 7.20-7.13 (m, 2H). |
| **10** | 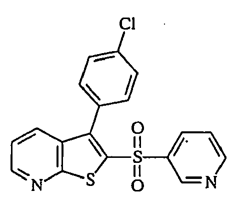 | 387,2 | * | *** | (500 MHz, DMSO-*d₆*, 25 °C): 8.85-8.80 (m, 2H); 8.58 (dd, *J* = 2.5, 0.6 Hz, 1H); 7.97 (ddd, *J* = 8.2, 2.5, 1.6 Hz, 1H); 7.83 (dd, *J* = 8.3, 1.6 Hz, 1H); 7.59-7.52 (m, 4H); 7.30-7.26 (m, 2H). |
| **11** | 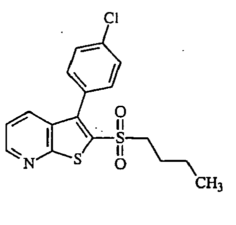 | 366,2 | ** | *** | (300 MHz, CDCl₃, 30 °C): 8.77 (dd, *J* = 4.6, 1.7 Hz, 1H); 7.82 (dd, *J* = 8.3, 1.7 Hz, 1H); 7.56-7.51 (m, 2H); 7.48-7.43 (m, 2H); 7.40 (dd, *J* = 8.3, 4.6 Hz, 1H); 2.97-2.87 (m, 2H); 1.70-1.57 (m, 2H); 1.38-1.20 (m, 2H); 0.81 (t, *J* = 7.3 Hz, 3H). |
| **12** | 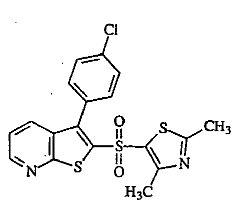 | 421,2 | ** | *** | (300 MHz, DMSO-*d₆*, 30 °C): 8.83 (dd, *J* = 4.6, 1.6 Hz, 1H); 7.83 (dd, *J* = 8.3, 1.6 Hz, 1H); 7.62-7.53 (m, 3H); 7.38-7.31 (m, 2H); 2.59 (s, 3H); 2.25 (s, 3H). |
| **13** | 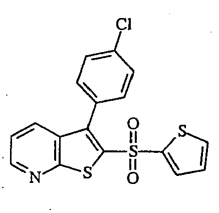 | 391,9 | ** | *** | (300 MHz, DMSO-*d₆*, 30 °C): 8.81 (dd, *J* = 4.6, 1.6 Hz, 1H); 8.10 (dd, J = 5.0, 1.4 Hz, 1H); 7.84 (dd, *J* = 8.3, 1.6 Hz, 1H); 7.63-7.58 (m, 2H); 7.56 (dd, *J* = 8.3, 4.6 Hz, 1H); 7.45 (dd, *J* = 3.9, 1.4 Hz, 1H); 7.41-7.34 (m, 2H); 7.15 (dd, *J* = 5.0, 3.9 Hz, 1H). |
| **14** | 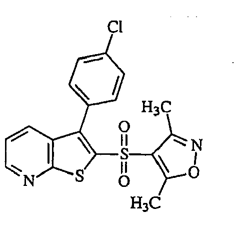 | 405,2 | *** | *** | (500 MHz, DMSO-*d₆*, 25 °C): 8.84 (dd, *J* = 4.6, 1.6 Hz, 1H); 7.83 (dd, *J* = 8.3, 1.6 Hz, 1H); 7.64-7.60 (m, 2H); 7.58 (dd, *J* = 8.3, 4.6 Hz, 1H); 7.40-7.35 (m, 2H); 2.20 (s, 3H); 2.05 (s, 3H). |
| **15** | 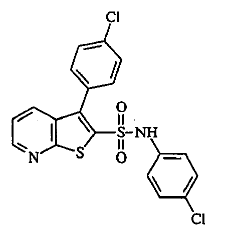 | | ** | *** | (300 MHz, DMSO-*d₆*, 30 °C): 10.74 (s, 1H); 8.76 (dd, *J* = 4.6, 1.6 Hz, 1H); 7.77 (dd, *J* = 8.3, 1.6 Hz, 1H); 7.56-7.48 (m, 3H); 7.31-7.21 (m, 4H); 7.01-6.93 (m,2H). |
| **16** | 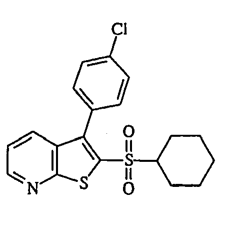 | 392,2 | *** | *** | (300 MHz, CDCl₃, 30 °C): 8.75 (dd, *J* = 4.5, 1.6 Hz, 1H); 7.80 (dd, *J* = 8.3, 1.6 Hz, 1H); 7.55-7.48 (m, 2H); 7.46-7.40 (m, 2H); 7.39 (dd, *J* = 8.3, 4.5 Hz, 1H); 2.68 (tt, *J* = 12.0, 3.4 Hz, 1H); 1.98-1.72 (m, 4H); 1.70-1.36 (m, 3H); 1.20-0.96 (m, 3H). |
| **17** | 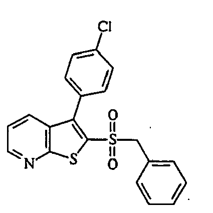 | | ** | *** | (300 MHzDMSO-*d₆*, 30 °C): 8.81 (dd, *J* = 4.5, 1.6 Hz, 1H); 7.86 (dd, *J* = 8.3, 1.6 Hz, 1H); 7.60-7.54 (m, 3H); 7.37-7.26 (m, 5H); 7.16-7.11 (m, 2H); 4.61 (s, 2H). |
| **18** | 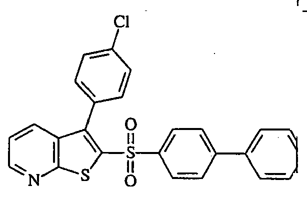 | 462,1 | *** | *** | (300 MHz, CDCl₃, 30 °C): 8.71 (dd, *J* = 4.6, 1.7 Hz, 1H); 7.69 (dd, *J* = 8.3, 1.7 Hz, 1H); 7.63-7.50 (m, 6H); 7.50-7.38 (m, 5H); 7.32 (dd, J = 8.3, 4.6 Hz, 1H); 7.22-7.15 (m, 2H). |
| **19** | 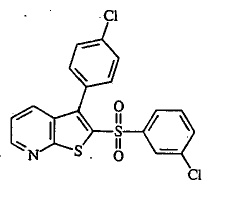 | 421,2 | * | *** | (300 MHz, DMSO-*d₆*, 30 °C): 8.83 (dd, *J* = 4.6, 1.6 Hz, 1H); 7.83 (dd, *J* = 8.3, 1.6 Hz, 1H); 7.75 (dt, J = 7.2, 2.0 Hz, 1H); 7.62-7.51 (m, 5H); 7.29-7.21 (m, 3H). |
| **20** | 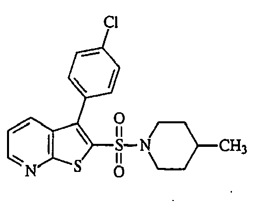 | | ** | | (300 MHz, DMSO-*d₆*, 30 °C): 8.79 (dd, *J* = 4.6, 1.6 Hz, 1H); 7.84 (dd, *J* = 8.3, 1.6 Hz, 1H); 7.64-7.50 (m, 5H); 3.42-3.28 (m, 2H); 2.54-2.39 (m, 2H); 1.63-1.49 (m, 2H); 1.44-1.22 (m, 1H); 1.06-0.86 (m, 2H); 0.82 (d, *J* = 6.6 Hz, 3H). |
| **21** | 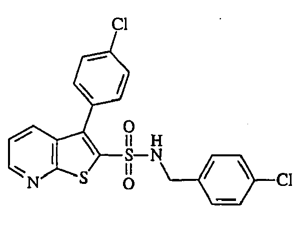 | | *** | *** | (300 MHz, DMSO-*d₆*, 30 °C): 8.77 (dd, *J* = 4.6, 1.6 Hz, 1H); 8.72-8.55 (brm, 1H), 7.83 (dd, *J* = 8.3, 1.6 Hz, 1H); 7.63-7.56 (m, 2H); 7.54 (dd, *J* = 8.3, 4.6 Hz, 1H); 7.52-7.46 (m, 2H); 7.36-7.28 (m, 2H); 7.23-7.16 (m, 2H); 4.02 (s, 2H). |
| **22** | 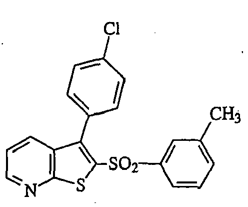 | 400,2 | * | | |
| **23** | 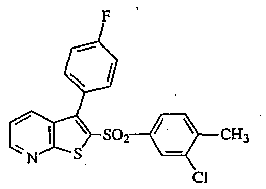 | 418,2 | * | | |
| **24** | 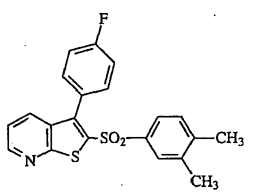 | 397,5 | * | | |
| **25** | 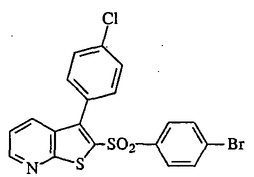 | MS(EI) 463 | * | | |
| **26** | 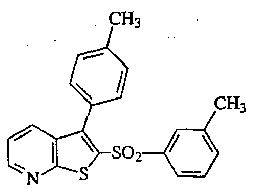 | 380,2 | * | | |
| **27** | 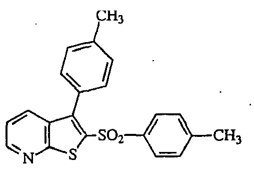 | 380,2 | * | | |
| **28** | 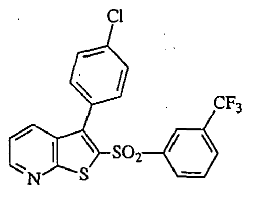 | MS(EI) 453 | * | *** | |
| **29** | 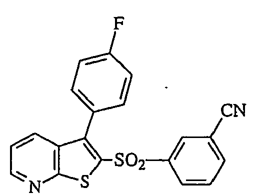 | 395,2 | * | | |
| **30** | 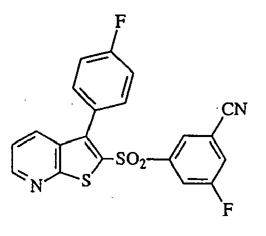 | MS (EI): 412 | * | | (500 MHz, DMSO-*d₆*, 25 °C): 8.84 (dd, *J* = 4.5, 1.6 Hz, 1H); 8.23-8.19 (m, 1H); 7.83 (dd, J = 8.3, 1.6 Hz, 1H); 7.64-7.59 (m, 2H); 7.57 (dd, J = 8.3, 4.5 Hz, 1H); 7.37-7.31 (m, 2H); 7.31-7.26 (m, 2H). |
| **31** | 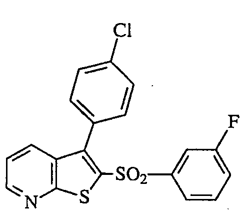 | 404,1 | * | | |
| **32** | 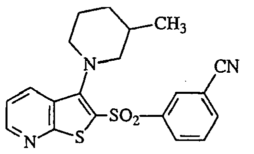 | MS(EI) 397 | * | | |
| **33** | 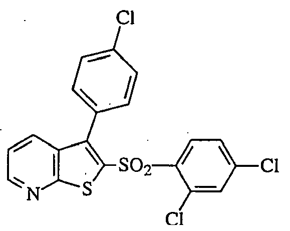 | 455,1 | ** | *** | |
| **34** | 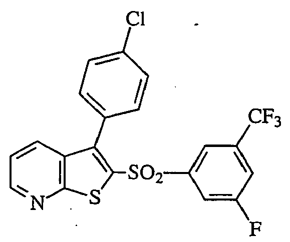 | 472,1 | * | | |
| **35** | 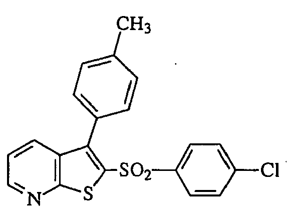 | 400,2 | * | *** | |
| **36** | 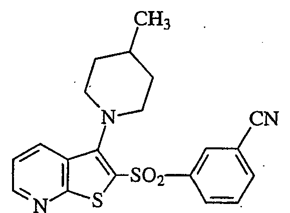 | MS(FIB) 398 | * | * | |
| **37** | 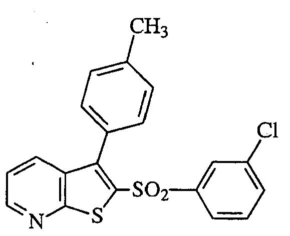 | 400,2 | * | | |
| **38** | 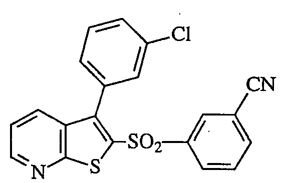 | 411,2 | * | * | |
| **39** | 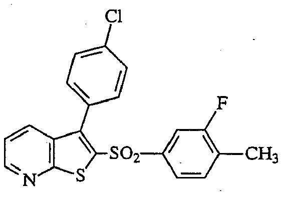 | 418,2 | * | | |
| **40** | 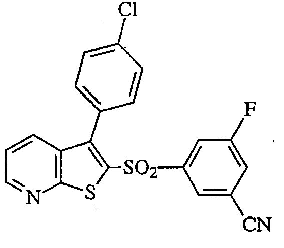 | 429,1 | * | ** | |
| **41** | 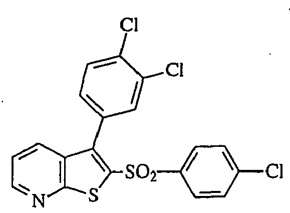 | 456,1 | * | | |
| **42** | 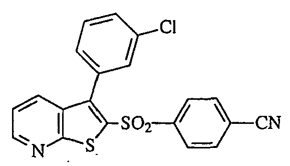 | 411,2 | * | | |
| **43** | 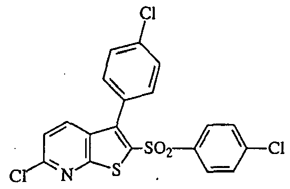 | 456,0 | * | | |
| **44** | 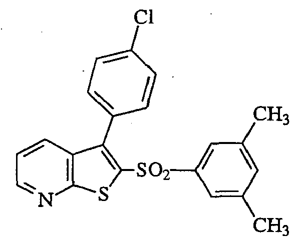 | 414,2 | * | | |
| **45** | 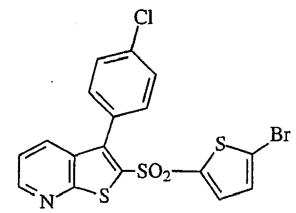 | 471,9 | * | | |
| **46** | 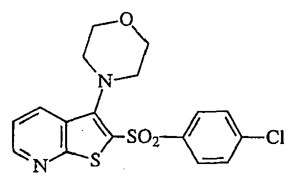 | MS(EI) 394 | * | | |
| **47** | 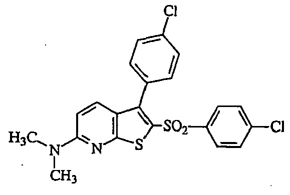 | 464,1 | ** | | |
| **48** | 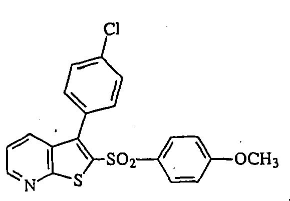 | 416,2 | * | * | |
| **49** | 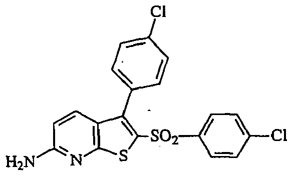 | 436,6 | * | | |
| **50** | 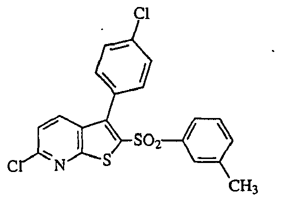 | 435,1 | * | | |
| **51** | 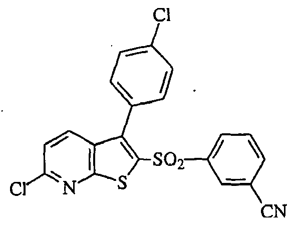 | 446,2 | *** | | |
| **52** | 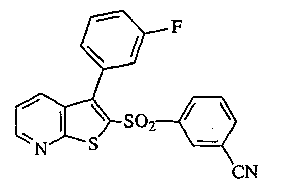 | 395,2 | * | * | |
| **53** | 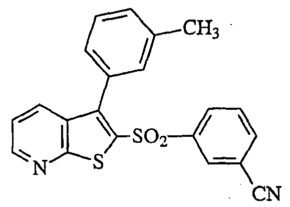 | MS(EI) 390 | * | ** | |
| **54** | 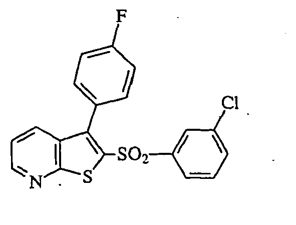 | 404,3 | * | | |
| **55** | 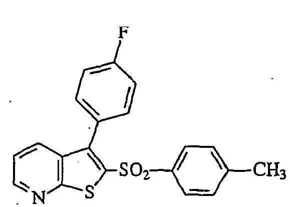 | 384,0 | * | | |
| **56** | 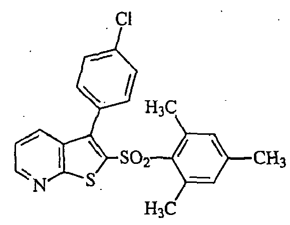 | 428,2 | *** | | |
| **57** | 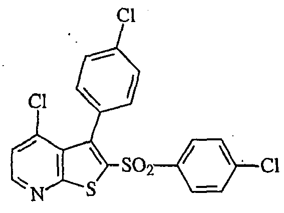 | 456,0 | * | | |
| **58** | 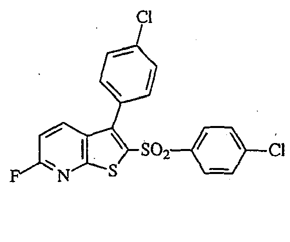 | (FAB) 438 | * | | (500 MHz, DMSO-*d₆*, 25 °C): 7.99 (dd, J = 8.8, 7.5 Hz, 1H); 7.61-7.51 (m, 6H); 7.35 (dd, J = 8.8, 1.4 Hz, 1H); 7.31-7.24 (m, 2H). |
| **59** | 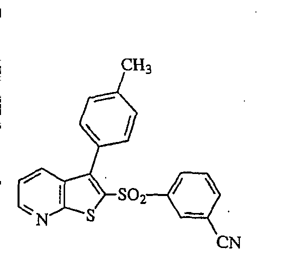 | MS(FIB) 391 | * | * | |
| **60** | 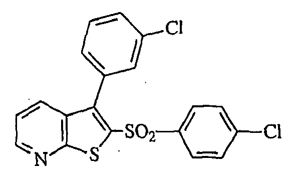 | 421,1 | * | | |
| **61** | 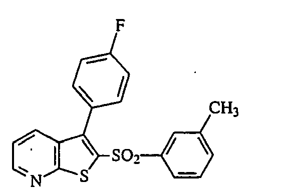 | 384,0 | * | | |
| **62** | 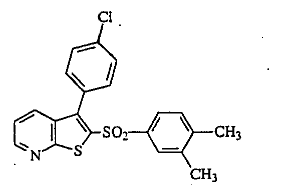 | MS(EI) 413 | * | | |
| **63** | 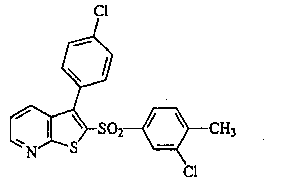 | MS(EI) 433 | * | | |
| **64** | 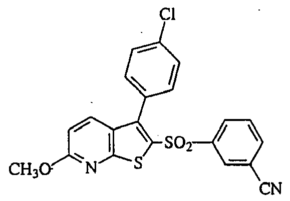 | 441,2 | * | | |
| **65** | 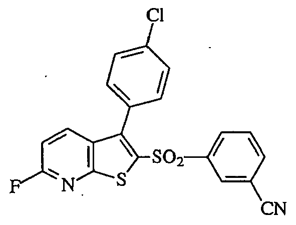 | 429,2 | * | | (300 MHz, DMSO-*d₆*, 30 °C): 8.14 (ddd, J = 7.7, 1.5, 1.1 Hz, 1H); 8.00 (dd, J = 8.8, 7.5 Hz, 1H); 7.86 (ddd, J = 8.0, 2.0, 1.1 Hz, 1H); 7.75-7.65 (m, 2H); 7.59-7.51 (m, 2H); 7.36 (dd, J = 8.8, 1.6 Hz, 1H); 7.27-7.19 (m, 2H). |
| **66** | 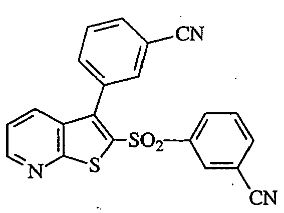 | MS(FIB) 402 | * | | |
| **67** | 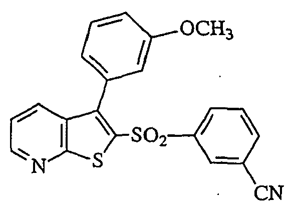 | MS(FIB) 407 | * | * | |
| **68** | 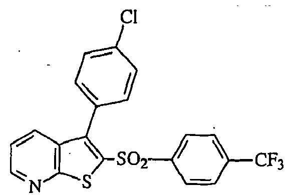 | 454,2 | * | | |
| **69** | 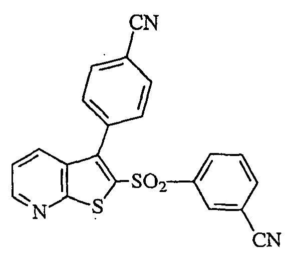 | MS(EI) 401 | * | | |
| **70** | 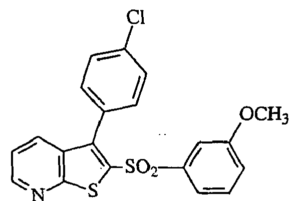 | 416,2 | * | | |
| **71** | 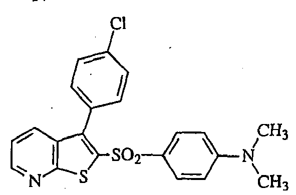 | 429,2 | * | | |
| **72** | 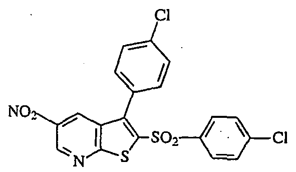 | 466,1 | ** | | |
| **73** | 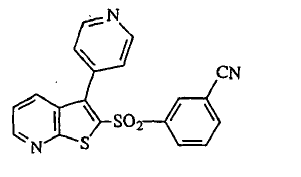 | MS(EI) 377 | * | | |
| **74** | 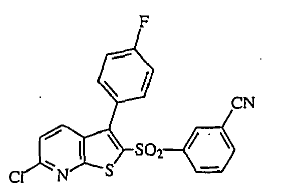 | MS(EI) 428 | * | | |
| **75** | 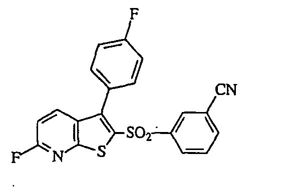 | MS (EI): 412 | * | *** | (500 MHz, DMSO-*d₆*, 25 °C): 8.13 (dm, J = 7.9 Hz, 1H); 7.99 (dd, J = 8.8, 7.6 Hz, 1H); 7.82 (dm, J = 8.1 Hz, 1H); 7.72-7.66 (m, 2H); 7.36 (dd, J = 8.8, 1.4 Hz, 1H); 7.36-7.29 (m, 2H); 7.29-7.22 (m, 2H). |
| **76** | 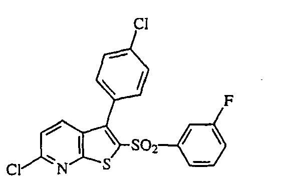 | MS(EI) 437 | ** | | |
| **77** | 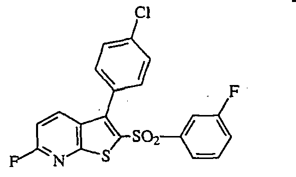 | MS(EI) 421 | * | | |
| **78** | 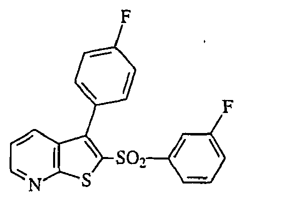 | 388,2 | * | *** | |
| **79** | 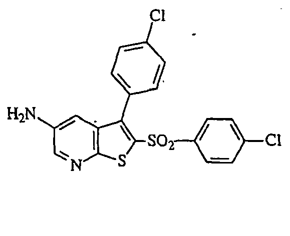 | MS (EI) 434 | * | * | (300 MHz, CDCl₃, 30 °C): 8.38-8.27 (brm, 1H); 7.50-7.38 (m, 4H); 7.36-7.28 (m, 2H); 7.19-7.09 (m, 2H); 6.96-6.87 (brm, 1H); 2.52 (vbrs, 2H). |
| | | | | | |
| **80** | 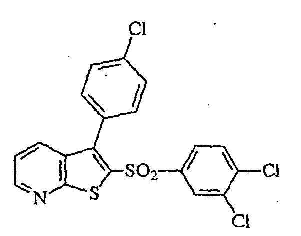 | 455,9 | * | ** | |
| **81** | 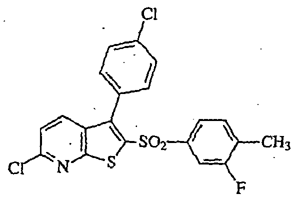 | MS(EI) 451 | * | | |
| **82** | 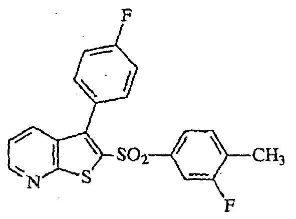 | *** | * | *** | |
| **83** | 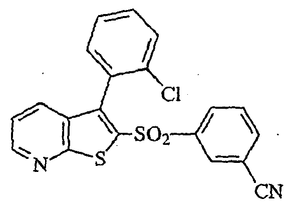 | 411,2 | * | * | |
| **84** | 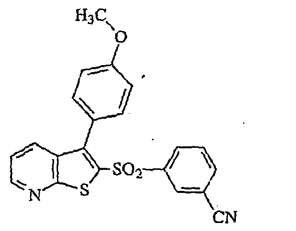 | MS(FIB) 407 | * | | |
| **85** | 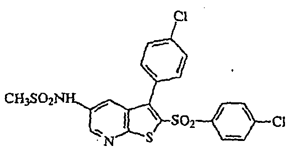 | 514,2 | * | | |
| **86** | 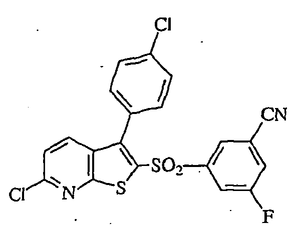 | MS (EI): 462 | * | | (500 MHz, DMSO-*d₆*, 25 °C): 8.22 (ddd, *J* = 8.4, 2.3, 1.3 Hz, 1H); 7.88 (d, *J* = 8.7 Hz, 1H); 7.67-7.61 (m, 3H); 7.59-7.54 (m, 2H); 7.27-7.22 (m, 2H). |
| **87** | 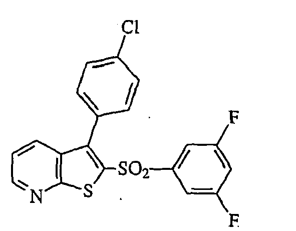 | 422,2 | * | | |
| **88** | 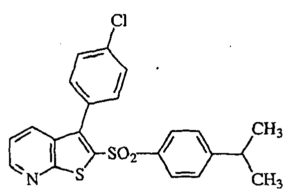 | 428,1 | * | | |
| **89** | 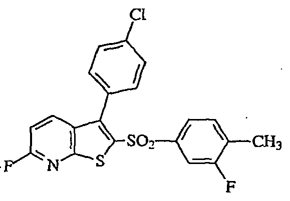 | MS (EI): 435 | * | *** | (500 MHz, DMSO-*d₆*, 25 °C): 7.99 (dd, *J* = 8.7, 7.6 Hz, 1H); 7.60-7.55 (m, 2H); 7.45 (t, *J* = 7.7 Hz, 1H); 7.35 (dd, *J* = 8.7, 1.3 Hz, 1H); 7.30 (dd, *J* = 8.0, 1.8 Hz, 1H); 7.29-7.25 (m, 2H); 7.12 (dd, *J* = 9.0, 1.8 Hz, 1H); 2.27 (d, *J* = 1.5 Hz, 3H). |
| **90** | 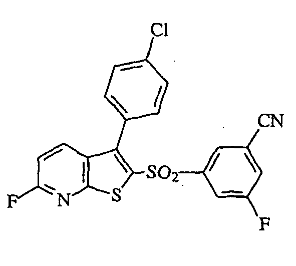 | MS (EI): 446 | * | *** | (500 MHz, DMSO-*d₆*, 25 °C): 8.23 (dm, *J* = 8.4 Hz, 1H); 8.03 (dd, *J* = 8.8, 7.5 Hz, 1H); 7.67-7.61 (m, 2H); 7.59-7.54 (m, 2H); 7.38 (dd, *J* = 8.8, 1.4 Hz, 1H); 7.28-7.22 (m, 2H). |
| **91** | 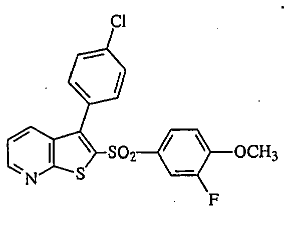 | MS (EI): 433 | * | ** | (500 MHz, DMSO-*d₆*, 25 °C): 8.84 (dd, *J* = 4.5, 1.6 Hz, 1H); 8.23-8.19 (m, 1H); 7.83 (dd, *J* = 8.3, 1.6 Hz, 1H); 7.64-7.59 (m, 2H); 7.57 (dd, *J* = 8.3, 4.5 Hz, 1H); 7.37-7.31 (m, 2H); 7.31-7.26 (m, 2H); 3.90 (s, 3H). |
| **92** | 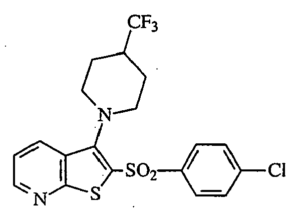 | MS(EI) 460 | * | | |
| **93** | 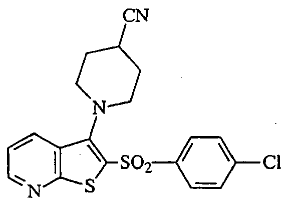 | MS(EI) 417 | ** | | |
| **94** | 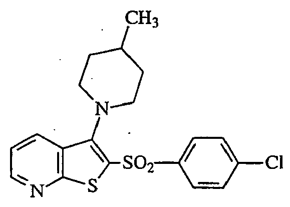 | MS(EI) 406 | * | | |
| **95** | 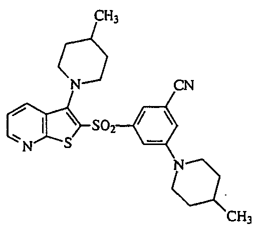 | 496,2 | ** | | |
| **96** | 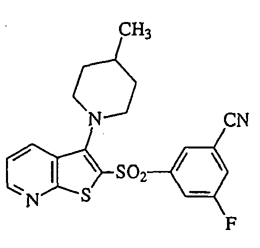 | 416,1 | * | | (500 MHz, DMSO-*d₆*, 25 °C): 8.77 (dd, *J* = 4.5, 1.5 Hz, 1H); 8.55 (dd, *J* = 8.4, 1.5 Hz, 1H); 8.36-8.29 (m, 2H); 8.19 (ddd, *J* = 7.8,' 2.4, 1.6 Hz, 1H); 7.54 (dd, *J* = 8.4, 4.5 Hz, 1H); 3.27-3.18 (m, 2H); 3.03-2.95 (m, 2H); 1.65-1.49 (m, 3H); 1.10-0.99 (m, 2H); 0.96 (d, *J*= 6.4 Hz, 3H). |
| **97** | 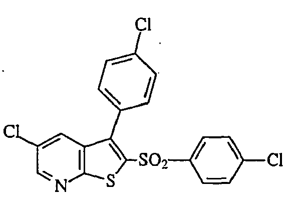 | MS 453 | * | | (500 MHz, CDCl₃, 25 °C): 8.38 (d, *J* = 2.3 Hz, 1H); 7.64 (d, *J* = 2.3 Hz, 1H); 7.50-7.41 (m, 4H); 7.36-7.30 (m, 2H); 7.19-7.13 (m, 2H). |
| **98** | 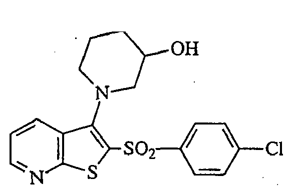 | MS(EI) 408 | * | | |
| **99** | 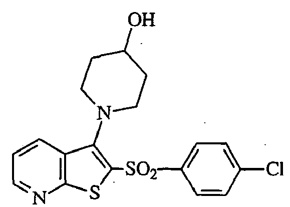 | MS(EI) 408 | ** | | |
| **100** | 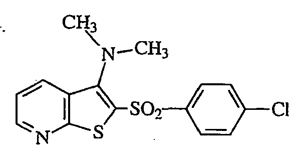 | 353,1 | * | | |
| **101** | 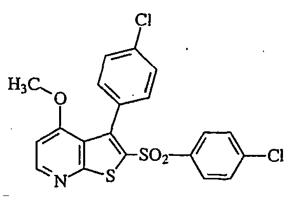 | 451,2 | * | | |
| **102** | 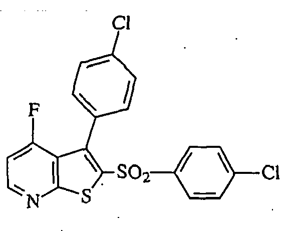 | - | * | | (500 MHz, DMSO-*d₆*, 25 °C): 8.83 (dd, *J* = 7.4, 5.4 Hz, 1H); 7.61-7.55 (m, 2H); 7.52-7.44 (m, 4H); 7.44 (dd, *J* = 11.2, 5.4 Hz, 1H); 7.28-7.23 (m, 2H). |
| **103** | 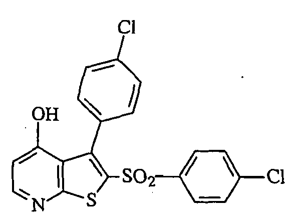 | 437,2 | ** | | |
| **104** | 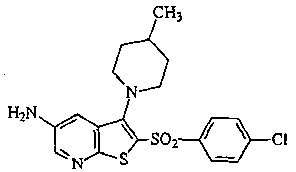 | 422,2 | * | | |
| **105** | 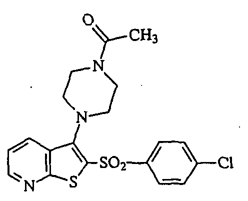 | MS(FIB) 436 | *** | | |
| **106** | 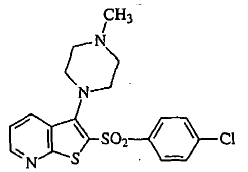 | MS(EI) 407 | ** | | |
| **107** | 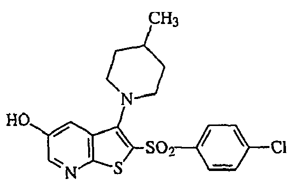 | 423,2 | * | | |
| **108** | 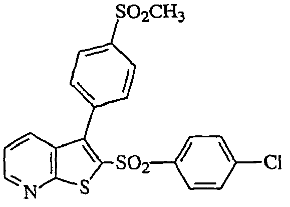 | 464,1 | *** | | |
| **109** | 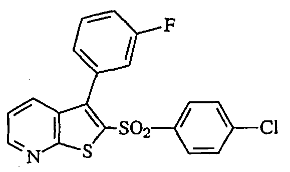 | 404,1 | * | | |
| **110** | 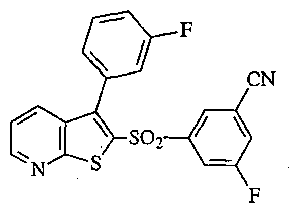 | MS 412 | * | | (400 MHz, DMSO-*d₆*, 25 °C): 8.85 (dd, *J* = 4.6, 1.5 Hz, 1H); 8.24 (ddd, *J* = 8.5, 2.4, 1.3 Hz, 1H); 7.86 (dd, *J* = 8.3, 1.5 Hz, 1H); 7.69-7.65 (m, 1H); 7.63 (ddd, *J* = 7.7, 2.4, 1.7 Hz, 1H); 7.60-7.51 (m, 2H); 7.46-7.38 (m, 1H); 7.13-7.05 (m, 2H). |
| **111** | 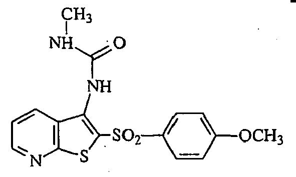 | 378,2 | *** | | |
| **112** | 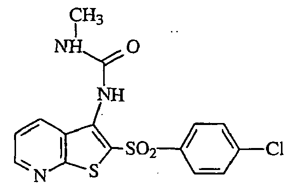 | 382,1 | *** | | |
| **113** | 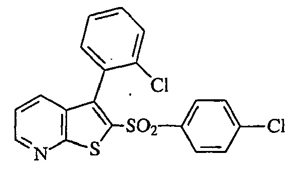 | 421,3 | * | | |
| **114** | 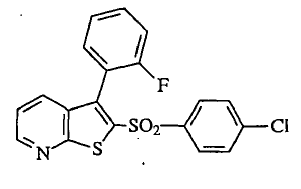 | 404,1 | * | | |
| **115** | 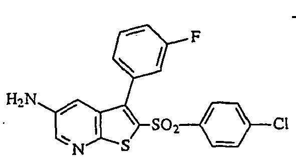 | 419,1 | * | | |
| **116** | 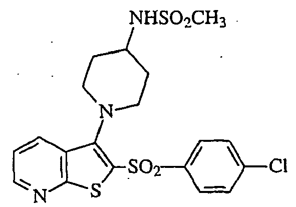 | MS(EI) 485 | *** | | |
| **117** | 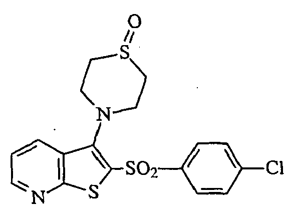 | MS(EI) 426 | *** | | |
| **118** | 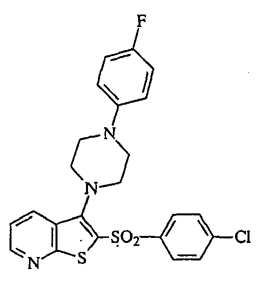 | MS(EI) 487 | *** | | |
| **119** | 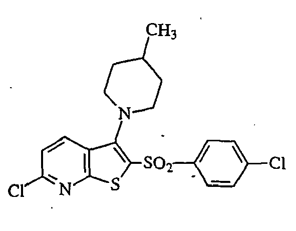 | MS (FAB): 440 | * | | (400 MHz, DMSO-*d₆*, 25 °C): 8.51 (d, *J* = 8.7 Hz, 1H); 8.02-7.94 (m, 2H); 7.78-7.70 (m, 2H); 7.59 (d, *J* = 8.7 Hz, 1H); 3.23-3.11 (m, 2H); 3.06-2.96 (m, 2H); 1.63-1.43 (m, 3H); 1.18-1.03 (m, 2H); 0.95 (d, *J* = 6.3 Hz, 3H). |
| **120** | 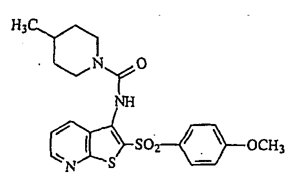 | 446,3 | *** | | |
| **121** | 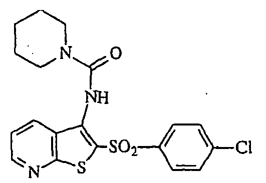 | 436,2 | *** | | |
| **122** | 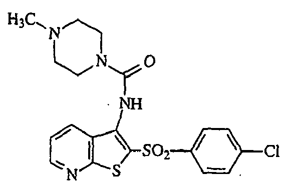 | 451,2 | *** | | |
| **123** | 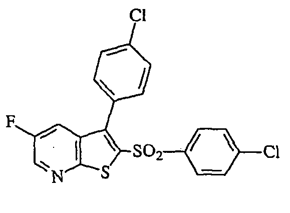 | MS (EI): 437 | * | | ¹H NMR (500 MHz, CDCl₃, 25 °C): 8.62 (dd, *J* = 2.8, 0.8 Hz, 1H); 7.49-7.42 (m, 4H); 7.40 (dd, *J* = 8.4, 2.8 Hz, 1H); 7.35-7.30 (m, 2H); 7.18-7.14 (m, 2H). |
| **124** | 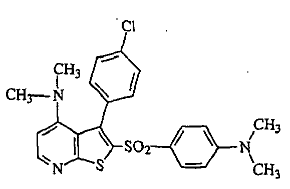 | 472,3 | *** | | |
| **125** | 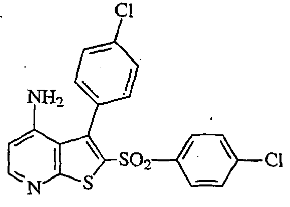 | 436,1 | * | | |
| **126** | 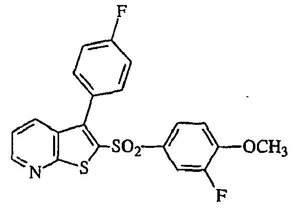 | 418,2 | * | | |
| **127** | 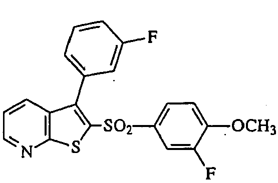 | 418,2 | * | | |
| **128** | 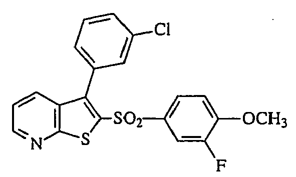 | 434,2 | * | | |
| **129** | 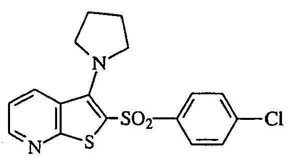 | MS(EI) 378 | * | | |
| **130** | 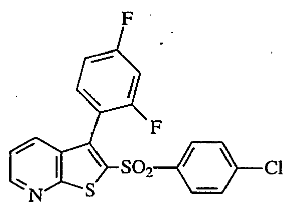 | 422,1 | * | | |
| **131** | 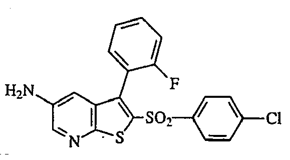 | 419,2 | * | | |
| **132** | 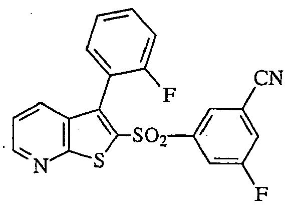 | 413,2 | * | | |
| **133** | 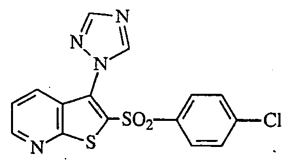 | MS(EI) 376 | ** | | |
| **134** | 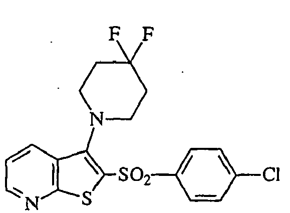 | MS(EI) 428 | * | | |
| **135** | 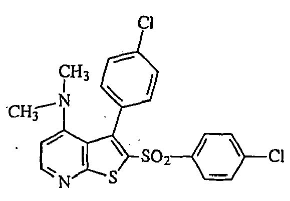 | 464,2 | ** | | |
| **136** | 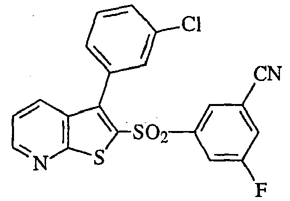 | 429,2 | * | | |
| **137** | 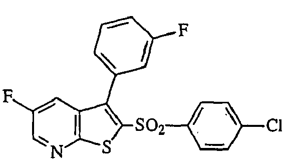 | 422,1 | * | | |
| **138** | 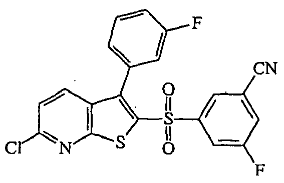 | 447,2 | * | | |
| **139** | 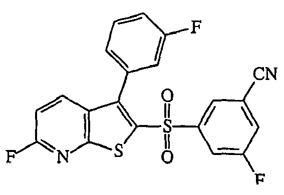 | 431,1 | * | | |
| **140** | 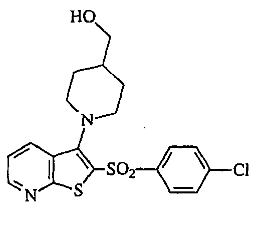 | 423,4 | * | | |
| **141** | 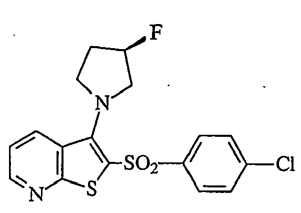 | 397,4 | * | | |

| | | | | | |
|---|---|---|---|---|---|
| * Kᵢ <500nM ** 500nM<Kᵢ < 1500nM *** Kᵢ >1500nM | | | | | |

¹H NMR spectra were obtained on a Varian Unity Inova 300, Varian Unity Inova 500 or on a Varian V-400 spectrometer. Chemical shifts are reported in parts per million relative to TMS as internal standard.

The invention is further illustrated by the following non-limiting examples.

### Examples

### Example 1

### (4-Chloro-phenyl)-(2-chloro-pyridin-3-yl)-methanone

### Intermedier (II)

Thionyl chloride (15 ml, 0.2 mol) and DMF (0.5 ml) were added dropwise to the suspension of 2-chloro-nicotinic acid (31.5 g, 0,2 mol) in chlorobenzene (100 ml) and the reaction mixture was stirred at 120 °C for 4 hours.
Aluminium chloride (33 g, 0.25 mol) was added at 0 °C to the reaction mixture, and it was boiled for 6 hours. The reaction mixture was poured onto ice (100 ml) and ethyl acetate (100 ml) was added. The mixture was stirred for half an hour at room temperature. The pH was adjusted to 8 by aqueous sodium hydroxide solution (40%). The emulsion was filtered, the filtrate was separated and extracted by ethyl acetate (2x50 ml). The organic phase was washed with water (100 ml) dried over Na₂SO₄ and concentrated in vacuo. The crude product was crystallized from isopropanol (20 ml) to yield 19.5 g (34%) of the titled compound.
The reaction is the same when we started from 2-chloro-5-nitro-nicotinic acid (Klunder, J. M. et al. J. Med. Chem. 1992, 35, 1887.)

### Example 2

### (4-Chloro-phenyl)-(2-mercapto-pyridin-3-yl)-methanone hydrochloride salt

The solution of thiourea (15.6 g, 0,200 mmol) in water (50 ml) and ethanol (25 ml) was added dropwise to the suspension of (4-chloro-phenyl)-(2-chloro-pyridin-3-yl)-methanone (7.65 g, 30 mmol) in ethanol (20 ml). The reaction mixture was heated for 24 hours, then cooled and stirred at 0 °C for 2-3 hours. The precipitate was filtered off, washed with water and purified by stirring with NaOH solution (2.5 g NaOH in 60 ml water) at room temperature for one hour. The mixture was filtered, and the filtrate was adjusted to pH 1 by 6N aqueous hydrochloric acid. The product was filtered off, washed with water to yield 6.48 g (76 %) of the titled compound.

### Example 3

### 2-(4-Chloro-benzenesulfonyl)-3-(4-chloro-phenyl)-thieno[2,3-b]pyridine

### General method for the preparation of compound of formula (Ia)

To the solution of (4-chloro-phenyl)-(2-mercapto-pyridin-1-yl)-methanone hydrochloride (0.7 g, 2.4 mmol) in DMF (15 ml) (4-chloro-phenyl)-chloromethyl sulfon (0.7 g,3.0 mmol) and NaOMe (0.28 g, 5.0 mmol) were added. The reaction mixture was kept between 120-130 °C temperature for 2 hours, followed evaporation in vacuo. Water (15 ml) was added to the residue and extracted with chloroform (3x20 ml). The organic phase was dried over Na₂SO₄, filtered and contcentrated in vacuo. The crude product was purified by crystallization from ether containing 10% dichloromethane and yielded 0.71 g of the title compound. Compounds of No.**1-14, 16, 17, 18, 19, 22, 23, 24, 26-31, 33-35, 37, 39-41, 44, 45, 48, 54-56, 59, 61-63 ,68, 70, 72, 78, 80, 82 ,87, 88 and 91** were prepared by the same reaction. The different chloromethyl sulfon reagents was commercially available or obtained by the method of Y.Yinfa et.al. (Synth. Communication., 2004, 34, 13, 2443).

### Example 4

### 3-(4-Chlorophenyl)-thieno[2,3-b]pyridine-2-sulfonic acid (4-chloro-phenyl)-amide

### (Compound 15)

A mixture of N-(4-Chloro-phenyl)chloromethanesulfonamide (0.265 g, 1.1 mmol) (Intermediate A), (4-chloro-phenyl)-(2-mercapto-pyridin-3-yl)-methanone hydrochloride (0.29 g, 1 mmol) (Example 2) and NaOCH₃ (0.12 g, 2.2 mmol) in DMF (5 ml) was stirred at 130 °C for 2 hours. Then the solvent was removed in vacuo, water (15 ml) was added to the residue, and the mixture was extracted three times with chloroform (15-15 ml). The organic phase was dried and concentrated. The residue was triturated with n-hexane (10 ml), and after decantation of n-hexane the oily product was purified by column chromatography (Kieselgel 60, eluent: chloroform:methanol = 98:2) to yield 0.225 g (52 %) of the title compound.

### N-(4-Chloro-phenyl)-chloromethanesulfonamide

### (Intermediate A )

To a stirred solution of 4-chloroaniline (0.65 g; 5.0 mmol) in dichloromethane (10 ml) TEA (1.4 ml, 10 mmol) was added. The solution was cooled to 5 °C and the solution of chloromethanesulfonyl chloride (0.9 ml, 10 mmol) in dichloromethane (5 ml) was added dropwise keeping temperature at 5 °C. After the addition the reaction mixture was stirred for 30 min at 10 °C, and then at ambient temperature for 20 hours. The reaction mixture was evaporated to dryness and 2 N aqueous NaOH (25 ml; 50 mmol) was added, and the mixture was stirred for 2 hours. Then the solution was neutralized with aqueous HCl to pH=7 and extracted three times with ethyl acetate (30-30 ml). The organic phase was dried and concentrated. The crude product was purified by column chromatography (Kieselgel 60, eluent: n-hexane:ethyl acetate = 2: 1) to yield 0.7 g (58 %) of the title compound as crystalline substance. Melting point: 102-104 °C.

### Example 5

### 3-(4-Chlorophenyl)-thieno[2,3-b]pyridine-2-sulfonic acid 4-chloro-benzylamide

### (Compound 21)

The title compound was prepared from N-(4-Chloro-benzyl)-chloromethane-sulfonamide (Intermediate A) and (4-chloro-phenyl)-(2-mercapto-pyridin-1-yl)-methanone hydrochloride (Example 2) according to the method described in Example 4. The crude product was purified by column chromatography (Kieselgel 60, eluent: n-hexane:ethyl acetate = 2:1) to yield 0.2 g (45 %) of the title compound.

### N-(4-Chloro-benzyl-)chloromethanesulfonamide

### (Intermediate A )

The title compound was prepared from 4-chlorobenzylamine and chloromethanesulfonyl chloride according to the method described in Example 4 for Intermediate A. The compound was purified by column chromatography to yield (54%) crystalline product.
Melting point: 91-92 °C.

### Example 6

### 3-(4-Chlorophenyl)-2-(4-methyl-piperidine-1-sulfonyl)-thieno[2,3-b]pyridine

### (Compound 20)

The title compound was prepared from 1-Chloromethanesulfonyl-4-methylpiperidine (Intermediate A) and (4-chloro-phenyl)-(2-mercapto-pyridin-1-yl)-methanone hydrochloride (Example 2) according to the method described in Example 3. The crude product was purified by column chromatography (Kieselgel 60, eluent: n-hexane:ethyl acetate = 1:1) to yield 0.23 g (56 %) of the title compound.

### 1-Chloromethanesulfonyl-4-methylpiperidine

### (Intermediate A)

To a stirred solution of 4-methylpiperidine (0.6 ml; 10 mmol) in dichloromethane (10 ml) TEA (1.4 ml, 10 mmol) was added. The solution was cooled to 5 °C and the solution of chloromethanesulfonyl chloride (0.4 ml, 4.4 mmol) in dichloromethane (5 ml) was added dropwise keeping temperature at 5 °C. After the addition the reaction mixture was stirred for 30 min at 10 °C, and then at ambient temperature for 4 hours. The reaction mixture was diluted with dichloromethane (10 ml) and washed twice with 0.5 N aqueous hydrochloric acid (10-10 ml), then with water. The organic phase was dried and concentrated. The crude product was solidified with n-hexane (5 ml), and then crystallized from n-hexane: ethyl acetate (3:1) mixture (4 ml) to yield 0.45 g (48 %) of the title compound as crystalline substance.
Melting point: 90-92 °C.

### Example 7

### 2-(4-Chloro-benzenesulfonyl)-thieno[2,3-b]pyridin-3-yl amine

### General method for the preparation of intermediate (IV):

A solution of 3-cyanopyridine-2-thiol (6.8 g, 50 mmol), chloromethyl-(4-chloro-phenyl)-sulfone (12.3 g, 55 mmol) and sodium methoxide (3.0 g, 55 mmol) in anhydrous N,N-dimethylformamide (150 ml) was stirred at 120 °C for 2 hours. The reaction mixture was cooled to room temperature and water (150 ml) was dropped into. The solid was filtered, washed (H₂O) and dried to yield 13.7 g (84%) of the titled compound.

### Example 8

### 3-Bromo-2-(4-chloro-benzenesulfonyl)-thieno[2,3-b]pyridine

### General method for the preparation of intermediate (V):

To a mixture of anhydrous copper(II) bromide (11.2 g, 50 mmol) and tert-butyl-nitrite (9.0 ml, 75 mmol) in anhydrous acetonitrile (100ml) under argon atmosphere at 2-(4-chloro-benzenesulfonyl)-thieno[2,3-*b*]pyridin-3-ylamine (10.7 g, 34 mmol) intermediate (IV) was added in portions meanwhile keep the temperature under 70°C. The reaction mixture was kept at 65°C for 1.5 hour, then it was cooled to room temperature. The crystalline pruduct was filtered and washed with acetonitrile to give a pure product (7.7 g, 58.3%).

### Example 9

### 2-(3-Cyano-5-fluoro-benzenesulfonyl)-3-(3-fluoro-phenyl)-thieno[2,3-b]pyridine,

### (compound 110)

### General process for the preparation of compounds of formula (Ib):

3-Bromo-2-(3-cyano-5-fluoro-benzenesulfonyl)-thieno[2,3-b]pyridine (Example 8) (0.2 g, 0.5 mmol) was dissolved in toluene (4 ml) and ethanol (4.5 ml) under argon atmosphere. To the solution Pd(PPh₃)₄ (28mg, 0.025 mmol), 3-fluorophenylboronic acid (85 mg, 0.6 mmol) and 2M solution of Na₂CO₃ (2.3 ml) was added. The reaction mixture was refluxed for 1.5-2 hours, then the organic solvent was evaporated in vacuo. Water (10 ml) was added to the residue and the obtained suspension was extracted three times with chloroform (3x10 ml). The organic phase was washed with water (5.0 ml), dried over Na₂SO₄, filtered, and contcentrated in vacuo. The crude product was purified by column chromatography (Kieselgel 60, chloroform :methanol = 5:0.05) to yield 0.1 g (25 %) of the title compound.

Compounds **38, 42, 52, 53, 60, 66, 67, 69, 71, 73, 83, 84, 108-110, 113, 114, 126-128, 130, 132, 136** and**137** were prepared according the procedure described above.

### Example 10

### 2-(3-Cyano-benzenesulfonyl)-3-(4-methyl-piperidinyl)-thieno[2,3-b]pyridine,

### (compound 36)

### General procedure for the synthesis of some compounds of formula(Ic) (Scheme 3a):

3-Bromo-2-(3-cyano-benzenesulfonyl)-thieno[2,3-b]pyridine (prepared according to Example 8) ( 2.18 g, 5:75 mmol) and 4-methyl-piperidine (2.0 ml, 17 mmol) was dissolved in DMF (9 ml) and heated to 100°C temperature for one hour under argon. The reaction mixture was cooled and water (60ml) was added and extracted with chloroform (3x20ml). The organic layer was washed with water (2x20ml), dried and evaporated in vacuo. The crude product was purified via chromatography (Kieselgel 60, hexan :ethylacetate = 2:1) yielded 1.64g (72.4%).
Analog prepared compounds were **32, 36, 46, 92-96, 98-100, 106, 116-118, 129, 133, 134, 140** and **141.**

### Example 11

### N-[2-(4-methoxy-benzenesulfonyl)-thieno[2,3-b]pyridin-3-yl]-4-methyl-piperidine-1-carboxamide (compound 120)

### General procedure for the synthesis of some compounds of formula(Ic) (Scheme 3b):

2-(4-Methoxy-benzenesulfonyl)-thieno[2,3-*b*]pyridin-3-yl-amine (IV)( prepared as see Example 7) (0.48 g, 1.5 mmol) was suspended in dry tetrahydrofurane , triethylamine (0.3 ml, 2 mmol) was added and triphosgene (0.18 g 0.6 mmol) dissolved in tetrahydrofurane was dropped in. After one hour stirring at room temperature 4-methyl-piperidine (0.36 ml, 3 mmol) was added and the stirring continued for 20 hours. The mixture was evaporated in vacuo, the residue dissolved in chloroform, washed with water, dried and evaporated in vacuo. Purifying with flash chromatography gave the title compound (0.43 g, 65 %).
Applying the above procedure the following compounds were prepared: **111, 112, 120-122.**

### Example 12

### 2-(4-Chloro-benzenesulfonyl)-3-(4-chloro-phenyl)-thieno[2,3-b]pyridin-5-ylamine

### (Compound 79)

To the solution of 2-(4-chloro-benzenesulfonyl)-3-(4-chloro-phenyl)-5-nitro-thieno[2,3-*b*]pyridine (Example 3, compound 72) (0.90 g, 1.93 mmol) in ethyl acetate (12 ml) SnCl₂ 2H₂O (2.19 g, 9.71 mmol) was added in one portion. The reaction mixture was stirred at 70 °C for 1 hour. The mixture was poured into a flask charged with ice and neutralized with NaHCO₃ solution (10%), than dichloromethane (60 ml) was added and the organic layer separated. The water phase was extracted with dichloromethane (2x30 ml). The organic phases were collected, dried over Na₂SO₄, filtered and concentrated in vacuo. The residue was subjected to column chromatography (SiO₂, cyclohexane : acetone = 7:3) giving 2-(4-chloro-benzenesulfonyl)-3-(4-chloro-phenyl)-thieno[2,3-b]pyridin-5-ylamine (0.46 g, Yield: 55%).

### Example 13

### N-[2-(4-Chloro-benzenesulfonyl)-3-(4-chloro-phenyl)-thieno[2,3-b]pyridin-5-yl]-methanesulfonamide (Compound 85)

To the pyridine solution (2 ml) of 2-(4-chloro-benzenesulfonyl)-3-(4-chloro-phenyl)-thieno[2,3-*b*]pyridin-5-ylamine (0.095 g, 0.22 mmol) (Example 12) methanesulfonyl chloride (0.025 g, 0.22 mmol) was added in one portion. The reaction mixture was refluxed for 3 hours. Than the solvent was evaporated in vacuo, the residue was suspended up in methanol (5 ml) and filtered off. To the filtrate water (10 ml) was added and the formed mixture was extracted with dichloromethane (2x10 ml). The organic phases were collected, dried, filtered and concentrated in vacuo. The crystalls were collected, washed with cold methanol and filtered off, to give(73 mg, Yield: 65%) of title compound.

### Example 14

### 5-Chloro-2-(4-chloro-benzenesulfonyl)-3-(4-chloro-phenyl)-thieno[2,3-b]pyridine

### (Compound 97)

A suspension of 2-(4-chloro-benzenesulfonyl)-3-(4-chloro-phenyl)-thieno[2,3-*b*] pyridin-5-ylamine (Example 12) (0.69 g, 1.58 mmol), concentrated HCl (6 ml) and water (4 ml) was cooled to 0 °C and ice cold water solution of NaNO₂ (3 ml, 0.11 g, 1.58 mmol) was added. The mixture was stirred for half an hour at 0 °C. Than cooled, concentrated HCl (4 ml) solution of Cu(I)Cl (0.188 g, 1.9 mmol) was added in one portion. The mixture was let to warm up to room temperature and than immersed in a 60 °C oil bath for one hour. The cooled mixture was poured into ice (10g), neutralized with NaHCO₃ solution (10%) and extracted with dichloromethane (3x50 ml). The organic phases were collected, dried over Na₂SO₄, filtered and concentrated in vacuo. The residue was purified by column chromatography (SiO₂, cyclohexane : acetone = 7:3) giving the title compound (0.27 g, 38%).

### Example 15

### 2-(4-Chloro-benzenesulfonyl)-3-(4-chloro-phenyl)-5-fluoro-thieno[2,3-b]pyridine

### (Compound 123)

A suspension of 2-(4-Chloro-benzenesulfonyl)-3-(4-chloro-phenyl)-thieno[2,3-*b*] pyridin-5-ylamine (0.50 g, 1.14 mmol) (Example 12), concentrated HCl (8 ml) and water (4 ml) was cooled to 0 °C and than ice cold water solution of NaNO₂ (5 ml, 0.084 g, 1.21 mmol) was added. The mixture was stirred for half an hour at 0 °C. Than cooled, water solution of NaBF₄ (0.176 g, 1.59 mmol) was added in one portion. The mixture was stirred at 0 °C for half an hour and filtered. The obtained solid was washed with cold water, methanol and dried overnight. The compound was melted at 179-182 °C and stirred for 10 minutes. Than the mixture was cooled to room temperature and subjected to column chromatography (SiO₂, cyclohexane : acetone = 9:1) giving the 5-fluoro compound (0.126 g, 25%).

### Example 16

### 3-Bromo-2-(3-cyano-5-fluoro benzenesulfonyl)- thieno[2,3-b]pyridine-N-oxide

### General method for the preparation of Intermediate X:

To the solution of 3-bromo-2-(3-cyano-5-fluoro benzenesulfonyl)- thieno[2,3-b] pyridine (3.51 g, 8.8 mmol) in chloroform (130 ml) m-chloroperoxybenzoic acid (6.08 g, 35 mmol, 77%) was added and the reaction mixture was stirred at room temperature overnight. The solution was washed with NaHCO₃ (10%, 3 x 50 ml) then twice with water (25 ml), dried over Na₂SO₄, filtered, and contcentrated in vacuo. The crude product was purified by a treatment with ether to give 3.06 g (84%) of the title compound.

### Example 17

### 3-Bromo-2-(4-chloro- benzenesulfonyl)-5-nitro-thieno[2,3-b]pyridine-N-oxide

### General method for the preparation of Intermediate XI:

3-Bromo-2-(4-chloro-benzenesulfonyl)- thieno[2,3-b]pyridine-N-oxide (prepared as described in Example 16) (4.95 g, 12.2mmol) was boiled at 120°C with 70% nitric acid (0.75ml, 12 mmol) in acetic acid for 4 hours. The reaction mixture was evaporated and the residue was purified by chromatography (Kieselgel 60, chloroform : methanol = 5:0.1) to yield a yelow solid compound (0.8 g, 15%) and starting material (2.4 g, 49%).

### Example 18

### 5-Amino-3-bromo-2-(4-chloro- benzenesulfonyl) -thieno[2,3-b]pyridine

### General method for the preparation of Intermediate XII:

3-Bromo-2-(4-chloro- benzenesulfonyl)-5-nitro-thieno[2,3-b]pyridine-N-oxide ( Example 17) (0.8 g, 1,78 mmol) was dissolved in acetic acid (12 ml) and Fe powder was added (0,61 g, 10 mmol). The reactiom mixture was stirred at 70 °C for 30 minutes, followed an evaporation by vacuo, and residue was purified by chromatography (Kieselgel 60, chloroform : methanol = 5:0.1) to yield the title compound (0.51 g, 71 %).

### Example 19

### 5-Amino-2-(4-chloro-benzenesulfonyl) -3-(3-fluor-phenyl)-thieno[2,3-b]pyridine

### (Compound 115)

5-Amino-3-bromo-2-(4-chloro- benzenesulfonyl) -thieno[2,3-b]pyridine was reacted with 3-fluorophenylboronic acid as described in Example 9. Yield is 20%.
Compound 131 was prepared in accordance with the same method.

### Example 20

### 5-Amino-2-(4-chloro-benzenesulfonyl) -3-(4-methyl-piperidine)-thieno[2,3-b]pyridine

### (Compound 104)

5-Amino-3-bromo-2-(4-chloro- benzenesulfonyl) -thieno[2,3-b]pyridine was reacted with 4-methyl-piperidine as described in Example 10.

### Example 21

### 2-(4-Chloro-benzenesulfonyl) -3-(3-fluoro-phenyl)-5-fluoro-thieno[2,3-b]pyridine

### (Compound 137)

5-Amino-2-(4-chloro-benzenesulfonyl) -3-(3-fluor-phenyl)-thieno[2,3-b]pyridine (Example 19) was treated as described in Example 15 to give the 5-fluoro compound .

### Example 22

### 2-(3-Cyano-5-fluoro -benzenesulfonyl)-3-(4-chloro-phenyl)-thieno[2,3-b]pyridine-N-oxide

### General method for the preparation of intermediate XIV (R₂ is phenyl or aromatic heterocycle):

3-Bromo-2-(3-cyano-5-fluoro benzenesulfonyl)- thieno[2,3-b]pyridine-N-oxide (Example 16) was reacted with 4-chlorophenylboronic acid using the same procedure as describe in Example 9 to give the title compound (yield 63%).

### Example 23

### 2-(4-Chloro -benzenesulfonyl)-3-(4-methyl-piperidinyl)-thieno[2,3-b]pyridine-N-oxide

### General method for the preparation of intermediate XIV (R₂ is substituted amino group):

3-Bromo-2-(4-chloro -benzenesulfonyl)- thieno[2,3-b]pyridine-N-oxide (prepared Example 16) was reacted with 4-methyl- piperidine according the procedure Example 10 at 80°C temperature to give the title compound.

### Example 24

### 2-(3-Cyano-5-fluoro-benzenesulfonyl)-3-(4-chloro-phenyl)-7H-thieno[2,3-b]pyridin-6-one

### General method for the preparation of intermediate XV:

2-(3-Cyano-5-fluoro-benzenesulfonyl)-3-(4-chloro-phenyl)-thieno[2,3-b]pyridine-N-oxide (1.35 g, 3.0 mmol) was treated in DMF suspension(11 ml) with trifluoroacetic anhydrate (8.6 ml, 61 mmol) at room temperature for 1,5 hours. By the end of the reaction it became a solution, which was concentrated by half of the whole volume in vacuo. Water (8,6 ml) was added, the crystalline product was filtered off and washed with water.The reaction resulted in 0.93 g (68.9%) of the titled compound.

### Example 25

### 2-(4-Chloro -benzenesulfonyl)-3-(4-methyl-piperidinyl)-5-hydroxy-thieno[2,3-b]pyridine

Starting from 2-(4-chloro -benzenesulfonyl)-3-(4-methyl-piperidinyl)-thieno[2,3-b] pyridine-N-oxide (Example 23) the reaction was prepared using the same procedure as described in Example 24 and as a side product the titled compound (yield 10%) was isolated. The yield of the corresponding 2-(4-chloro-benzenesulfonyl)-3-(4-methyl piperidinyl)-7H-thieno[2,3-b]pyridin-6-one was 60% (intermediate XV)

### Example 26

### 2-(3-Cyano-5-fluoro-benzenesulfonyl)-3-(4-chloro-phenyl)-6-chloro-thieno[2,3-b] pyridine

### (Compound 86)

### General method for the preparation of 6-chloro compounds of formula (I) e.g. compound 43, 5, 51, 74, 76, 81, 119 and 138.

2-(3-Cyano-5-fluoro-benzenesulfonyl)-3-(4-chloro-phenyl)-7H-thieno[2,3-b]pyridin-6-one (0.92 g, 2.1 mmol) was dissolved in DMF (14 ml) and phosphoroxychloride (2.0 ml, 21 mmol) was added, and the reaction mixture was heated at 90-100°C for 2,5 hours. It was then cooled , quenched with ice (10g) and alkalined with ammonium hydroxide (25%, 2.5 ml). The solid product was filtered, washed with water and methanol. It was recrystallized from hot dioxane yielded 0.73 g (75%) the titled compound.

### Example 27

### 2-(3-Cyano-5-fluoro-benzenesulfonyl)-3-(4-chloro-phenyl)-6-fluoro-thieno[2,3-b] pyridine

### (Compound 90)

### General method for the preparation of 6-fluoro compounds of formula (I) e.g. compound 58, 65, 75, 77, 89 and 139.

2-(3-Cyano-5-fluoro-benzenesulfonyl)-3-(4-chloro-phenyl)-6-chloro-thieno[2,3-b] pyridine (0.83 g, 1.8 mmol) was dissolved in DMSO (10 ml) and potassium fluoride (0.3 g, 5.0 mmol) was added. The reaction mixture was heated to 140°C for 1,5 hours. After cooling water (10 ml) was added and the crystalline product was filtered, washed with water. It was recrystallized from hot methanol to give 0.65 g ( 62.5%) the compound no. 90.

### Example 28

### 2-(3-Cyano-benzenesulfonyl)-3-(4-chloro-phenyl)-6-methoxy-thieno[2,3-b]pyridine

### (Compound 64)

2-(3-Cyano-benzenesulfonyl)-3-(4-chloro-phenyl)-7H-thieno[2,3-b]pyridin-6-one ( 0.11 g, 0.25 mmol) (Example 24), silver (I) carbonate (95 mg, 0.35 mmol) and iodomethane (0.15 ml, 2.4 mmol) were suspended in dichloromethane (5 ml) and DMF (0.5 ml). The reaction mixture was stirred at room temperature for 24 hours. It was filtered , washed with dichloromethane (2x10 ml), the filtrate was washed with Na₂CO₃ solution (10%, 10 ml) and water (10 ml). The organic solvent was dried , evaporated in vacuo and residue was purified by chromatography (Kiselgehl 60, ethylacetate : hexane = 1 : 1) to give the title product ( 47 mg, 43%).

### Example 29

### 6-Amino-2-(4-chloro-benzenesulfonyl)-3-(4-chloro-phenyl)-thieno[2,3-b]pyridine

### (Compound 49)

2-(4-Chloro-benzenesulfonyl)-3-(4-chloro-phenyl)-7H-thieno[2,3-b]pyridin-6-one (0.30 g, 0.69 mmol) (Example 24), was dissolved in abs. DMF (10 ml), cooled to 0°C and abs. pyridine (0.06 ml, 0.74 mmol) and trifluoromethanesulphonic anhydride (0.026 ml, 0.77 mmol) was added. The reactiom mixture was kept at 0°C for 1 hour, then dimethylchloride (10 ml) and 1N NaHCO₃ (10 ml) were added. After separation the organic phase washed with water (10 ml), dried and evaporated in vacuo. The crude triflate was treated with methanol saturated with NH₃ (3 ml) at at 0-5°C for 2 hours. The reaction mixture was evaporated in vacuo and the residue purified by cromatography (Kieselgehl-60, chloroform : methanol = 5 : 0.2) to give the title compound (120 mg, 40%).

### Example 30

### 2-(4-Chloro-benzenesulfonyl)-3-(4-chloro-phenyl)-6-dimethylamino-thieno[2,3-b] pyridine

### (Compound 47)

2-(4-Chloro-benzenesulfonyl)-3-(4-chloro-phenyl)-7H-thieno[2,3-b]pyridin-6-one (0.19 mmol) (Example 24) was reacted using the same procedure as for Example 29, but employing dimethylamine hidrochloride as the amine source ( 0.49 mmol) in DMSO (2.0 ml) in the presence of TEA (0.49 mmol) at room temperature for 2 hours. Yield is 73.7%.

### Example 31

### 4-Chloro-2-(4-chloro-benzenesulfonyl)-3-(4-chloro-phenyl)-thieno[2,3-b]pyridine

### (Compound 57)

### 4-Chloro-2-(4-chloro-benzenesulfonyl)-3-(4-chloro-phenyl)-thieno[2,3-b]pyridine

### (Compound 43)

The mixture of 2-(4-chloro-benzenesulfonyl)-3-(4-chloro-phenyl)-thieno[2,3-b] Pyridine N-oxide ( prepared according the procedure described in Example 16)(6.4 g, 14.7 mmol) and phosphorus(III) oxychloride (10.2 ml, 109.4 mmol) was stirred and heated at 60°C for 3 hours. The obtained solution was poured onto ice and was alkalized with 5M sodium hydroxyde solution. After cooling the precipitate was filtered and washed with water. The crude product was purified by column chromatography on silica gel (Kieselgel 60, eluent: dichloromethane) to obtain the 4-chloro compound (3.31 g) and 6-chloro-2-(4-chloro-benzenesulfonyl)-3-(4-chloro-phenyl)-thieno[2,3-b]pyridine (3.03 g) (compound 43).

### Example 32

### 2-(4-Chloro-benzenesulfonyl)-3-(4-chloro-phenyl)-4-dimethylamino-thieno[2,3-b] pyridine

### (Compound 135)

4-Chloro-2-(4-chloro-benzenesulfonyl)-3-(4-chloro-phenyl)-thieno[2,3-b]pyridine (100 mg, 0.22 mmol) in DMF (5 ml) was irradiated in a reactor with 300 W microwave at 210°C for 10 minutes. The solvent was evaporated *in vacuo.* The crude product was purified by column chromatography on silica gel (Kieselgel 60, eluent: chloroform : methanol = 98:2) to obtain the title compound (36 mg).

### Example 33

### 4-Fluor-2-(4-chloro-benzenesulfonyl)-3-(4-chloro-phenyl)-thieno[2,3-b]pyridine

### (Compound 102)

The mixture of 4-chloro-2-(4-chloro-benzenesulfonyl)-3-(4-chloro-phenyl)-thieno[2,3-b] pyridine (454 mg, 1 mmol) and potassium fluoride (174 mg, 3 mmol) in DMF (5 ml) was stirred and heated at 140°C for 48 hours. The solvent was evaporated *in vacuo.* The crude product was purified by column chromatography on silica gel (Kieselgel 60, eluent: n-hexane: ethyl acetate = 2:1) to obtain the title compound (101 mg).

### Example 34

### 2-(4-chloro-benzenesulfonyl)-3-(4-chloro-phenyl)-4-methoxy-thieno[2,3-b]pyridine

### (Compound 101)

The mixture of 4-chloro-2-(4-chloro-benzenesulfonyl)-3-(4-chloro-phenyl)-thieno[2,3-b] pyridine (150 mg, 0.33 mmol) and sodium methoxide (1.5 g, (27.8 mmol) in methanol (150 ml) was stirred and heated at reflux for 4 hours. The solvent was evaporated *in vacuo.* Water (100 ml) was added, and the obtained mixture was extracted with chloroform (3x20 ml). The combined organic layer was dried over sodium sulfate, filtered, and evaporated *in vacuo.* The crude residue was purified by crystallization from diethyl ether to obtain the title compound (105 mg).

### Example 35

### 2-(4-chloro-benzenesulfonyl)-3-(4-chloro-phenyl)-4-hydroxy-thieno[2,3-b]pyridine

### (Compound 103)

The mixture of 4-chloro-2-(4-chloro-benzenesulfonyl)-3-(4-chloro-phenyl)-thieno[2,3-b] pyridine (150 mg, 0.33 mmol) and sodium acetate (280 mg, 3.4 mmol) in acetic acid (7 ml) and water (0.1 ml) was stirred and heated at 100°C for 48 hours. The solution was diluted with water (25 ml). The precipitate was filtered, washed with water (3x10 ml) and dried. The crude product was purified by crystallization from chloroform to obtain the title compound (58 mg).

### Example 36

### 4-Amino-2-(4-chloro-benzenesulfonyl)-3-(4-chloro-phenyl)-thieno[2,3-b]pyridine

### (Compound 125)

The mixture of 4-chloro-2-(4-chloro-benzenesulfonyl)-3-(4-chloro-phenyl)-thieno[2,3-b]pyridine (400mg, 0.88 mmol) and sodium azide (360 mg, 5.54 mmol) in DMF (11 ml) and water (4 ml) was stirred and heated at 105°C for 5 hours. The solution was diluted with water (15 ml). The precipitate was filtered, washed with water (5x10 ml) and dried. The obtained crude 4-azido-2-(4-chloro-benzenesulfonyl)-3-(4-chloro-phenyl)-thieno[2,3-b]pyridine (400 mg, 0.87 mmol) was dissolved in methanol (20 ml). To the solution sodium borohydride (400 mg, 10.6 mmol) was added, and the mixture was stirred at ambient temperature for 24 hours. Water (30 ml) was added, and the obtained mixture was extracted with chloroform (3x60 ml). The combined organic layer was dried over sodium sulfate, filtered, and evaporated *in vacuo.* The crude residue was purified by crystallization from diethyl ether to obtain the title compound (193 mg).

### Example 37

### 3-(4-chloro-phenyl)-4-dimethylamino-2-(4-dimethylamino-benzenesulfonyl)-thieno[2,3-b]pyridine

### (Compound 124)

4-Chloro-2-(4-chloro-benzenesulfonyl)-3-(4-chloro-phenyl)-thieno[2,3-b]pyridine (100 mg, 0.22 mmol) in DMF (5 ml) was irradiated in a reactor with 300 W microwave at 250°C for 10 minutes. The solvent was evaporated *in vacuo.* The crude product was purified by column chromatography on silica gel (Kieselgel 60, eluent: chloroform : methanol = 98:2) to obtain the title compound (26 mg).

**All compounds was analysed by HPLC-MS or MS and ¹H-NMR. Purity of compounds of formula (I) was measured by HPLC methods and all compounds have >95% purity.**

### Example 38

### Preparation of pharmaceutical compositions:

### a) Tablets:

0.01-50 % of active ingredient of formula (I), 15-50 % of lactose, 15-50 % of potato starch, 5-15 % of polyvinyl pyrrolidone, 1-5 % of talc, 0.01-3 % of magnesium stearate, 1-3 % of colloid silicon dioxide and 2-7 % of ultraamylopectin were mixed, then granulated by wet granulation and pressed to tablets.

### b) Dragées, filmcoated tablets:

The tablets made according to the method described above were coated by a layer consisting of entero- or gastrosolvent film, or of sugar and talc. The dragées were polished by a mixture of beeswax and carnuba wax.

### c) Capsules:

0.01-50 % of active ingredient of formula (I), 1-5 % of sodium lauryl sulfate, 15-50 % of starch, 15-50 % of lactose, 1-3 % of colloid silicon dioxide and 0.01-3 % of magnesium stearate were thoroughly mixed, the mixture was passed through a sieve and filled in hard gelatin capsules.

### d) Suspensions:

Ingredients: 0.01-15 % of active ingredient of formula (I), 0.1-2 % of sodium hydroxide, 0.1-3 % of citric acid, 0.05-0.2 % of nipagin (sodium methyl 4-hydroxybenzoate), 0.005-0.02 % of nipasol, 0.01-0.5 % of carbopol (polyacrilic acid), 0.1-5 % of 96 % ethanol, 0.1-1 % of flavoring agent, 20-70 % of sorbitol (70 % aqueous solution) and 30-50 % of distilled water.

To solution of nipagin and citric acid in 20 ml of distilled water, carbopol was added in small portions under vigorous stirring, and the solution was left to stand for 10-12 h. Then the sodium hydroxide in 1 ml of distilled water, the aqueous solution of sorbitol and finally the ethanolic raspberry flavor were added with stirring. To this carrier the active ingredient was added in small portions and suspended with an immersing homogenizator. Finally the suspension was filled up to the desired final volume with distilled water and the suspension syrup was passed through a colloid milling equipment.

### e) Suppositories:

For each suppository 0.01-15% of active ingredient of, formula (I) and 1-20% of lactose were thoroughly mixed, then 50-95% of adeps pro suppository (for example Witepsol 4) was melted, cooled to 35 °C and the mixture of active ingredient and lactose was mixed in it with homogenizator. The obtained mixture was mould in cooled forms.

### f) Lyophilized powder ampoule compositions:

A 5 % solution of mannitol or lactose was made with bidistilled water for injection use, and the solution was filtered so as to have sterile solution. A 0.01-5 % solution of the active ingredient of formula (I) was also made with bidistilled water for injection use, and this solution was filtered so as to have sterile solution. These two solutions were mixed under aseptic conditions, filled in 1 ml portions into ampoules, the content of the ampoules was lyophilized, and the ampoules were sealed under nitrogen. The contents of the ampoules were dissolved in sterile water or 0.9 % (physiological) sterile aqueous sodium chloride solution before administration.

## Claims

1. A compound of formula (I): 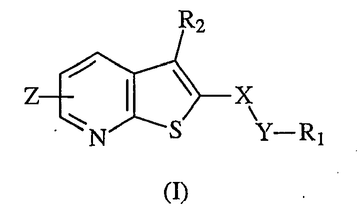 wherein
X represents a group selected from SO, SO₂;
Y represents a group selected from (CH₂)n, NH, NHCH₂;
n is an integer of 0 to 1;
Z is H or monosubstituted by alkyl, nitro, halogen, alkoxy, trifluoromethyl, cyano, amino, alkylamino, dialkylamino, aminomethyl, alkylaminomethyl, dialkylaminomethyl, hydroxyl, alkylsulfonylamino;
R₁ is an optionally substituted alkyl, cycloalkyl, phenyl, biphenyl, heterocyclyl;
R₂ is an optionally substituted phenyl, heterocyclyl, or
NR3R4 group wherein R₃ and R₄ are independently selected from the group of hydrogen and an optionally substituted alkyl, or R₃ and R₄ together with the N atom to which they are attached form an optionally substituted C₅₋₇ heterocyclyl group, containing one or more heteroatom(s), or
NH-CO-NR₅R₆ group, wherein R₅ and R₆ are independently selected from the group of hydrogen and an optionally substituted alkyl, or R₅ and R₆ together with the N atom to which they are attached form an optionally substituted C₅₋₇ heterocyclyl group, containing one or more heteroatom(s); and/or hydrates and/or solvates and/or pharmaceutically acceptable salts thereof formed with acids or bases.

2. A compound according to claim 1 of formula (I) 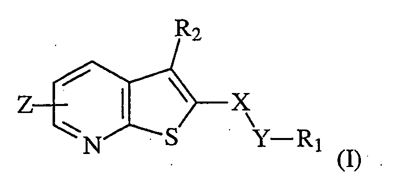 wherein
X represents a group selected from SO, SO₂;
Y represents a group selected from (CH₂)n, NH, NHCH₂;
n is an integer of 0 to 1;
Z is H or monosubstituted by alkyl, nitro, halogen, alkoxy, trifluoromethyl, cyano, amino, alkylamino, dialkylamino, aminomethyl, alkylaminomethyl, dialkylaminomethyl, hydroxyl, alkylsulfonylamino;
R₁ is alkyl or C₃₋₁₀ cycloalkyl group optionally substituted with one or more substituent(s) selected from alkoxy, trifluoromethyl, amino, alkylamino, dialkylamino, aminomethyl, alkylaminomethyl, dialkylaminomethyl, acylamino, cyano, halogen, or
phenyl or biphenyl optionally substituted with one or more substituent(s) selected from alkyl, methoxy, trifluoromethyl, amino, alkylamino, dialkylamino, aminomethyl, alkylaminomethyl, dialkylaminomethyl, acylamino, alkylsulfonyl, alkylsulfonylamino, cyano, halogen, or
saturated or unsaturated monocyclic or bicyclic heterocyclyl, containing 1-4 heteroatom(s) selected from O, N or S, such as pyridyl, quinolinyl, thiazolyl, piperidinyl, morpholyl, tetrahydroquinolinyl, oxazolyl, isoxazolyl, furyl thiophen, triazolyl, pyrrolidinyl ring optionally substituted with one or more hydroxy, alkylhydroxy, alkyl, alkoxy, trifluoromethyl, amino, alkylamino, dialkylamino, aminomethyl, alkylaminomethyl, dialkylaminomethyl, acylamino, cyano, halogen or oxo group;
R₂ is phenyl optionally substituted with one or more substituent(s) selected from alkyl, methoxy, trifluoromethyl, amino, alkylamino, dialkylamino, aminomethyl, alkylaminomethyl, dialkylaminomethyl, acylamino, alkylsulfonyl, alkylsulfonylamino, cyano, halogen, or
saturated or unsaturated monocyclic or bicyclic C₅₋₇ heterocyclyl group, containing 1-4 heteroatom(s) selected from O, N or S, such as pyridyl, quinolinyl, thiazolyl, piperidinyl, morpholyl, tetrahydroquinolinyl, oxazolyl, isoxazolyl, furyl thiophen, triazolyl, pyrrolidinyl ring optionally substituted with one or more hydroxy, alkylhydroxy, alkyl, alkoxy, trifluoromethyl, amino, alkylamino, dialkylamino, aminomethyl, alkylaminomethyl, dialkylaminomethyl, acylamino, cyano, halogen or oxo group, or
NR₃R₄ group wherein R₃ and R₄ are independently selected from the group of hydrogen and alkyl group optionally substituted with one or more substituent(s) selected from alkoxy, trifluoromethyl, amino, alkylamino, dialkylamino, aminomethyl, alkylaminomethyl, dialkylaminomethyl, acylamino, cyano, halogen, or R₃ and R₄ together with the N atom to which they are attached form a C₅₋₇ heterocyclyl group, containing one or more heteroatom(s), selected from O, N or S, optionally substituted with one or more hydroxy, alkylhydroxy, alkyl, alkoxy, trifluoromethyl, amino, alkylamino, dialkylamino, aminomethyl, alkylaminomethyl, dialkylaminomethyl, acylamino, cyano, halogen or oxo group, or
NH-CO-NR₅R₆ group, wherein R₅ and R₆ are independently selected from the group hydrogen and alkyl group optionally substituted with one or more substituent(s) selected from alkoxy, trifluoromethyl, amino, alkylamino, dialkylamino, aminomethyl, alkylaminomethyl, dialkylaminomethyl, acylamino, cyano, halogen, or R₅ and R₆ together with the N atom to which they are attached form a C₅₋₇ heterocyclyl group, containing one or more heteroatom(s), selected from O, N or S, optionally substituted with one or more hydroxy, alkylhydroxy, alkyl, alkoxy, trifluoromethyl, amino, alkylamino, dialkylamino, aminomethyl, alkylaminomethyl, dialkylaminomethyl, acylamino, cyano, halogen or oxo group and/or hydrates and/or solvates and/or pharmaceutically acceptable salts thereof formed with acids or bases.

3. A compound according to claim 1 selected from
2-(4-chloro-benzenesulfonyl)-3-(4-chloro-phenyl)-thieno[2,3-*b*]pyridine,
2-(4-chloro-benzenesulfinyl)-3-(4-chloro-phenyl)-thieno[2,3-*b*]pyridine,
3-(4-chloro-phenyl)-2-(4-fluoro-benzenesulfonyl)-thieno[2,3-*b*]pyridine,
3-(4-chloro-phenyl)-2-(toluene-4-sulfonyl)-thieno[2,3-*b*]pyridine,
4-[3-(4-chloro-phenyl)-thieno[2,3-*b*]pyridine-2-sulfonyl]-benzonitrile,
2-benzenesulfonyl-3-(4-chloro-phenyl)-thieno[2,3-*b*]pyridine,
3-[3-(4-chloro-phenyl)-thieno[2,3-*b*]pyridine-2-sulfonyl]-benzonitrile,
3-(4-chloro-phenyl)-2-(pyridine-3-sulfonyl)-thieno[2,3-*b*]pyridine,
2-(butane-1-sulfonyl)-3-(4-chloro-phenyl)-thieno[2,3-*b*]pyridine,
3-(4-chloro-phenyl)-2-(2,4-dimethyl-thiazole-5-sulfonyl)-thieno[2,3-*b*]pyridine,
3-(4-chloro-phenyl)-2-(thiophene-2-sulfonyl)-thieno[2,3-*b*]pyridine,
3-(4-chloro-phenyl)-thieno[2,3-*b*]pyridine-2-sulfonic acid (4-chloro-phenyl)-amide,
3-(4-chloro-phenyl)-2-phenylmethanesulfonyl-thieno[2,3-*b*]pyridine,
2-(3-chloro-benzenesulfonyl)-3-(4-chloro-phenyl)-thieno[2,3-*b*]pyridine,
2-(4-chloro-benzenesulfonyl)-3-(4-chloro-phenyl)-6-chloro-thieno[2,3-*b*]pyridin, 2-(3-fluoro-benzenesulfonyl)-3-(4-chloro-phenyl)- thieno[2,3-*b*]pyridin, 2-(3-cyano-benzenesulfonyl)-3-(3-fluoro-phenyl)-thieno[2,3-*b*]pyridine, 2-(4-chloro-benzenesulfonyl)-3-(4-chloro-phenyl)-6-fluoro-thieno[2,3-*b*]pyridin, 2-(4-cyano-benzenesulfonyl)-3-(3-chloro-phenyl)-thieno[2,3-*b*]pyridine, 2-(3-cyano-benzenesulfonyl)-3-(3-methyl-piperidinyl)-thieno[2,3-*b*]pyridine, 2-(3-cyano-benzenesulfonyl)-3-(4-methyl-piperidinyl)-thieno[2,3-*b*]pyridine, 2-(3-cyano-benzenesulfonyl)-3-(4-tolyl)-thieno[2,3-*b*]pyridine, 2-(3-cyano-benzenesulfonyl)-3-(3-methoxy-phenyl)-thieno[2,3-*b*]pyridine, 2-(3-methoxy-benzenesulfonyl)-3-(4-chloro-phenyl)-thieno[2,3-*b*]pyridine, 2-(3-cyano-5-fluoro-benzenesulfonyl)-3-(4-chloro-phenyl)-thieno[2,3-*b*]pyridine, 2-(3-fluoro-4-methyl-benzenesulfonyl)-3-(4-chloro-phenyl)-thieno[2,3-*b*]pyridine, 2-(3,4-dimethyl-benzenesulfonyl)-3-(4-fluoro-phenyl)-thieno[2,3-*b*]pyridine, 2-(3-cyano-benzenesulfonyl)-3-(4-fluoro-phenyl)-6-chloro-thieno[2,3-*b*]pyridine, 2-(3-cyano-5-fluoro-benzenesulfonyl)-3-(4-fluoro-phenyl)-thieno[2,3-*b*]pyridine, 2-(3-cyano-benzenesulfonyl)-3-(4-fluoro-phenyl)-6-fluoro-thieno[2,3-*b*]pyridine, 2-(4-bromo-benzenesulfonyl)-3-(4-chloro-phenyl)-thieno[2,3-*b*]pyridin, 5-amino-2-(4-chloro-benzenesulfonyl)-3-(4-chloro-phenyl)-thieno[2,3-*b*]pyridin, 2-(3,4-dichloro-benzenesulfonyl)-3-(4-chloro-phenyl)-thieno[2,3-*b*]pyridin, 2-(3-fluoro-4-methyl-benzenesulfonyl)-3-(4-fluoro-phenyl)-thieno[2,3-*b*]pyridine, 2-(3-cyano-benzenesulfonyl)-3-(2-chloro-phenyl)-thieno[2,3-*b*]pyridine, 2-(3-cyano-5-fluoro-benzenesulfonyl)-3-(4-chloro-phenyl)-6-chloro-thieno[2,3-*b*]pyridine, 2-(3-fluoro-4-methoxy-benzenesulfonyl)-3-(4-chloro-phenyl)-thieno[2,3-*b*]pyridine, 2-(4-chloro-benzenesulfonyl)-3-(4-methyl-piperidinyl)-thieno[2,3-*b*]pyridine, 2-(3-ciano-5-fluoro-benzenesulfonyl)-3-(4-methyl-piperidinyl)-thieno[2,3-*b*]pyridine, 2-(4-chloro-benzenesulfonyl)-3-(4-chloro-phenyl)-5-fluoro-thieno[2,3-*b*]pyridin, 2-(4-chloro-benzenesulfonyl)-3-(4-chloro-phenyl)-5-chloro-thieno[2,3-*b*]pyridin, 2-(4-chloro-benzenesulfonyl)-3-(4-chloro-phenyl)-4-chloro-thieno[2,3-*b*]pyridin, 2-(4-chloro-benzenesulfonyl)-3-(4-chloro-phenyl)-4-fluoro-thieno[2,3-*b*]pyridin, 5-amino-2-(4-chloro-benzenesulfonyl)-3-(4-methyl-piperidinyl)-thieno[2,3-*b*]pyridin, 2-(3-cyano-5-fluoro-benzenesulfonyl)-3-(3-fluoro-phenyl)-thieno[2,3-*b*]pyridine, 5-amino-2-(4-chloro-benzenesulfonyl)-3-(3-fluoro-phenyl)-thieno[2,3-*b*]pyridin, 2-(4-chloro-benzenesulfonyl)-3-(4-methyl-piperidinyl)-6-chloro-thieno[2,3-*b*]pyridin,
2-(3-fluoro-4-methoxy-benzenesulfonyl)-3-(4-fluoro-phenyl)-thieno[2,3-*b*]pyridine,
2-(3-fluoro-4-methoxy-benzenesulfonyl)-3-(3-fluoro-phenyl)-thieno[2,3-*b*]pyridine,
2-(3-cyano-5-fluoro-benzenesulfonyl)-3-(2-fluoro-phenyl)-thieno[2,3-*b*]pyridine,
2-(3-cyano-5-fluoro-benzenesulfonyl)-3-(3-chloro-phenyl)-thieno[2,3-*b*]pyridine.

4. A process for preparing a compound of formula (Ia) 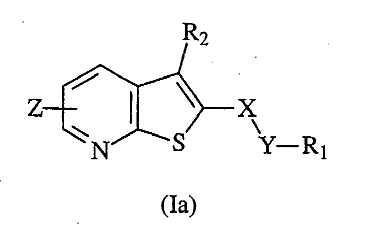 wherein
X represents a group selected from SO, SO₂;
Y represents a group selected from (CH₂)ₙ, NH, NHCH₂;
n is an integer of 0 to 1;
Z is H or monosubstituted by alkyl or nitro, group
R₁ is an optionally substituted alkyl, cycloalkyl, phenyl, biphenyl, heterocyclyl;
R₂ is phenyl, optionally substituted with alkyl or halogen;
by reacting a compound of formula (III): 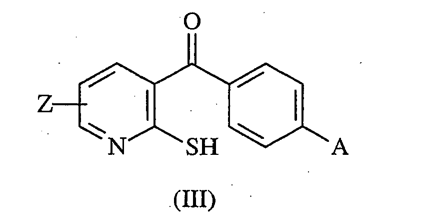 wherein A is a substituent as defined R₂ in formula (Ia) and Z is H or monosubstituted by alkyl group, or monosubstituted by nitro with a compound of formula (VII):
HlgCH₂SOYR₁ (VII)
wherein Hlg is chloro or bromo, Y and R₁ are as described above for the formula (Ia), or with a compound of formula (VIII):
HlgCH₂SO₂YR₁ (VIII)
wherein Hlg is chloro or bromo, Y and R₁ are as described above for the formula (Ia) in the presence of sodium methoxide in dimethylformamide as solvent to obtain a compound of formula (Ia), and optionally thereafter forming salts and/or hydrates and/or solvates of compounds of formula (Ia).

5. A process for preparing a compound of formula (Ib) 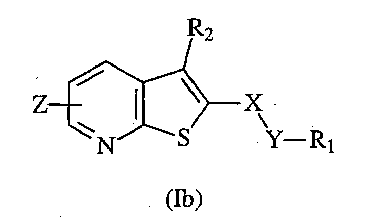 wherein
X represents SO, SO₂;
Y represents a group selected from (CH₂)ₙ, NH, NHCH₂;
n is an integer of 0 to 1;
Z is H or alkyl group;
R₁ is an optionally substituted alkyl, cycloalkyl, phenyl, biphenyl, heterocyclyl;
R₂ is phenyl, or aromatic heterocyclyl, optionally substituted with one or more substituent(s) selected from alkyl, alkoxy, trifluoromethyl, amino, alkylamino, dialkylamino, aminomethyl, alkylaminomethyl, dialkylaminomethyl, acylamino, cyano, fluoro, chloro, bromo, hydroxyl, methylsulfonyl,
by reacting a compound of formula (XI): 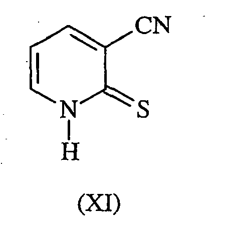 with a compound of formula (VII):
HlgCH₂SOYR₁ (VII)
wherein Hlg is chloro or bromo, Y and R₁ are as described above for the formula (Ib), or with a compound of formula (VIII):
HlgCH₂SO₂YR₁ (VIII)
wherein Hlg is chloro or bromo, Y and R₁ are as described above for the formula (Ib) in the presence of sodium methoxide in dimethylformamide as solvent and
reacting the obtained compound of formula (IV): 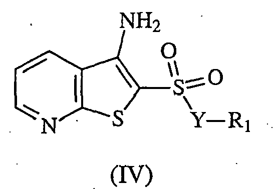 wherein R₁ are as described above for the formula (Ib) with copper(II) bromide and *tert*-butyl nitrite in acetonitrile; and reacting the obtained compound of formula (V): 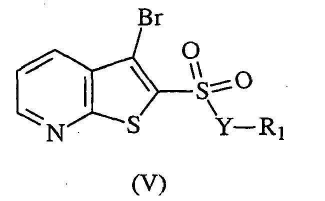 wherein R₁ are as described above for the formula (Ib) with a compound of formula (IX):
R₂-B(OH)₂ (IX)
wherein R₂ is as defined above for formula (Ib), in the presence of base and catalyst in a solvent to obtain a compound of formula (Ib), and optionally thereafter forming salts and/or hydrates and/or solvates of compounds of formula (Ib).

6. A process for preparing a compound of formula (Ic): 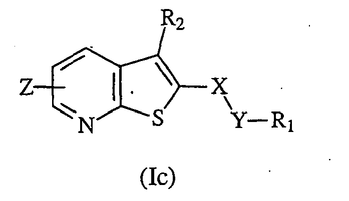 wherein
X represents SO₂;
Y represents a group selected from (CH₂)ₙ, NH, NHCH₂;
n is an integer of 0 to 1;
Z is H or alkyl group;
R₁ is an optionally substituted alkyl, cycloalkyl, phenyl, biphenyl, heterocyclyl;
R₂ is NR₃R₄ wherein R₃ and R₄ are independently selected from the group of hydrogen an optionally substituted alkyl group, or R₃ and R₄ together with the N atom to which they are attached form an optionally substituted C₅₋₇ heterocyclyl group, containing one or more heteroatom(s),
by reacting a compound of formula (V): 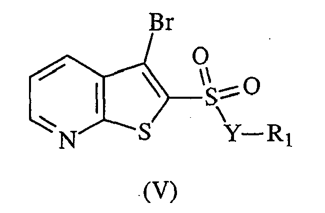 wherein R₁ are as described above for the formula (Ic),
with a compound of formula (XVI):
HNR₃R₄ (XVI)
wherein R₃ and R₄ are as described above for formula (Ic) to obtain compound of formula (Ic), and optionally thereafter forming salts and/or hydrates and/or solvates of compounds of formula (Ic).

7. A process for preparing a compound of formula (Id): 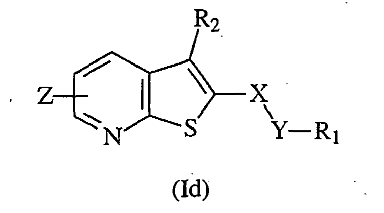 wherein
X represents SO₂;
Y represents a group selected from (CH₂)ₙ, NH, NHCH₂;
n is an integer of 0 to 1;
Z is H or alkyl group;
R₁ is an optionally substituted alkyl, cycloalkyl, phenyl, biphenyl, heterocyclyl;
R₂ is NH-CO-NR₃R₄ group, wherein R₅ and R₆ are independently selected from the group of hydrogen or optionally substituted alkyl group, or R₃ and R₄ together with the N atom to which they are attached form an optionally substituted C₅₋₇ heterocyclyl group, containing one or more heteroatom(s),
by reacting a compound of formula (IV): 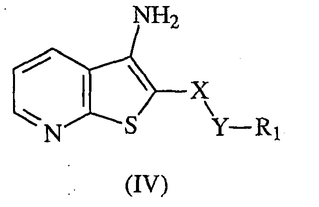 wherein R₁ are as described above for the formula (Id), with a compound of formula (XIII):
HNR₃R₄ (XIII)
wherein R₃ and R₄ are as described above for formula (Id), in the presence of phosgene or triphosgene and base to obtain a compound of formula (Id), and optionally thereafter forming salts and/or hydrates and/or solvates of compounds of formula (Id).

8. A process for preparing a compound of formula (Ie): 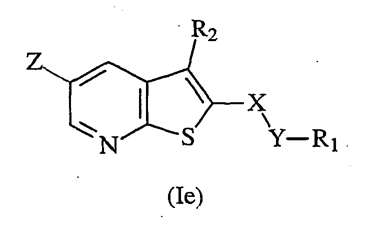 wherein
X represents a group selected from SO, SO₂;
Y represents a group selected from (CH₂)ₙ, NH, NHCH₂;
n is an integer of 0 to 1;
Z is amino, alkylsulfonylamino, halogen;
R₁ is an optionally substituted alkyl, cycloalkyl, phenyl, biphenyl, heterocyclyl;
R₂ is phenyl, optionally substituted with alkyl or halogen;
by reacting a compound of formula (Ia): 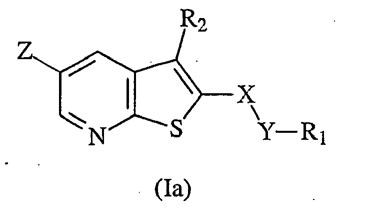 wherein
X represents a group selected from SO, SO₂;
Y represents a group selected from (CH₂)ₙ, NH, NHCH₂;
n is an integer of 0 to 1;
Z is nitro;
R₁ is an optionally substituted alkyl, cycloalkyl, phenyl, biphenyl, heterocyclyl;
R₂ is phenyl, optionally substituted with alkyl or halogen,
with an appropriate reducing agent to obtain compound of formula (Ie) 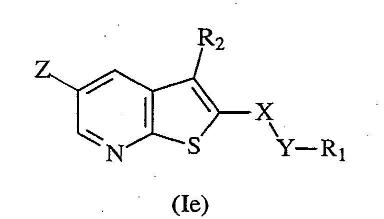 wherein Z is amino; X, Y, n, R₁ and R₂ are as described above for formula (Ie) and optionally thereafter forming salts and/or hydrates and/or solvates of the obtained amino derivatives, or
a. optionally reacting the obtained amino derivatives of formula (Ie), wherein Z is amino; X, Y, n, R₁ and R₂ are as described above for formula (Ie) with sodium nitrite in the presence of hydrochloric acid and copper(I) iodide or copper(I) chloride or copper(I) bromide or sodium tetrafluoroborate to obtain a compound of formula (Ie), ), wherein Z is halogen; X, Y, n, R₁ and R₂ are as described above for formula (Ie), and optionally thereafter forming salts and/or hydrates and/or solvates of the obtained halogen derivatives of formula Ie or
b. optionally reacting the obtained amino derivatives of formula (Ie), wherein Z is amino; X, Y, n, R₁ and R₂ are as described above for formula (Ie) with alkylsulfonyl chloride derivatives to obtain compounds of formula (Ie), wherein Z is alkylsulfonylamino; X, Y, n, R₁ and R₂ are as described above for formula (Ie), and optionally thereafter forming salts and/or hydrates and/or solvates of the obtained alkylsulfonylamino derivatives of formula (Ie).

9. A process for preparing a compound of formula (If): 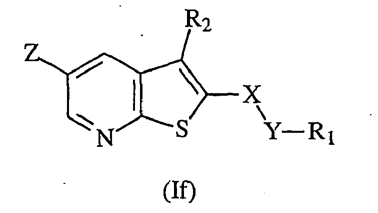 wherein
X represents SO₂;
Y represents a group selected from (CH₂)ₙ, NH, NHCH₂;
n is an integer of 0 to 1;
Z is amino, alkylsulfonylamino, halogen;
R₁ is an optionally substituted alkyl, cycloalkyl, phenyl, biphenyl, heterocyclyl;
R₂ is optionally substituted phenyl, heterocyclyl, or NR₃R₄ group wherein R₃ and R₄ are independently selected from the group of hydrogen or optionally substituted alkyl, or R₃ and R₄ together with the N atom to which they are attached form an optionally substituted C₅₋₇ heterocyclyl group, containing one or more heteroatom(s),
by reacting a compound of formula (V): 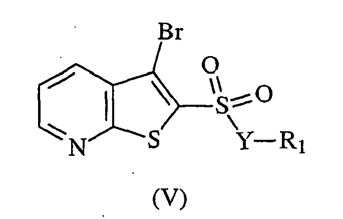 wherein R₁ and Y are as described above for the formula (If), with an appropriate oxidizing agent and reacting the obtained N-oxide derivative of formula (X): 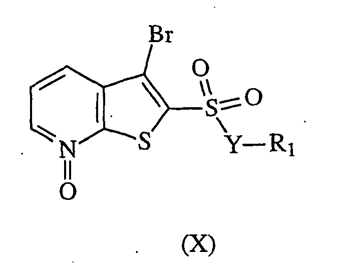 wherein R₁ and Y are as described above for the formula (If), with aquaeous nitric acid in acetic acid, thereafter reacting the obtained nitro derivative of formula (XI): 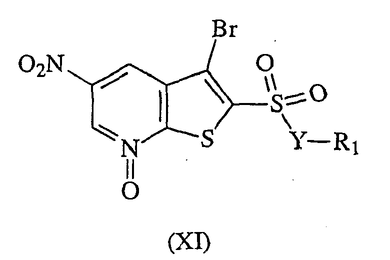 wherein R₁ and Y are as described above for the formula (If), with an appropriate reducing agent, and reacting the obtained amino derivative of formula (XII): 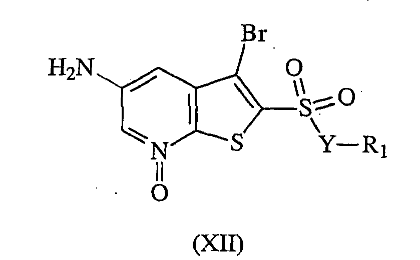 wherein R₁ and Y are as described above for the formula (If),
a. with a compound of formula (IX):
R₂-B(OH)₂ (IX)
wherein R₂ is optionally substituted phenyl, heterocyclyl, in the presence of base and catalyst in a solvent to obtain a compound of formula (If), wherein Z is amino; R₂ is optionally substituted phenyl, heterocyclyl, Y and R₁ are as described above for formula (If) and optionally thereafter forming salts and/or hydrates and/or solvates of the obtained amino derivatives of formula (If), and
(i) optionally reacting the obtained amino derivatives of formula (If), wherein Z is amino; R₂ is optionally substituted phenyl, heterocyclyl, Y and R₁ are as described above for formula (If), with sodium nitrite in the presence of hydrochloric acid and copper(I) iodide or copper(I) chloride or copper(I) bromide or sodium tetrafluoroborate to obtain a compound of formula (If), wherein Z is halogen; R₂ is optionally substituted phenyl, heterocyclyl, Y and R₁ are as described above for formula (If), and optionally thereafter forming salts and/or hydrates and/or solvates of the obtained halogen derivatives of formula (If), or
(ii) optionally reacting the obtained amino of formula (If), wherein Z is amino;
R2 is optionally substituted phenyl, heterocyclyl, Y and R1 are as described above for formula (If), with alkylsulfonyl chloride derivatives to obtain a compound of formula (If), and optionally thereafter forming salts and/or hydrates and/or solvates of the obtained alkylsulfonylamino derivatives of formula (If), or
b. with a compound of (XIII):
NHR₃R₄ (XIII)
wherein R₃ and R₄ are as described above for formula (If), at 70-200°C to obtain a compound of formula (If), wherein Z is amino; Y, R₁ and R₂ which is NR₃R₄ group, are as described above for formula (If) and optionally thereafter forming salts and/or hydrates and/or solvates of the obtained amino derivatives of formula (If), or
(i) optionally reacting the obtained amino derivatives of formula (If), wherein Z is amino; Y, R₁ and R₂ which is NR₃R₄ group, are as described above for formula (If) with sodium nitrite in the presence of hydrochloric acid and copper(I) iodide or copper(I) chloride or copper(I) bromide or sodium tetrafluoroborate to obtain a compound of formula (If), wherein Z is halogen Y, R₁ and R₂ which is NR₃R₄ group, are as described above for formula (If) and optionally thereafter forming salts and/or hydrates and/or solvates of the obtained halogen derivatives of formula (If), or
(ii) optionally reacting the obtained amino derivatives wherein Z is amino; Y, R₁ and R₂ which is NR₃R₄ group, are as described above for formula (If) with alkylsulfonyl chloride derivatives a compound of formula (If), wherein Z alkylsulfonylamino; Y, R₁ and R₂ which is NR₃R₄ group, are as described above for formula (If), and optionally thereafter forming salts and/or hydrates and/or solvates of the obtained alkylsulfonylamino derivatives of formula (If).

10. A process for preparing a compound of formula (Ig): 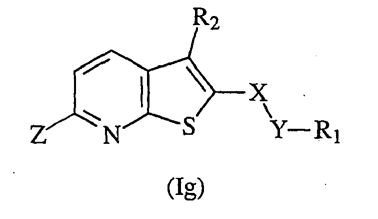 wherein
X represents SO₂;
Y represents a group selected from (CH₂)ₙ, NH, NHCH₂;
n is an integer of 0 to 1;
Z is amino, bromo, chloro, iodo, methoxy, mono- or dialkyamino;
R₁ is an optionally substituted alkyl, cycloalkyl, phenyl, biphenyl, heterocyclyl;
R₂ is an optionally substituted phenyl, heterocyclyl, or
NR₃R₄ group wherein R₃ and R₄ are independently selected from the group of hydrogen or an optionally substituted alkyl, or R₃ and R₄ together with the N atom to which they are attached form an optionally substituted C₅₋₇ heterocyclyl group, containing one or more heteroatom(s),
by reacting a compound of formula (X): 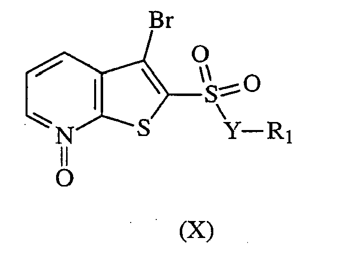 wherein R₁ and Y are as described above for the formula (Ig), with a compound of formula (IX):
R₂-B(OH)₂ (IX)
wherein R₂ is optionally substituted phenyl, heterocyclyl, in the presence of base and catalyst in a solvent, to obtain compounds of formula (XIV): 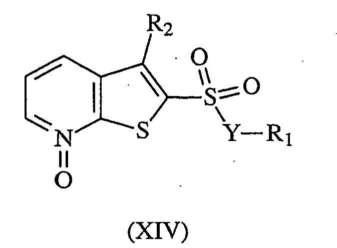 wherein R₂ is an optionally substituted phenyl, heterocyclyl, R₁ and Y are as described above for compounds of formula (Ig), or
with a compound of (XIII):
NHR₃R₄ (XIII)
wherein R₃ and R₄ are as described above for formula (Ig) to obtain compounds of formula (XIV), wherein R₂ is NR₃R₄ group, wherein R₃ and R₄ are independently selected from the group of hydrogen or an optionally substituted alkyl, or R₃ and R₄ together with the N atom to which they are attached form an optionally substituted C₅₋₇ heterocyclyl group, containing one or more heteroatom(s); and
reacting compounds of formula (XIV), wherein R₁, R₂ and Y are as described above for formula (Ig), with trifluoroacetic anhydride in a solvent to obtain compounds of formula (XV): 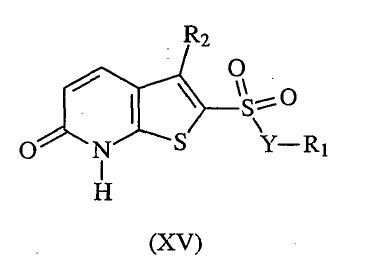 wherein R₁, R₂ and Y are as described above for formula (Ig); and reacting compounds of formula (XV) (wherein R₁, R₂ and Y are as described above for formula (Ig),
a. with phosphorous(III) oxybromide to obtain compounds of formula (Ig), wherein Z is bromo, X, Y, n, R₁ and R₂ are as described above for formula (Ig), and optionally thereafter forming salts and/or hydrates and/or solvates of the obtained bromo derivatives of formula (Ig); or
(i) reacting the bromo derivatives of formula (Ig), wherein Z is bromo; X, Y, n, R₁ and R₂ are as described above for formula (Ig)) with potassium fluoride in DMSO to obtain compounds of formula (Ig), wherein Z is fluoro, X, Y, n, R₁ and R₂ are as described above for formula (Ig), and optionally thereafter forming salts and/or hydrates and/or solvates of the obtained fluoro derivatives of formula (Ig); or
b. with phosphorous(III) oxychloride to obtain compounds of formula (Ig), wherein Z is chloro, X, Y, n, R₁ and R₂ are as described above for formula (Ig), and optionally thereafter forming salts and/or hydrates and/or solvates of the obtained chloro deriivatives of formula (Ig); or
(i) reacting the chloro derivatives of formula (Ig), wherein Z is chloro; X, Y, n, R₁ and R₂ are as described above for formula (Ig)) with potassium fluoride in DMSO to obtain compounds of formula (Ig), wherein Z is fluoro, X, Y, n, R₁ and R₂ are as described above for formula (Ig), and optionally thereafter forming salts and/or hydrates and/or solvates of the obtained fluoro derivatives of formula (Ig); or
c. with iodomethane in the presence of silver carbonate in a solvent to obtain compounds of formula (Ig), wherein Z is methoxy, X, Y, n, R₁ and R₂ are as described above for formula (Ig), and optionally thereafter forming salts and/or hydrates and/or solvates of the obtained methoxy derivatives of formula (Ig); or
d. with trifluoromethanesulfonic anhydride in a solvent and then with ammonia or mono- and dialkylamines to obtain compounds of formula (Ig), wherein Z is respectivelly amino or monoalkylamino or dialkylamino, X, Y, n, R₁ and R₂ are as described above for formula (Ig), and optionally thereafter forming salts and/or hydrates and/or solvates of the obtained methoxy derivatives of formula (Ig).

11. A process for preparing a compound of formula (Ih): 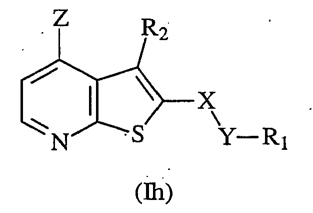 wherein
X represents SO₂;
Y represents a group selected from (CH₂)ₙ, NH, NHCH₂;
n is an integer of 0 to 1;
Z is amino, hydroxy, bromo, chloro, fluoro, methoxy, mono- or dialkyamino;
R₁ is an optionally substituted alkyl, cycloalkyl, phenyl, biphenyl, heterocyclyl;
R₂ is optionally substituted phenyl, heterocyclyl, or
NR₃R₄ group wherein R₃ and R₄ are independently selected from the group of hydrogen or an optionally substituted alkyl, or R₃ and R₄ together with the N atom to which they are attached form an optionally substituted C₅₋₇ heterocyclyl group, containing one or more heteroatom(s),
by reacting a compound of formula (XIV): 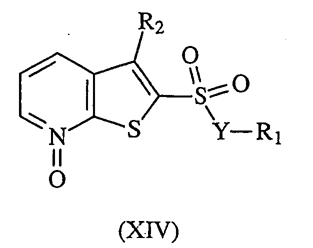 wherein R₁, R₂ and Y are as described above for formula (Ih),
a. with phosphorous(III) oxychloride or phosphorous pentachloride to obtain compounds of formula (Ih), wherein Z is chloro, X, Y, n, R₁ and R₂ are as described above for formula (Ih), or
b. with phosphorous(III) oxybromide to obtain compounds of formula (Ih), wherein Z is chloro, X, Y, n, R₁ and R₂ are as described above for formula (Ih), and optionally thereafter forming salts and/or hydrates and/or solvates of the obtained chloro or bromo derivatives of formula (Ih), wherein Z is chloro or bromo; X, Y, n, R₁ and R₂ are as described above for formula (Ih)); or
(i) reacting the chloro or bromo derivatives of formula (Ih), wherein Z is chloro or bromo; X, Y, n, R₁ and R₂ are as described above for formula (Ih) with potassium fluoride in DMSO to obtain compounds of formula (Ih), wherein Z is fluoro, X, Y, n, R₁ and R₂ are as described above for formula (Ih), and optionally thereafter forming salts and/or hydrates and/or solvates of the obtained fluoro derivatives of formula (Ih); or
(ii) reacting the chloro or bromo derivatives formula (Ih), wherein Z is chloro or bromo; X, Y, n, R₁ and R₂ are as described above for formula (Ih) with sodium methylate in methanol to obtain compounds of formula (Ih), wherein Z is methoxy, X, Y, n, R₁ and R₂ are as described above for formula (Ih), and optionally thereafter forming salts and/or hydrates and/or solvates of the obtained methoxy derivatives of formula (Ih), or
(iii) reacting the chloro or bromo derivatives of formula (Ih), wherein Z is chloro or bromo; X, Y, n, R₁ and R₂ are as described above for formula (Ih)) with sodium acetate in acetic acid to obtain compounds of formula (Ih), wherein Z is hydroxy, X, Y, n, R₁ and R₂ are as described above for formula (Ih), and optionally thereafter forming salts and/or hydrates and/or solvates of the obtained hydroxy derivatives of formula (Ih), or
(iv) reacting the chloro or bromo derivatives formula (Ih), wherein Z is chloro or bromo; X, Y, n, R₁ and R₂ are as described above for formula (Ih) with trifluoromethanesulfonic anhydride in a solvent and then with ammonia or mono- and dialkylamines to obtain compounds of formula (Ih), wherein Z is respectivelly amino or monoalkylamino or dialkylamino, X, Y, n, R₁ and R₂ are as described above for formula (Ih), and optionally thereafter forming salts and/or hydrates and/or solvates of the obtained methoxy derivatives of formula (Ih), wherein Z is respectivelly amino or monoalkylamino or dialkylamino; or
(v) reacting the chloro or bromo derivatives of formula (Ih), wherein Z is chloro or bromo; X, Y, n, R₁ and R₂ are as described above for formula (Ih)) with sodium azide in DMF and water to obtain compounds of formula (Ih), wherein Z is azido, X, Y, n, R₁ and R₂ are as described above for formula (Ih), then reacting the obtained azido derivatives with sodium borohydride in a solvent to obtain compounds of formula (Ih), wherein Z is amino, X, Y, n, R₁ and R₂ are as described above for formula (Ih), and optionally thereafter forming salts and/or hydrates and/or solvates of the obtained amino derivatives of formula (Ih).

12. A pharmaceutical formulation comprising a therapeutically effective amount of a compound of formula (I): 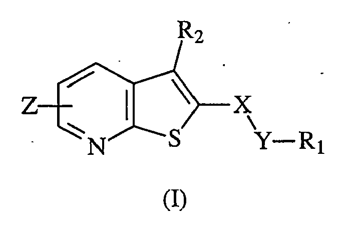 wherein
X represents a group selected from SO, SO₂;
Y represents a group selected from (CH₂)ₙ, NH, N-HCH₂;
n is an integer of 0 to 1;
Z is H or monosubstituted by alkyl, nitro, halogen, *alk*oxy, trifluoromethyl, cyano, amino, alkylamino, dialkylamino, aminomethyl, alkylaminomethyl, dialkylaminomethyl, hydroxyl, alkylsulfonylamino;
R₁ is an optionally substituted alkyl, cycloalkyl, phenyl, biphenyl, heterocyclyl;
R₂ is an optionally substituted phenyl, heterocyclyl, or
NR₃R₄ group wherein R₃ and R₄ are independently selected from the group of hydrogen and an optionally substituted alkyl, or R₃ and R₄ together with the N atom to which they are attached form an optionally substituted C₅₋₇ heterocyclyl group, containing one or more heteroatom(s), or
NH-CO-NR₅R₆ group, wherein R₅ and R₆ are independently selected from the group of hydrogen and an optionally substituted alkyl, or R₅ and R₆ together with the N atom to which they are attached form an optionally substituted C₅₋₇ heterocyclyl group, containing one or more heteroatom(s); and/or hydrates and/or solvates and/or pharmaceutically acceptable salts thereof formed with acids or bases in association with one or more physiologically acceptable diluents, excipients and/or inert carriers.

13. A pharmaceutical composition according to claim 12. for use in the prevention and/or treatment of mGluR1 and mGluR5 receptor-mediated disorders.

14. The use of a compound of formula (I): 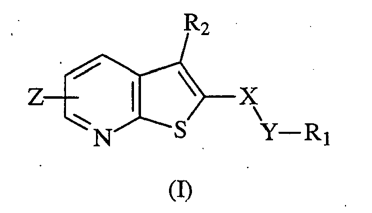 wherein
X represents a group selected from SO, SO₂
Y represents a group selected from (CH₂)ₙ, NH, NHCH₂;
n is an integer of 0 to 1;
Z is H or monosubstituted by alkyl, nitro, halogen, *alk*oxy, trifluoromethyl, cyano, amino, alkylamino, dialkylamino, aminomethyl, alkylaminomethyl, dialkylaminomethyl, hydroxyl, alkylsulfonylamino;
R₁ is an optionally substituted alkyl, cycloalkyl, phenyl, biphenyl, heterocyclyl;
R₂ is an optionally substituted phenyl, heterocyclyl, or
NR₃R₄ group wherein R3 and R₄ are independently selected from the group of hydrogen and an optionally substituted alkyl, or R₃ and R₄ together with the N atom to which they are attached form an optionally substituted C₅₋₇ heterocyclyl group, containing one or more heteroatom(s), or
NH-CO-NR₅R₆ group, wherein R₅ and R₆ are independently selected from the group of hydrogen and an optionally substituted alkyl, or R₅ and R₆ together with the N atom to which they are attached form an optionally substituted C₅₋₇ heterocyclyl group, containing one or more heteroatom(s); and/or hydrates and/or solvates and/or pharmaceutically acceptable salts thereof formed with acids or bases for the manufacture of a medicament for the treatment and/or prevention of mGluR1 and mGluR5 receptor-mediated disorders.

15. The use of a compound according to claim 14. wherein said mGluR1 and mGluR5 receptor-mediated disorders are psychiatric disorders.

16. The use of a compound according to claim 14. wherein said mGluR1 and mGluR5 receptor-mediated disorders are neurological disorders.

17. The use of a compound according to claim 14. wherein said mGluR1 and mGluR5 receptor-mediated disorders are chronic and acute pain.

18. The use of a compound according to claim 14. wherein said mGluR1 and mGluR5 receptor-mediated disorders are neuromuscular dysfunction of the lower urinary tract and gastrointestinal disorders.

19. A compound of formula (I): 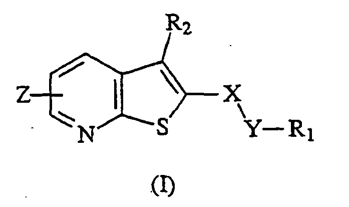 wherein
X represents a group selected from SO, SO₂;
Y represents a group selected from (CH₂)ₙ, NH, NHCH₂;
n is an integer of 0 to 1;
Z is H or monosubstituted by alkyl, nitro, halogen, *alk*oxy, trifluoromethyl, cyano, amino, alkylamino, dialkylamino, aminomethyl, alkylaminomethyl, dialkylaminomethyl, hydroxyl, alkylsulfonylamino;
R₁ is an optionally substituted alkyl, cycloalkyl, phenyl, biphenyl, heterocyclyl;
R₂ is an optionally substituted phenyl, heterocyclyl, or
NR₃R₄ group wherein R₃ and R₄ are independently selected from the group of hydrogen and an optionally substituted alkyl, or R₃ and R₄ together with the N atom to which they are attached form an optionally substituted C₅₋₇ heterocyclyl group, containing one or more heteroatom(s), or
NH-CO-NR₅R₆ group, wherein R₅ and R₆ are independently selected from the group of hydrogen and an optionally substituted alkyl, or R₅ and R₆ together with the N atom to which they are attached form an optionally substituted C₅₋₇ heterocyclyl group, containing one or more heteroatom(s); and/or hydrates and/or solvates and/or pharmaceutically acceptable salts thereof formed with acids or bases,
for use in a method of prevention and/or treatment of mGluR1 and mGluR5 receptor-mediated disorders.

20. The compound as claimed in claim 19., wherein said mGluR1 and mGluR5 receptor-mediated disorders are psychiatric disorders.

21. The compound as claimed in claim 19., wherein said mGluR1 and mGluR5 receptor-mediated disorders are neurological disorders.

22. The compound as claimed in claim 19., wherein said mGluR1 and mGluR5 receptor-mediated disorders are chronic and acute pain disorders.

23. The compound as claimed in claim 19., wherein said mGluR1 and mGluR5 receptor-mediated disorders are neuromuscular dysfunction of the lower urinary tract and gastrointestinal disorders.

## Patentansprüche

1. Verbindung der Formel (I): 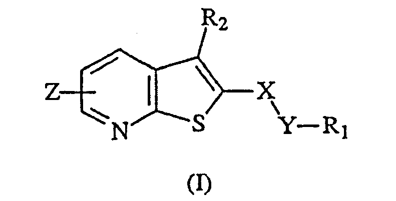 worin:
X eine Gruppe darstellt, die ausgewählt ist aus SO oder SO₂;
Y eine Gruppe darstellt, die ausgewählt ist aus (CH₂)ₙ, NH oder NHCH₂;
n eine ganze Zahl von 0 bis 1 ist;
Z H oder mit Alkyl, Nitro, Halogen, Alkoxy, Trifluormethyl, Cyano, Amino, Alkylamino, Dialkylamino, Aminomethyl, Alkylaminomethyl, Dialkylaminomethyl, Hydroxy oder Alkylsulfonylamino monosubstituiert ist;
R₁ ein gegebenenfalls substituiertes Alkyl, Cycloalkyl, Phenyl, Biphenyl oder Heterocyclyl ist;
R₂ ein gegebenenfalls substituiertes Phenyl oder Heterocyclyl oder eine NR₃R₄-Gruppe, worin R₃ und R₄ unabhängig ausgewählt sind aus der Gruppe aus Wasserstoff und einem gegebenenfalls substituierten Alkyl, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e), oder eine NH-CO-NR₅R₆-Gruppe, worin R₅ und R₆ unabhängig ausgewählt sind aus der Gruppe aus Wasserstoff und einem gegebenenfalls substituierten Alkyl, oder R₅ und R₆ zusammen mit dem N-Atom, an das sie gebunden sind, eine gegebenenfalls substituierte C₅₋₇-Heterocyclylgruppe bilden, enthaltend ein oder mehrere Heteroatom(e), ist;
und/oder Hydrate und/oder Solvate und/oder pharmazeutisch annehmbare Salze davon, gebildet mit Säuren oder Basen.

2. Verbindung gemäß Anspruch 1 der Formel (I): 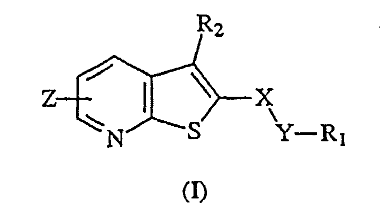 worin
X eine Gruppe darstellt, die ausgewählt ist aus SO oder SO₂;
Y eine Gruppe darstellt, die ausgewählt ist aus (CH₂)ₙ, NH oder NHCH₂;
n eine ganze Zahl von 0 bis 1 ist;
Z H oder mit Alkyl, Nitro, Halogen, Alkoxy, Trifluormethyl, Cyano, Amino, Alkylamino, Dialkylamino, Aminomethyl, Alkylaminomethyl, Dialkylaminomethyl, Hydroxy oder Alkylsulfonylamino monosubstituiert ist;
R₁ eine Alkyl- oder C₃₋₁₀-Cycloalkylgruppe, gegebenenfalls substituiert mit einem oder mehreren Substituent(en), ausgewählt aus Alkoxy, Trifluormethyl, Amino, Alkylamino, Dialkylamino, Aminomethyl, Alkylaminomethyl, Dialkylaminomethyl, Acylamino, Cyano, Halogen, oder
ein Phenyl oder Biphenyl, gegebenenfalls substituiert mit einem oder mehreren Substituent(en), ausgewählt aus Alkyl, Methoxy, Trifluormethyl, Amino, Alkylamino, Dialkylamino, Aminomethyl, Alkylaminomethyl, Dialkylaminomethyl, Acylamino, Alkylsulfonyl, Alkylsulfonylamino, Cyano, Halogen, oder
ein gesättigtes oder ungesättigtes monocyclisches oder bicyclisches Heterocyclyl, enthaltend 1-4 Heteroatom(e), ausgewählt aus O, N oder S, wie Pyridyl, Chinolinyl, Thiazolyl, Piperidinyl, Morpholyl, Tetrahydrochinolinyl, Oxazolyl, Isoxazolyl, Furylthiophen, Triazolyl, Pyrrolidinylring, gegebenenfalls substituiert mit einem oder mehreren Hydroxy, Alkylhydroxy, Alkyl, Alkoxy, Trifluormethyl, Amino, Alkylamino, Dialkylamino, Aminomethyl, Alkylaminomethyl, Dialkylaminomethyl, Acylamino, Cyano, Halogen oder Oxogruppe, ist;
R₂ Phenyl, gegebenenfalls substituiert mit einem oder mehreren Substituent(en), ausgewählt aus Alkyl, Methoxy, Trifluormethyl, Amino, Alkylamino, Dialkylamino, Aminomethyl, Alkylaminomethyl, Dialkylaminomethyl, Acylamino, Alkylsulfonyl, Alkylsulfonylamino, Cyano, Halogen, oder
eine gesättigte oder ungesättigte monocyclische oder bicyclische C₅₋₇-Heterocyclylgruppe, enthaltend 1-4 Heteroatom(e), ausgewählt aus O, N oder S, wie Pyridyl, Chinolinyl, Thiazolyl, Piperidinyl, Morpholyl, Tetrahydrochinolinyl, Oxazolyl, Isoxazolyl, Furylthiophen, Triazolyl, Pyrrolidinylring, gegebenenfalls substituiert mit einem oder mehreren Hydroxy, Alkylhydroxy, Alkyl, Alkoxy, Trifluormethyl, Amino, Alkylamino, Dialkylamino, Aminomethyl, Alkylaminomethyl, Dialkylaminomethyl, Acylamino, Cyano, Halogen oder Oxogruppe, oder
eine NR₃R₄-Gruppe, worin R₃ und R₄ unabhängig ausgewählt sind aus der Gruppe aus Wasserstoff und einer Alkylgruppe, gegebenenfalls substituiert mit einem oder mehreren Substituent(en), ausgewählt aus Alkoxy, Trifluormethyl, Amino, Alkylamino, Dialkylamino, Aminomethyl, Alkylaminomethyl, Dialkylaminomethyl, Acylamino, Cyano, Halogen, oder R₃ und R₄ zusammen mit dem N-Atom, an das sie gebunden sind, eine C₅₋₇-Heterocyclylgruppe bilden, enthaltend ein oder mehrere Heteroatom(e), ausgewählt aus O, N oder S, gegebenenfalls substituiert mit einem oder mehreren Hydroxy, Alkylhydroxy, Alkyl, Alkoxy, Trifluormethyl, Amino, Alkylamino, Dialkylamino, Aminomethyl, Alkylaminomethyl, Dialkylaminomethyl, Acylamino, Cyano, Halogen oder Oxogruppe, oder
eine NH-CO-NR₅R₆-Gruppe, worin R₅ und R₆ unabhängig ausgewählt sind aus der Gruppe aus Wasserstoff und einer Alkylgruppe, gegebenenfalls substituiert mit einem oder mehreren Substituent(en), ausgewählt aus Alkoxy, Trifluormethyl, Amino, Alkylamino, Dialkylamino, Aminomethyl, Alkylaminomethyl, Dialkylaminomethyl, Acylamino, Cyano, Halogen, oder R₅ und R₆ zusammen mit dem N-Atom, an das sie gebunden sind, eine C₅₋₇-Heterocyclylgruppe bilden, enthaltend ein oder mehrere Heteroatom(e), ausgewählt aus O, N oder S, gegebenenfalls substituiert mit einem oder mehreren Hydroxy, Alkylhydroxy, Alkyl, Alkoxy, Trifluormethyl, Amino, Alkylamino, Dialkylamino, Aminomethyl, Alkylaminomethyl, Dialkylaminomethyl, Acylamino, Cyano, Halogen oder Oxogruppe, ist,
und/oder Hydrate und/oder Solvate und/oder pharmazeutisch annehmbare Salze davon, gebildet mit Säuren oder Basen.

3. Verbindung gemäß Anspruch 1, ausgewählt aus:
2-(4-Chloro-benzolsulfonyl)-3-(4-chloro-phenyl)-thieno[2,3-b]pyridin,
2-(4-Chloro-benzolsulfinyl)-3-(4-chloro-phenyl)-thieno[2,3-b]pyridin,
3-(4-Chloro-phenyl)-2-(4-fluoro-benzolsulfonyl)-thieno[2,3-b]pyridin,
3-(4-Chloro-phenyl)-2-(toluol-4-sulfonyl)-thieno[2,3-b]pyridin,
4-[3-(4-Chloro-phenyl)-thieno[2,3-b]pyridin-2-sulfonyl]-benzonitril,
2-Benzolsulfonyl-3-(4-chloro-phenyl)-thieno[2,3-b]pyridin,
3-[3-(4-Chloro-phenyl)-thieno[2,3-b]pyridin-2-sulfonyl]-benzonitril,
3-(4-Chloro-phenyl)-2-(pyridin-3-sulfonyl)-thieno[2,3-b]pyridin,
2-(Butan-1-sulfonyl)-3-(4-chloro-phenyl)-thieno[2,3-b]pyridin,
3-(4-Chloro-phenyl)-2-(2,4-dimethyl-thiazol-5-sulfonyl)-thieno[2,3-b]pyridin,
3-(4-Chloro-phenyl)-2-(thiophen-2-sulfonyl)-thieno[2,3-b]pyridin,
3-(4-Chloro-phenyl)-thieno[2,3-b]pyridin-2-sulfonsäure-(4-chloro-phenyl)-amid,
3-(4-Chloro-phenyl)-2-phenylmethansulfonylthieno[2,3-b]pyridin,
2-(3-Chloro-benzolsulfonyl)-3-(4-chloro-phenyl)-thieno[2,3-b]pyridin,
2-(4-Chloro-benzolsulfonyl)-3-(4-chloro-phenyl)-6-chlorothieno[2,3-b]pyridin,
2-(3-Fluoro-benzolsulfonyl)-3-(4-chloro-phenyl)-thieno[2,3-b]pyridin,
2-(3-Cyano-benzolsulfonyl)-3-(3-fluoro-phenyl)-thieno[2,3-b]-pyridin,
2-(4-Chloro-benzolsulfonyl)-3-(4-chloro-phenyl)-6-fluorothieno[2,3-b]pyridin,
2-(4-Cyano-benzolsulfonyl)-3-(3-chloro-phenyl)-thieno[2,3-b]pyridin,
2-(3-Cyano-benzolsulfonyl)-3-(3-methyl-piperidinyl)-thieno[2,3-b]pyridin,
2-(3-Cyano-benzolsulfonyl)-3-(4-methyl-piperidinyl)-thieno[2,3-b]pyridin,
2-(3-Cyano-benzolsulfonyl)-3-(4-tolyl)-thieno[2,3-b]pyridin,
2-(3-Cyano-benzolsulfonyl)-3-(3-methoxy-phenyl)-thieno[2,3-b]pyridin,
2-(3-Methoxy-benzolsulfonyl)-3-(4-chloro-phenyl)-thieno[2,3-b]pyridin,
2-(3-Cyano-5-fluoro-benzolsulfonyl)-3-(4-chloro-phenyl)-thieno[2,3-b]pyridin,
2-(3-Fluoro-4-methyl-benzolsulfonyl)-3-(4-chloro-phenyl)-thieno[2,3-b]pyridin,
2-(3,4-Dimethyl-benzolsulfonyl)-3-(4-fluoro-phenyl)-thieno[2,3-b]pyridin,
2-(3-Cyano-benzolsulfonyl)-3-(4-fluoro-phenyl)-6-chlorothieno[2,3-b]pyridin,
2-(3-Cyano-5-fluoro-benzolsulfonyl)-3-(4-fluoro-phenyl)-thieno[2,3-b]pyridin,
2-(3-Cyano-benzolsulfonyl)-3-(4-fluoro-phenyl)-6-fluorothieno[2,3-b]pyridin,
2-(4-Bromo-benzolsulfonyl)-3-(4-chloro-phenyl)-thieno[2,3-b]pyridin,
5-Amino-2-(4-chloro-benzolsulfonyl)-3-(4-chloro-phenyl)-thieno[2,3-b]pyridin,
2-(3,4-Dichloro-benzolsulfonyl)-3-(4-chloro-phenyl)-thieno[2,3-b]pyridin,
2-(3-Fluoro-4-methyl-benzolsulfonyl)-3-(4-fluoro-phenyl)-thieno[2,3-b]pyridin,
2-(3-Cyano-benzolsulfonyl)-3-(2-chloro-phenyl)-thieno[2,3-b]pyridin,
2-(3-Cyano-5-fluoro-benzolsulfonyl)-3-(4-chloro-phenyl)-6-chloro-thieno[2,3-b]pyridin,
2-(3-Fluoro-4-methoxy-benzolsulfonyl)-3-(4-chloro-phenyl)-thieno[2,3-b]pyridin,
2-(4-Chloro-benzolsulfonyl)-3-(4-methyl-piperidinyl)-thieno[2,3-b]pyridin,
2-(3-Cyano-5-fluoro-benzolsulfonyl)-3-(4-methylpiperidinyl)-thieno[2,3-b]pyridin,
2-(4-Chloro-benzolsulfonyl)-3-(4-chloro-phenyl)-5-fluoro-thieno[2,3-b]pyridin,
2-(4-Chloro-benzolsulfonyl)-3-(4-chloro-phenyl)-5-chloro-thieno[2,3-b]pyridin,
2-(4-Chloro-benzolsulfonyl)-3-(4-chloro-phenyl)-4-chloro-thieno[2,3-b]pyridin,
2-(4-Chloro-benzolsulfonyl)-3-(4-chloro-phenyl)-4-fluoro-thieno[2,3-b]pyridin,
5-Amino-2-(4-chloro-benzolsulfonyl)-3-(4-methyl-piperidinyl)-thieno[2,3-b]pyridin,
2-(3-Cyano-5-fluoro-benzolsulfonyl)-3-(3-fluoro-phenyl)-thieno[2,3-b]pyridin,
5-Amino-2-(4-chloro-benzolsulfonyl)-3-(3-fluoro-phenyl)-thieno[2,3-b]pyridin,
2-(4-Chloro-benzolsulfonyl)-3-(4-methyl-piperidinyl)-6-chloro-thieno[2,3-b]pyridin,
2-(3-Fluoro-4-methoxy-benzolsulfonyl)-3-(4-fluoro-phenyl)-thieno[2,3-b]pyridin,
2-(3-Fluoro-4-methoxy-benzolsulfonyl)-3-(3-fluoro-phenyl)-thieno[2,3-b]pyridin,
2-(3-Cyano-5-fluoro-benzolsulfonyl)-3-(2-fluoro-phenyl)-thieno[2,3-b]pyridin,
2-(3-Cyano-5-fluoro-benzolsulfonyl)-3-(3-chloro-phenyl)-thieno[2,3-b]pyridin.

4. Verfahren zur Herstellung einer Verbindung der Formel (Ia) 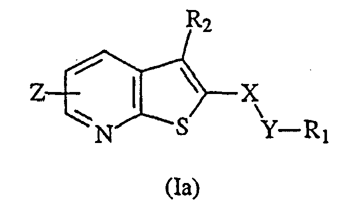 worin
X eine Gruppe darstellt, die ausgewählt ist aus SO oder SO₂;
Y eine Gruppe darstellt, die ausgewählt ist aus (CH₂)ₙ, NH oder NHCH₂;
n eine ganze Zahl von 0 bis 1 ist;
Z H ist oder mit einer Alkyl- oder Nitrogruppe monosubstituiert ist;
R₁ ein gegebenenfalls substituiertes Alkyl, Cycloalkyl, Phenyl, Biphenyl oder Heterocyclyl ist;
R₂ Phenyl ist, das gegebenenfalls mit Alkyl oder Halogen substituiert ist;
durch Umsetzen einer Verbindung der Formel (III): 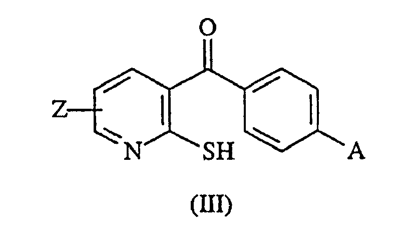 worin A ein wie R₂ in Formel (Ia) definierter Substituent ist und Z H ist oder mit einer Alkylgruppe monosubstituiert oder mit Nitro monosubstituiert ist, mit einer Verbindung der Formel (VII):
WgCH₂SOYR₁ (VII)
worin Hlg Chlor oder Brom ist und Y und R₁ wie vorstehend für die Formel (Ia) beschrieben sind, oder mit einer Verbindung der Formel (VIII):
HlgCH₂SO₂YR₁ (VIII)
worin Hlg Chlor oder Brom ist und Y und R₁ wie vorstehend für die Formel (Ia) beschrieben sind, in Gegenwart von Natriummethoxid in Dimethylformamid als Lösungsmittel zum Erhalten einer Verbindung der Formel (Ia), und gegebenenfalls anschließend Bilden von Salzen und/oder Hydraten und/oder Solvaten der Verbindungen der Formel (Ia).

5. Verfahren zur Herstellung einer Verbindung der Formel (Ib) 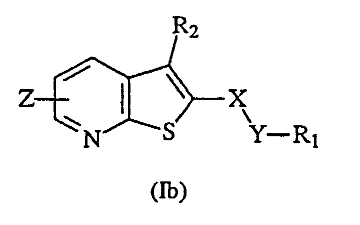 worin
X SO oder SO₂ darstellt;
Y eine Gruppe darstellt, die ausgewählt ist aus (CH₂)ₙ, NH oder NHCH₂;
n eine ganze Zahl von 0 bis 1 ist;
Z H oder eine Alkylgruppe ist;
R₁ ein gegebenenfalls substituiertes Alkyl, Cycloalkyl, Phenyl, Biphenyl oder Heterocyclyl ist;
R₂ Phenyl oder ein aromatisches Heterocyclyl ist, gegebenenfalls substituiert mit einem oder mehreren Substituent(en), ausgewählt aus Alkyl, Alkoxy, Trifluormethyl, Amino, Alkylamino, Dialkylamino, Aminomethyl, Alkylaminomethyl, Dialkylaminomethyl, Acylamino, Cyano, Fluor, Chlor, Brom, Hydroxy, Methylsulfonyl,
durch Umsetzen einer Verbindung der Formel (XI): 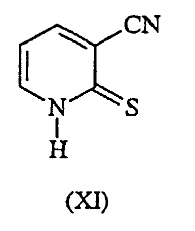 mit einer Verbindung der Formel (VII):
Hl_{g}CH₂SOYR₁ (VII)
worin Hlg Chlor oder Brom ist und Y und R₁ wie vorstehend für die Formel (Ib) beschrieben sind, oder mit einer Verbindung der Formel (VIII):
Hl_{g}CH₂SO₂YR₁ (VIII)
worin Hlg Chlor oder Brom ist und Y und R₁ wie vorstehend für die Formel (Ib) beschrieben sind, in Gegenwart von Natriummethoxid in Dimethylformamid als Lösungsmittel und Umsetzen der erhaltenen Verbindung der Formel (IV): 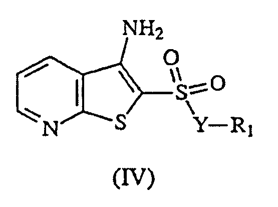 worin R₁ wie vorstehend für die Formel (Ib) beschrieben ist, mit Kupfer(II)bromid und tert-Butylnitrit in Acetonitril; und Umsetzen der erhaltenen Verbindung der Formel (V): 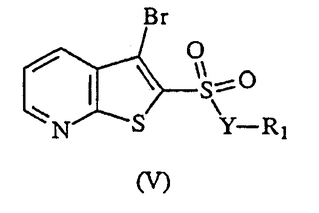
worin R₁ wie vorstehend für die Formel (Ib) beschrieben ist, mit einer Verbindung der Formel (IX):
R₂-B(OH)₂ (IX)
worin R₂ wie vorstehend für Formel (Ib) beschrieben ist, in Gegenwart einer Base und eines Katalysators in einem Lösungsmittel zum Erhalten einer Verbindung der Formel (Ib), und gegebenenfalls anschließend Bilden von Salzen und/oder Hydraten und/oder Solvaten der Verbindungen der Formel (Ib).

6. Verfahren zur Herstellung einer Verbindung der Formel (Ic): 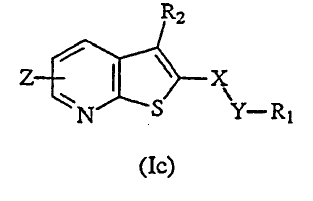 worin
X SO₂ darstellt;
Y eine Gruppe darstellt, die ausgewählt ist aus (CH₂)ₙ, NH oder NHCH₂;
n eine ganze Zahl von 0 bis 1. ist;
Z H oder eine Alkylgruppe ist;
R₁ ein gegebenenfalls substituiertes Alkyl, Cycloalkyl, Phenyl, Biphenyl oder Heterocyclyl ist;
R₂ NR₃R₄ ist, worin R₃ und R₄ unabhängig ausgewählt sind aus der Gruppe aus Wasserstoff oder einer gegebenenfalls substituierten Alkylgruppe, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e),
durch Umsetzen einer Verbindung der Formel (V): 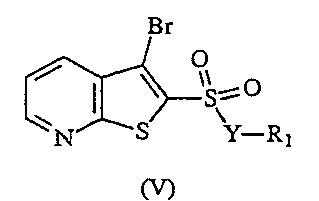 worin R₁ wie vorstehend für die Formel (Ic) beschrieben ist,
mit einer Verbindung der Formel (XVI):
HNR₃R₄ (XVI)
worin R₃ und R₄ wie vorstehend für Formel (Ic) beschrieben sind, zum Erhalten einer Verbindung der Formel (Ic), und gegebenenfalls anschließend Bilden von Salzen und/oder Hydraten und/oder Solvaten der Verbindungen der Formel (Ic).

7. Verfahren zur Herstellung einer Verbindung der Formel (Id): 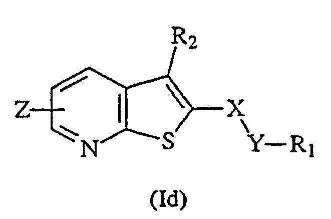 worin
X SO₂ darstellt;
Y eine Gruppe darstellt, die ausgewählt ist aus (CH₂)ₙ, NH oder NHCH₂;
n eine ganze Zahl von 0 bis 1 ist;
Z H oder eine Alkylgruppe ist;
R₁ ein gegebenenfalls substituiertes Alkyl, Cycloalkyl, Phenyl, Biphenyl oder Heterocyclyl ist;
R₂ eine NH-CO-NR₃R₄-Gruppe ist, worin R₅ und R₆ unabhängig ausgewählt sind aus der Gruppe aus Wasserstoff oder einer gegebenenfalls substituierten Alkylgruppe, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e),
durch Umsetzen einer Verbindung der Formel (IV): 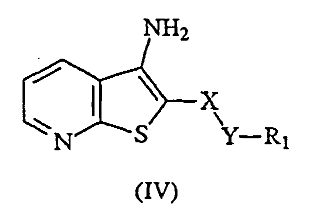 worin R₁ wie vorstehend für die Formel (Id) beschrieben ist, mit einer Verbindung der Formel (XIII):
HNR₃R₄ (XIII)
worin R₃ und R₄ wie vorstehend für Formel (Id) beschrieben sind, in Gegenwart von Phosgen oder Triphosgen und einer Base zum Erhalten einer Verbindung der Formel (Id), und gegebenenfalls anschließend Bilden von Salzen und/oder Hydraten und/oder Solvaten der Verbindungen der Formel (Id).

8. Verfahren zur Herstellung einer Verbindung der Formel (Ie): 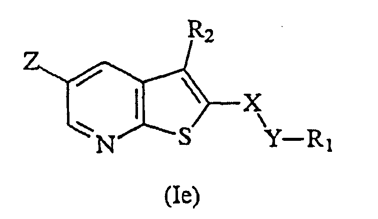 worin
X eine Gruppe darstellt, die ausgewählt ist aus SO oder SO₂;
Y eine Gruppe darstellt, die ausgewählt ist aus (CH₂)ₙ, NH oder NHCH₂;
n eine ganze Zahl von 0 bis 1 ist;
Z Amino, Alkylsulfonylamino oder Halogen ist;
R₁ ein gegebenenfalls substituiertes Alkyl, Cycloalkyl, Phenyl, Biphenyl oder Heterocyclyl ist;
R₂ Phenyl ist, das gegebenenfalls mit Alkyl oder Halogen substituiert ist;
durch Umsetzen einer Verbindung der Formel (Ia): 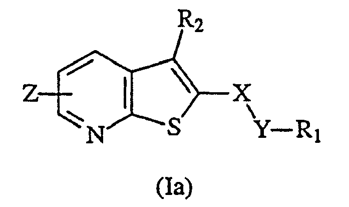 worin
X eine Gruppe darstellt, die ausgewählt ist aus SO oder SO₂;
Y eine Gruppe darstellt, die ausgewählt ist aus (CH₂)ₙ, NH oder NHCH₂;
n eine ganze zahl von 0 bis 1 ist;
Z Nitro ist;
R₁ ein gegebenenfalls substituiertes Alkyl, Cycloalkyl, Phenyl, Biphenyl oder Heterocyclyl ist;
R₂ Phenyl ist, das gegebenenfalls mit Alkyl oder Halogen substituiert, ist,
mit einem zweckdienlichen Reduktionsmittel zum Erhalten einer Verbindung der Formel (Ie) 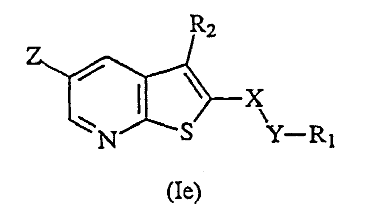 worin Z Amino ist; X, Y, n, R₁ und R₂ wie vorstehend für Formel (Ie) beschrieben sind, und gegebenenfalls anschließend Bilden von Salzen und/oder Hydraten und/oder Solvaten der erhaltenen Aminoderivate, oder
a. gegebenenfalls Umsetzen der erhaltenen Aminoderivate der Formel (Ie), worin Z Amino ist; X, Y, n, R₁ und R₂ wie vorstehend für Formel (Ie) beschrieben sind, mit Natriumnitrit in Gegenwart von Salzsäure und Kupfer(I)iodid oder Kupfer(I)chlorid oder Kupfer(I)bromid oder Natriumtetrafluorborat zum Erhalten einer Verbindung der Formel (Ie), worin Z Halogen ist; X, Y, n, R₁ und R₂ wie vorstehend für Formel (Ie) beschrieben sind, und gegebenenfalls anschließend Bilden von Salzen und/oder Hydraten und/oder Solvaten der erhaltenen Halogenderivate der Formel (Ie), oder
b. gegebenenfalls Umsetzen der erhaltenen Aminoderivate der Formel (Ie), worin Z Amino ist; X, Y, n, R₁ und R₂ wie vorstehend für Formel (Ie) beschrieben sind, mit Alkylsulfonylchloridderivaten zum Erhalten von Verbindungen der Formel (Ie), worin Z Alkylsulfonylamino ist; X, Y, n, R₁ und R₂ wie vorstehend für Formel (Ie) beschrieben sind, und gegebenenfalls anschließend Bilden von Salzen und/oder Hydraten und/oder Solvaten der erhaltenen Alkylsulfonylaminoderivate der Formel (Ie).

9. Verfahren zum Herstellen einer Verbindung der Formel (If): 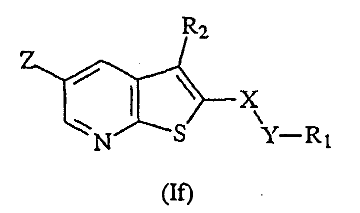 worin
X SO₂ darstellt;
Y eine Gruppe darstellt, die ausgewählt ist aus (CH₂)ₙ, NH oder NHCH₂;
n eine ganze Zahl von 0 bis 1 ist;
Z Amino, Alkylsulfonylamino oder Halogen ist;
R₁ ein gegebenenfalls substituiertes Alkyl, Cycloalkyl, Phenyl, Biphenyl oder Heterocyclyl ist;
R₂ ein gegebenenfalls substituiertes Phenyl, Heterocyclyl oder eine NR₃R₄-Gruppe ist, worin R₃ und R₄ unabhängig ausgewählt sind aus der Gruppe aus Wasserstoff oder einem gegebenenfalls substituiertem Alkyl, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e),
durch Umsetzen einer Verbindung der Formel (V): 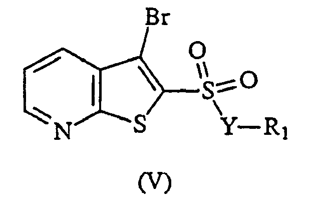 worin R₁ und Y wie vorstehend für die Formel (If) beschrieben sind, mit einem zweckdienlichen Oxidationsmittel und Umsetzen des erhaltenen N-Oxidderivats der Formel (X): 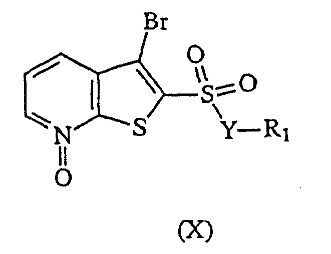 worin R₁ und Y wie vorstehend für die Formel (If) beschrieben sind, mit wässriger Salpetersäure in Essigsäure, anschließend Umsetzen des erhaltene Nitroderivats der Formel (XI): 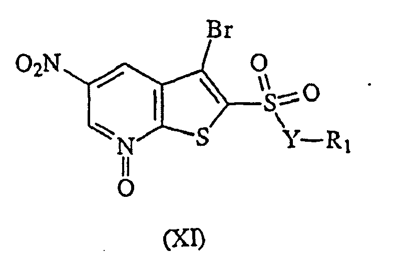 worin R₁ und Y wie vorstehend für die Formel (If) beschrieben sind, mit einem zweckdienlichen Reduktionsmittel und Umsetzen des erhaltenen Aminoderivats der Formel (XII): 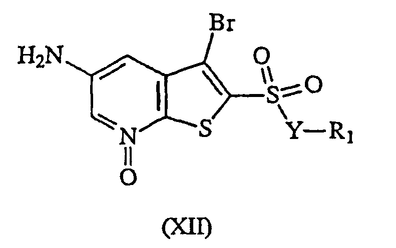 worin R₁ und Y wie vorstehend für die Formel (If) beschrieben sind,
a. mit einer Verbindung der Formel (IX):
R₂-B(OH)₂ (IX)
worin R₂ ein gegebenenfalls substituiertes Phenyl oder Heterocyclyl ist, in Gegenwart einer Base und eines Katalysators in einem Lösungsmittel zum Erhalten einer Verbindung der Formel (If), worin Z Amino ist; R₂ ein gegebenenfalls substituiertes Phenyl oder Heterocyclyl ist, Y und R₁ wie vorstehend für die Formel (If) beschrieben sind, und gegebenenfalls anschließend Bilden von Salzen und/oder Hydraten und/oder Solvaten der erhaltenen Aminoderivate der Formel (If), und
(i) gegebenenfalls Umsetzen der erhaltenen Aminoderivate der Formel (If), worin Z Amino ist; R₂ ein gegebenenfalls substituiertes Phenyl oder Heterocyclyl ist, Y und R₁ wie vorstehend für die Formel (If) beschrieben sind, mit Natriumnitrit in Gegenwart von Salzsäure und Kupfer(I)iodid oder Kupfer(I)chlorid oder Kupfer(I)bromid oder Natriumtetrafluorborat zum Erhalten einer Verbindung der Formel (If), worin Z Halogen ist; R₂ ein gegebenenfalls substituiertes Phenyl oder Heterocyclyl ist, Y und R₁ wie vorstehend für Formel (If) beschrieben sind, und gegebenenfalls anschließend Bilden von Salzen und/oder Hydraten und/oder Solvaten der erhaltenen Halogenderivate der Formel (If), oder
(ii) gegebenenfalls Umsetzen der erhaltenen Amino der Formel (If), worin Z Amino ist; R₂ gegebenenfalls ein substituiertes Phenyl oder Heterocyclyl ist, Y und R₁ wie vorstehend für die Formel (If) beschrieben sind, mit Alkylsulfonylchloridderivaten zum Erhalten einer Verbindung der Formel (If), und gegebenenfalls anschließend Bilden von Salzen und/oder Hydraten und/oder Solvaten der erhaltenen Alkylsulfonylaminoderivate der Formel (If), oder
b. mit einer Verbindung der Formel (XIII):
NHR₃R₄ (XIII)
worin R₃ und R₄ wie vorstehend für Formel (If) beschrieben sind, bei 70 bis 200°C zum Erhalten einer Verbindung der Formel (If), worin Z Amino ist; Y, R₁ und R₂, das eine NR₃R₄-Gruppe ist, wie vorstehend für Formel (If) beschrieben sind, und gegebenenfalls anschließend Bilden von Salzen und/oder Hydraten und/oder Solvaten der erhaltene Aminoderivate der Formel (If), oder
(i) gegebenenfalls Umsetzen der erhaltenen Aminoderivate der Formel (If), worin Z Amino ist; Y, R₁ und R₂, das eine NR₃R₄-Gruppe ist, wie vorstehend für die Formel (If) beschrieben sind, mit Natriumnitrit in Gegenwart von Salzsäure und Kupfer(I)iodid oder Kupfer(I)chlorid oder Kupfer(I)bromid oder Natriumtetrafluorborat zum Erhalten einer Verbindung der Formel (If), worin Z Halogen ist, Y, R₁ und R₂, das eine NR₃R₄-Gruppe ist, wie vorstehend für die Formel (If) beschrieben sind, und gegebenenfalls anschließend Bilden von Salzen und/oder Hydraten und/oder Solvaten der erhaltenen Halogenderivate der Formel (If), oder
(ii) gegebenenfalls Umsetzen der erhaltenen Aminoderivate, worin Z Amino ist; Y, R₁ und R₂, das eine NR₃R₄-Gruppe ist, wie vorstehend für die Formel (If) beschrieben sind, mit Alkylsulfonylchloridderivaten einer Verbindung der Formel (If), worin Z Alkylsulfonylamino ist; Y, R₁ und R₂, das eine NR₃R₄-Gruppe ist, wie vorstehend für Formel (If) beschrieben sind, und gegebenenfalls anschließend Bilden von Salzen und/oder Hydraten und/oder Solvaten der erhaltene Alkylsulfonylaminoderivate der Formel (If).

10. Verfahren zur Herstellung einer Verbindung der Formel (Ig): 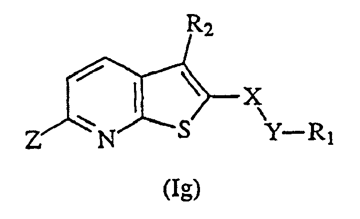 worin
X SO₂ darstellt;
Y eine Gruppe darstellt, die ausgewählt ist aus (CH₂)ₙ, NH oder NHCH₂;
n eine ganze Zahl von 0 bis 1 ist;
Z Amino, Brom, Chlor, Iod, Methoxy, Mono- oder Dialkyamino ist;
R₁ ein gegebenenfalls substituiertes Alkyl, Cycloalkyl, Phenyl, Biphenyl oder Heterocyclyl ist;
R₂ ein gegebenenfalls substituiertes Phenyl oder Heterocyclyl oder
eine NR₃R₄-Gruppe ist, worin R₃ und R₄ unabhängig ausgewählt sind aus der Gruppe aus Wasserstoff oder einem gegebenenfalls substituierten Alkyl, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e),
durch Umsetzen einer Verbindung der Formel (X): 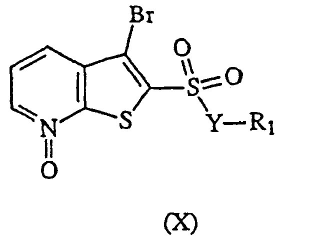 worin R₁ und Y wie vorstehend für die Formel (Ig) beschrieben sind, mit einer Verbindung der Formel (IX):
R₂₋B(OH)₂ (IX)
worin R₂ ein gegebenenfalls substituiertes Phenyl oder Heterocyclyl ist, in Gegenwart einer Base und eines Katalysators in einem Lösungsmittel zum Erhalten von Verbindungen der Formel (XIV): 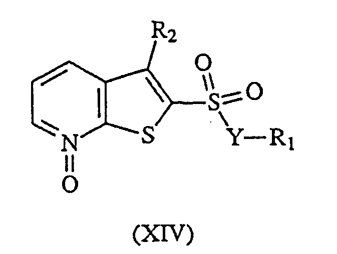 worin R₂ ein gegebenenfalls substituiertes Phenyl oder Heterocyclyl ist, R₁ und Y wie vorstehend für Verbindungen der Formel (Ig) beschrieben sind, oder
mit einer Verbindung der Formel (XIII):
NHR₃R₄ (XIII)
worin R₃ und R₄ wie vorstehend für Formel (Ig) beschrieben sind, zum Erhalten von Verbindungen der Formel (XIV), worin R₂ eine NR₃R₄-Gruppe ist, worin R₃ und R₄ unabhängig ausgewählt sind aus der Gruppe aus Wasserstoff oder einem gegebenenfalls substituierten Alkyl, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e); und
Umsetzen von Verbindungen der Formel (XIV), worin R₁, R₂ und Y wie vorstehend für Formel (Ig) beschrieben sind), mit Trifluoressigsäureanhydrid in einem Lösungsmittel zum Erhalten von Verbindungen der Formel (XV): 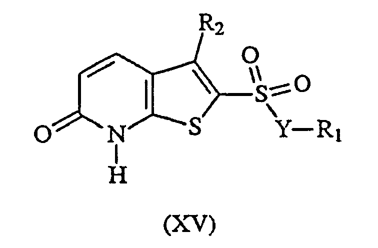 worin R₁, R₂ und Y wie vorstehend für Formel (Ig) beschrieben sind; und Umsetzen von Verbindungen der Formel (XV) (worin R₁, R₂ und Y wie vorstehend für Formel (Ig) beschrieben sind),
a. mit Phosphor(III)oxybromid zum Erhalten von Verbindungen der Formel (Ig), worin Z Brom ist, X, Y, n, R₁ und R₂ wie vorstehend für Formel (Ig) beschrieben sind, und gegebenenfalls anschließend Bilden von Salzen und/oder Hydraten und/oder Solvaten der erhaltenen Bromderivate der Formel (Ig); oder
(i) Umsetzen der Bromderivate der Formel (Ig), worin Z Brom ist; X, Y, n, R₁ und R₂ wie vorstehend für Formel (Ig) beschrieben sind, mit Kaliumfluorid in DMSO zum Erhalten von Verbindungen der Formel (Ig), worin Z Fluor ist, X, Y, n, R₁ und R₂ wie vorstehend für Formel (Ig) beschrieben sind, und gegebenenfalls anschließend Bilden von Salzen und/oder Hydraten und/oder Solvaten der erhaltenen Fluorderivate der Formel (Ig); oder
b. mit Phosphor(III)oxychlorid zum Erhalten von Verbindungen der Formel (Ig), worin Z Chlor ist, X, Y, n, R₁ und R₂ wie vorstehend für Formel (Ig) beschrieben sind, und gegebenenfalls anschließend Bilden von Salzen und/oder Hydraten und/oder Solvaten der erhaltenen Chlorderivate der Formel (Ig); oder
(i) Umsetzen der Chlorderivate der Formel (Ig), worin Z Chlor ist; X, Y, n, R₁ und R₂ wie vorstehend für Formel (Ig) beschrieben sind, mit Kaliumfluorid in DMSO zum Erhalten von Verbindungen der Formel (Ig), worin Z Fluor ist, X, Y, n, R₁ und R₂ wie vorstehend für Formel (Ig) beschrieben sind, und gegebenenfalls anschließend Bilden von Salzen und/oder Hydraten und/oder Solvaten der erhaltenen Fluorderivate der Formel (Ig); oder
c. mit Iodomethan in Gegenwart von Silbercarbonat in einem Lösungsmittel zum Erhalten von Verbindungen der Formel (Ig), worin Z Methoxy ist, X, Y, n, R₁ und R₂ wie vorstehend für Formel (Ig) beschrieben sind, und gegebenenfalls anschließend Bilden von Salzen und/oder Hydraten und/oder Solvaten der erhaltenen Methoxyderivate der Formel (Ig); oder
d. mit Trifluormethansulfonanhydrid in einem Lösungsmittel und dann mit Ammoniak oder Mono- und Dialkylaminen zum Erhalten von Verbindungen der Formel (Ig), worin Z respektive Amino oder Monoalkylamino oder Dialkylamino ist, X, Y, n, R₁ und R₂ wie vorstehend für Formel (Ig) beschrieben sind, und gegebenenfalls anschließend Bilden von Salzen und/oder Hydraten und/oder Solvaten der erhaltenen Methoxyderivate der Formel (Ig).

11. Verfahren zur Herstellung einer Verbindung der Formel (Ih): 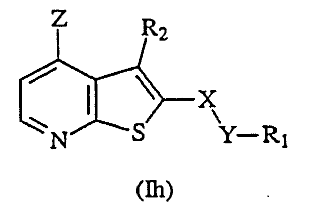 worin X SO₂ darstellt;
Y eine Gruppe darstellt, die ausgewählt ist aus (CH₂)ₙ. NH oder NHCH₂;
n eine ganze Zahl von 0 bis 1 ist;
Z Amino, Hydroxy, Brom, Chlor, Fluor, Methoxy, Mono- oder Dialkyamino ist;
R₁ ein gegebenenfalls substituiertes Alkyl, Cycloalkyl, Phenyl, Biphenyl oder Heterocyclyl ist;
R₂ ein gegebenenfalls substituiertes Phenyl oder Heterocyclyl oder eine NR₃R₄-Gruppe ist, worin R₃ und R₄ unabhängig ausgewählt sind aus der Gruppe aus Wasserstoff oder einem gegebenenfalls substituierten Alkyl, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e),
durch Umsetzen einer Verbindung der Formel (XIV): 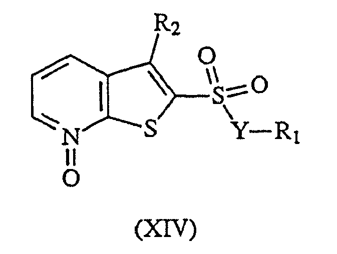 worin R₁, R₂ und Y wie vorstehend für Formel (Ih) beschrieben sind,
a. mit Phosphor(III)oxychlorid oder Phosphorpentachlorid zum Erhalten von Verbindungen der Formel (Ih), worin Z Chlor ist, X, Y, n, R₁ und R₂ wie vorstehend für Formel (Ih) beschrieben sind, oder
b. mit Phosphor(III)oxybromid zum Erhalten von Verbindungen der Formel (Ih), worin Z Chlor ist, X, Y, n, R₁ und R₂ wie vorstehend für Formel (Ih) beschrieben sind, und gegebenenfalls anschließend Bilden von Salzen und/oder Hydraten und/oder Solvaten der erhaltenen Chlor- oder Bromderivate der Formel (Ih), worin Z Chlor oder Brom ist; X, Y, n, R₁ und R₂ wie vorstehend für Formel (Ih) beschrieben sind; oder
(i) Umsetzen der Chlor- oder Bromderivate der Formel (Ih), worin Z Chlor oder Brom ist; X, Y, n, R₁ und R₂ wie vorstehend für Formel (Ih) beschrieben sind, mit Kaliumfluorid in DMSO zum Erhalten von Verbindungen der Formel (Ih), worin Z Fluor ist, X, Y, n, R₁ und R₂ wie vorstehend für Formel (Ih) beschrieben sind, und gegebenenfalls anschließend Bilden von Salzen und/oder Hydraten und/oder Solvaten der erhaltenen Fluorderivate der Formel (Ih); oder
(ii) Umsetzen der Chlor- oder Bromderivate der Formel (Ih), worin Z Chlor oder Brom ist; X, Y, n, R₁ und R₂ wie vorstehend für Formel (Ih) beschrieben sind, mit Natriummethylat in Methanol zum Erhalten von Verbindungen der Formel (Ih), worin Z Methoxy ist, X, Y, n, R₁ und R₂ wie vorstehend für Formel (Ih) beschrieben sind, und gegebenenfalls anschließend Bilden von Salzen und/oder Hydraten und/oder Solvaten der erhaltenen Methoxyderivate der Formel (Ih), oder
(iii) Umsetzen der Chlor- oder Bromderivate der Formel (Ih), worin Z Chlor oder Brom ist; X, Y, n, R₁ und R₂ wie vorstehend für Formel (Ih) beschrieben sind, mit Natriumacetat in Essigsäure zum Erhalten von Verbindungen der Formel (Ih), worin Z Hydroxy ist, X, Y, n, R₁ und R₂ wie vorstehend für Formel (Ih) beschrieben sind, und gegebenenfalls anschließend Bilden von Salzen und/oder Hydraten und/oder Solvaten der erhaltenen Hydroxyderivate der Formel (Ih), oder
(iv) Umsetzen der Chlor- oder Bromderivate der Formel (Ih), worin Z Chlor oder Brom ist; X, Y, n, R₁ und R₂ wie vorstehend für Formel (Ih) beschrieben sind, mit Trifluormethansulfonanhydrid in einem Lösungsmittel und dann mit Ammoniak oder Mono- und Dialkylaminen zum Erhalten von Verbindungen der Formel (Ih), worin Z respektive Amino oder Monoalkylamino oder Dialkylamino ist, X, Y, n, R₁ und R₂ wie vorstehend für Formel (Ih) beschrieben sind, und gegebenenfalls anschließend Bilden von Salzen und/oder Hydraten und/oder Solvaten der erhaltenen Methoxyderivate der Formel (Ih), worin Z respektive Amino oder Monoalkylamino oder Dialkylamino ist; oder
(v) Umsetzen der Chlor- oder Bromderivate der Formel (Ih), worin Z Chlor oder Brom ist; X, Y, n, R₁ und R₂ wie vorstehend für Formel (Ih) beschrieben sind, mit Natriumazid in DMF und Wasser zum Erhalten von Verbindungen der Formel (Ih), worin Z Azido ist, X, Y, n, R₁ und R₂ wie vorstehend für Formel (Ih) beschrieben sind, dann Umsetzen der erhaltenen Azidoderivate mit Natriumborhydrid in einem Lösungsmittel zum Erhalten von Verbindungen der Formel (Ih), worin Z Amino ist, X, Y, n, R₁ und R₂ wie vorstehend für Formel (Ih) beschrieben sind, und gegebenenfalls anschließend Bilden von Salzen und/oder Hydraten und/oder Solvaten der erhaltenen Aminoderivate der Formel (Ih).

12. Pharmazeutische Formulierung, umfassend eine therapeutisch wirksame Menge einer Verbindung der Formel (I): 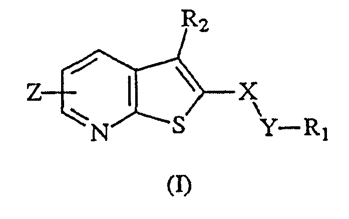 worin:
X eine Gruppe darstellt, die ausgewählt ist aus SO oder SO₂;
Y eine Gruppe darstellt, die ausgewählt ist aus (CH₂)ₙ, NH oder NHCH₂;
n eine ganze Zahl von 0 bis 1 ist;
Z H ist oder mit Alkyl, Nitro, Halogen, Alkoxy, Trifluormethyl, Cyano, Amino, Alkylamino, Dialkylamino, Aminomethyl, Alkylaminomethyl, Dialkylaminomethyl, Hydroxy oder Alkylsulfonylamino monosubstituiert ist;
R₁ ein gegebenenfalls substituiertes Alkyl, Cycloalkyl, Phenyl, Biphenyl oder Heterocyclyl ist;
R₂ ein gegebenenfalls substituiertes Phenyl oder Heterocyclyl oder eine NR₃R₄-Gruppe, worin R₃ und R₄ unabhängig ausgewählt sind aus der Gruppe aus Wasserstoff und einem gegebenenfalls substituierten Alkyl, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e), oder eine NH-CO-NR₅R₆-Gruppe ist, worin R₅ und R₆ unabhängig ausgewählt sind aus der Gruppe aus Wasserstoff und einem gegebenenfalls substituierten Alkyl, oder R₅ und R₆ zusammen mit dem N-Atom, an das sie gebunden sind, eine gegebenenfalls substituierte C₅₋₇-Heterocyclylgruppe bilden, enthaltend ein oder mehrere Heteroatom(e);
und/oder Hydrate und/oder Solvate und/oder pharmazeutisch annehmbare Salze davon, gebildet mit Säuren oder Basen, zusammen mit einem oder mehreren physiologisch annehmbaren Verdünnungsstoffen, Exzipienten und/oder inerten Trägern.

13. Pharmazeutische Zusammensetzung gemäß Anspruch 12 zur Verwendung in der Vorbeugung und/oder Behandlung von mGluR1- und mGluR5-Rezeptor-vermittelten Störungen.

14. Verwendung einer Verbindung der Formel (I): 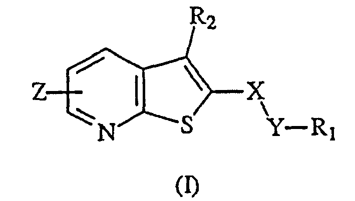 worin:
X eine Gruppe darstellt, die ausgewählt ist aus SO oder SO₂;
Y eine Gruppe darstellt, die ausgewählt ist aus (CH₂)ₙ, NH oder NHCH₂;
n eine ganze Zahl von 0 bis 1 ist;
Z H ist oder mit Alkyl, Nitro, Halogen, Alkoxy, Trifluormethyl, Cyano, Amino, Alkylamino, Dialkylamino, Aminomethyl, Alkylaminomethyl, Dialkylaminomethyl, Hydroxy oder Alkylsulfonylamino monosubstituiert ist;
R₁ ein gegebenenfalls substituiertes Alkyl, Cycloalkyl, Phenyl, Biphenyl oder Heterocyclyl ist;
R₂ ein gegebenenfalls substituiertes Phenyl oder Heterocyclyl oder eine NR₃R₄-Gruppe, worin R₃ und R₄ unabhängig ausgewählt sind aus der Gruppe aus Wasserstoff und einem gegebenenfalls substituierten Alkyl, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e), oder eine NH-CO-NR₅R₆-Gruppe ist, worin R₅ und R₆ unabhängig ausgewählt sind aus der Gruppe aus Wasserstoff und einem gegebenenfalls substituierten Alkyl, oder R₅ und R₆ zusammen mit dem N-Atom, an das sie gebunden sind, eine gegebenenfalls substituierte C₅₋₇-Heterocyclylgruppe bilden, enthaltend ein oder mehrere Heteroatom(e); und/oder Hydraten und/oder Solvaten und/oder pharmazeutisch annehmbaren Salzen davon, gebildet mit Säuren oder Basen, zur Herstellung eines Medikaments zur Behandlung und/oder Vorbeugung von mGluR1- und mGluR5-Rezeptor-vermittelten Störungen.

15. Verwendung einer Verbindung gemäß Anspruch 14, worin die mGluR1- und mGluR5-Rezeptor-vermittelten Störungen psychiatrische Störungen sind.

16. Verwendung einer Verbindung gemäß Anspruch 14, worin die mGluR1- und mGluR5-Rezeptor-vermittelten Störungen neurologische Störungen sind.

17. Verwendung einer Verbindung gemäß Anspruch 14, worin die mGluR1- und mGluR5-Rezeptor-vermittelten Störungen chronischer und akuter Schmerz sind.

18. Verwendung einer Verbindung gemäß Anspruch 14, worin die mGluR1- und mGluR5-Rezeptor-vermittelten Störungen neuromuskuläre Dysfunktion des unteren Harnwegs und gastrointestinale Störungen sind.

19. Verbindung der Formel (I): 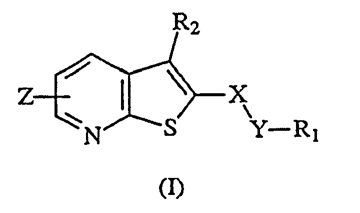 worin:
X eine Gruppe darstellt, die ausgewählt ist aus SO oder SO₂;
Y eine Gruppe darstellt, die ausgewählt ist aus (CH₂)ₙ, NH oder NHCH₂;
n eine ganze Zahl von 0 bis 1 ist;
Z H ist oder mit Alkyl, Nitro, Halogen, Alkoxy, Trifluormethyl, Cyano, Amino, Alkylamino, Dialkylamino, Aminomethyl, Alkylaminomethyl, Dialkylaminomethyl, Hydroxy oder Alkylsulfonylamino monosubstituiert ist;
R₁ ein gegebenenfalls substituiertes Alkyl, Cycloalkyl, Phenyl, Biphenyl oder Heterocyclyl ist;
R₂ ein gegebenenfalls substituiertes Phenyl oder Heterocyclyl oder eine NR₃R₄-Gruppe, worin R₃ und R₄ unabhängig ausgewählt sind aus der Gruppe aus Wasserstoff und einem gegebenenfalls substituierten Alkyl, oder R₃ und R₄ zusammen mit dem N-Atom, an das sie gebunden sind, eine gegebenenfalls substituierte C₅₋₇-Heterocyclylgruppe bilden, enthaltend ein oder mehrere Heteroatom(e), oder eine NH-CO-NR₅R₆-Gruppe ist, worin R₅ und R₆ unabhängig ausgewählt sind aus der Gruppe aus Wasserstoff und einem gegebenenfalls substituierten Alkyl, oder R₅ und R₆ zusammen mit dem N-Atom, an das sie gebunden sind, eine gegebenenfalls substituierte C₅₋₇-Heterocyclylgruppe bilden, enthaltend ein oder mehrere Heteroatom(e);
und/oder Hydrate und/oder Solvate und/oder pharmazeutisch annehmbare Salze davon, gebildet mit Säuren oder Basen, zur Verwendung in einem Verfahren zur Vorbeugung und/oder Behandlung von mGluR1- und mGluR5-Rezeptor-vermittelten Störungen.

20. Verbindung gemäß Anspruch 19, worin die mGluR1- und mGluR5-Rezeptor-vermittelten Störungen psychiatrische Störungen sind.

21. Verbindung gemäß Anspruch 19, worin die mGluR1- und mGluR5-Rezeptor-vermittelten Störungen neurologische Störungen sind.

22. Verbindung gemäß Anspruch 19, worin die mGluR1- und mGluR5-Rezeptor-vermittelten Störungen chronische und akute Schmerzstörungen sind.

23. Verbindung gemäß Anspruch 19, worin die mGluR1- und mGluR5-Rezeptor-vermittelten Störungen neuromuskuläre Dysfunktion des unteren Harnwegs und gastrointestinale Störungen sind.

## Revendications

1. Composé de formule (I) : 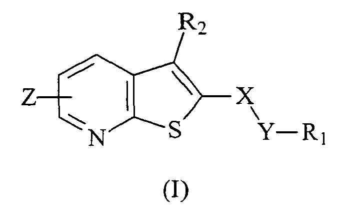 dans laquelle
X représente un groupe choisi parmi SO, SO₂ ;
Y représente un groupe choisi parmi (CH₂)n, NH, NHCH₂ ;
n est un entier de 0 à 1 ;
Z est H ou monosubstitué par un groupe alkyle, nitro, un atome d'halogène, un groupe alcoxy, trifluorométhyle, cyano, amino, alkylamino, dialkylamino, aminométhyle, alkylaminométhyle, dialkylaminométhyle, hydroxyle, alkylsulfonylamino ;
R₁ est un groupe alkyle, cycloalkyle, phényle, biphényle, hétérocyclyle facultativement substitué ;
R₂ est un groupe phényle, hétérocyclyle facultativement substitué, ou
un groupe NR₃R₄ où R₃ et R₄ sont indépendamment choisis dans le groupe consistant en un atome d'hydrogène et un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes, ou
un groupe NH-CO-NR₅R₆, où R₅ et R₆ sont indépendamment choisis dans le groupe consistant en un atome d'hydrogène et un groupe alkyle facultativement substitué, ou bien R₅ et R₆ conjointement avec l'atome N auquel ils sont attachés forment un groupe hétérocyclyle en C_{5 à 7} facultativement substitué, contenant un ou plusieurs hétéroatomes ; et/ou des hydrates et/ou solvates et/ou sels pharmaceutiquement acceptables de celui-ci formés avec des acides ou des bases.

2. Composé selon la revendication 1 de formule (I) : 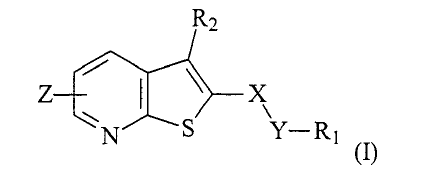 dans laquelle
X représente un groupe choisi parmi SO, SO₂ ;
Y représente un groupe choisi parmi (CH₂)n, NH, NHCH₂ ;
n est un entier de 0 à 1 ;
Z est H ou monosubstitué par un groupe alkyle, nitro, un atome d'halogène, un groupe alcoxy, trifluorométhyle, cyano, amino, alkylamino, dialkylamino, aminométhyle, alkylaminométhyle, dialkylaminométhyle, hydroxyle, alkylsulfonylamino ;
R₁ est un groupe alkyle ou cycloalkyle en C_{3 à 10} facultativement substitué par un ou plusieurs substituants choisis parmi les groupes alcoxy, trifluorométhyle, amino, alkylamino, dialkylamino, aminométhyle, alkylaminométhyle, dialkylaminométhyle, acylamino, cyano, un atome d'halogène, ou
un groupe phényle ou biphényle facultativement substitué par un ou plusieurs substituants choisi parmi les groupes alkyle, méthoxy, trifluorométhyle, amino, alkylamino, dialkylamino, aminométhyle, alkylaminométhyle, dialkylaminométhyle, acylamino, alkylsulfonyle, alkylsulfonylamino, cyano, un atome d'halogène, ou
un groupe hétérocyclyle monocyclique ou bicyclique saturé ou insaturé, contenant 1 à 4 hétéroatomes choisis parmi O, N ou S, tels que les cycles pyridyle, quinoléinyle, thiazolyle, pipéridinyle, morpholyle, tétrahydroquinoléinyle, oxazolyle, isoxazolyle, furyle, thiophène, triazolyle, pyrrolidinyle facultativement substitués par un ou plusieurs groupes hydroxy, alkylhydroxy, alkyle, alcoxy, trifluorométhyle, amino, alkylamino, dialkylamino, aminométhyle, alkylaminométhyle, dialkylaminométhyle, acylamino, cyano, un atome d'halogène ou un groupe oxo ;
R₂ est un groupe phényle facultativement substitué par un ou plusieurs substituants choisis parmi les groupes alkyle, méthoxy, trifluorométhyle, amino, alkylamino, dialkylamino, aminométhyle, alkylaminométhyle, dialkylaminométhyle, acylamino, alkylsulfonyle, alkylsulfonylamino, cyano, un atome d'halogène, ou
un groupe hétérocyclyle en C_{5 à 7} monocyclique ou bicyclique saturé ou insaturé, contenant 1 à 4 hétéroatomes choisis parmi O, N ou S, tels que les cycles pyridyle, quinoléinyle, thiazolyle, pipéridinyle, morpholyle, tétrahydroquinoléinyle, oxazolyle, isoxazolyle, furyle thiophène, triazolyle, pyrrolidinyle facultativement substitués par un ou plusieurs groupes hydroxy, alkylhydroxy, alkyle, alcoxy, trifluorométhyle, amino, alkylamino, dialkylamino, aminométhyle, alkylaminométhyle, dialkylaminométhyle, acylamino, cyano, atome d'halogène ou groupe oxo, ou
un groupe NR₃R₄ où R₃ et R₄ sont indépendamment choisis dans le groupe consistant en un atome d'hydrogène et un groupe alkyle facultativement substitué par un ou plusieurs substituants choisis parmi les groupes alcoxy, trifluorométhyle, amino, alkylamino, dialkylamino, aminométhyle, alkylaminométhyle, dialkylaminométhyle, acylamino, cyano, un atome d'halogène, ou R₃ et R₄ conjointement avec l'atome N auquel ils sont attachés forment un groupe hétérocyclyle en C_{5 à 7}, contenant un ou plusieurs hétéroatomes, choisis parmi O, N ou S, facultativement substitués par un ou plusieurs groupes hydroxy, alkylhydroxy, alkyle, alcoxy, trifluorométhyle, amino, alkylamino, dialkylamino, aminométhyle, alkylaminométhyle, dialkylaminométhyle, acylamino, cyano, atome d'halogène ou groupe oxo, ou
un groupe NH-CO-NR₅R₆, où R₅ et R₆ sont indépendamment choisis dans le groupe consistant en un atome d'hydrogène et un groupe alkyle facultativement substitué par un ou plusieurs substituants choisis parmi les groupes alcoxy, trifluorométhyle, amino, alkylamino, dialkylamino, aminométhyle, alkylaminométhyle, dialkylaminométhyle, acylamino, cyano, un atome d'halogène, ou bien R₅ et R₆ conjointement avec l'atome N auquel ils sont attachés forment un groupe hétérocyclyle en C_{5 à 7}, contenant un ou plusieurs hétéroatomes, choisis parmi O, N ou S, facultativement substitués par un ou plusieurs groupes hydroxy, alkylhydroxy, alkyle, alcoxy, trifluorométhyle, amino, alkylamino, dialkylamino, aminométhyle, alkylaminométhyle, dialkylaminométhyle, acylamino, cyano, atome d'halogène ou groupe oxo et/ou les hydrates et/ou solvates et/ou sels pharmaceutiquement acceptables de celui-ci formé avec des acides ou des bases.

3. Composé selon la revendication 1 choisi parmi
la 2-(4-chloro-benzènesulfonyl)-3-(4-chloro-phényl)-thiéno[2,3-*b*]pyridine,
la 2-(4-chloro-benzènesulfinyl)-3-(4-chloro-phényl)-thiéno[2,3-*b*]pyridine,
la 3-(4-chloro-phényl)-2-(4-fluoro-benzènesulfonyl)-thiéno[2,3-*b*]pyridine,
la 3-(4-chloro-phényl)-2-(toluène-4-sulfonyl)-thiéno[2,3-*b*]pyridine,
le 4-[3-(4-chloro-phényl)-thiéno[2,3-*b*]pyridine-2-sulfonyl]-benzonitrile,
la 2-benzènesulfonyl-3-(4-chloro-phényl)-thiéno[2,3-*b*]pyridine,
le 3-[3-(4-chloro-phényl)-thiéno[2,3-*b*]pyridine-2-sulfonyl]-benzonitrile,
la 3-(4-chloro-phényl)-2-(pyridine-3-sulfonyl)-thiéno[2,3-*b*]pyridine,
la 2-(butane-1-sulfonyl)-3-(4-chloro-phényl)-thiéno[2,3-*b*]pyridine,
la 3-(4-chloro-phényl)-2-(2,4-diméthyl-thiazole-5-sulfonyl)-thiéno[2,3-*b*]pyridine,
la 3-(4-chloro-phényl)-2-(thiophène-2-sulfonyl)-thiéno[2,3-*b*]pyridine,
le (4-chloro-phényl)-amide d'acide 3-(4-chloro-phényl)-thiéno[2,3-*b*]pyridine-2-sulfonique,
la 3-(4-chloro-phényl)-2-phénylméthanesulfonyl-thiéno[2,3-*b*]pyridine,
la 2-(3-chloro-benzènesulfonyl)-3-(4-chloro-phényl)-thiéno[2,3-*b*]pyridine,
la 2-(4-chloro-benzènesulfonyl)-3-(4-chloro-phényl)-6-chloro-thiéno[2,3-*b*]pyridine,
la 2-(3-fluoro-benzènesulfonyl)-3-(4-chloro-phényl)-thiéno[2,3-*b*]pyridine,
la 2-(3-cyano-benzènesulfonyl)-3-(3-fluoro-phényl)-thiéno[2,3-*b*]pyridine,
la 2-(4-chloro-benzènesulfonyl)-3-(4-chloro-phényl)-6-fluoro-thiéno[2,3-*b*]pyridine,
la 2-(4-cyano-benzènesulfonyl)-3-(3-chloro-phényl)-thiéno[2,3-*b*]pyridine,
la 2-(3-cyano-benzènesulfonyl)-3-(3-méthyl-pipéridinyl)-thiéno[2,3-*b*]pyridine,
la 2-(3-cyano-benzènesulfonyl)-3-(4-méthyl-pipéridinyl)-thiéno[2,3-*b*]pyridine,
la 2-(3-cyano-benzènesulfonyl)-3-(4-tolyl)-thiéno[2,3-*b*]pyridine,
la 2-(3-cyano-benzènesulfonyl)-3-(3-méthoxy-phényl)-thiéno[2,3-*b*]pyridine,
la 2-(3-méthoxy-benzènesulfonyl)-3-(4-chloro-phényl)-thiéno[2,3-*b*]pyridine,
la 2-(3-cyano-5-fluoro-benzènesulfonyl)-3-(4-chloro-phényl)-thiéno[2,3-*b*]pyridine,
la 2-(3-fluoro-4-méthyl-benzènesulfonyl)-3-(4-chloro-phényl)-thiéno[2,3-*b*]pyridine,
la 2-(3,4-diméthyl-benzènesulfonyl)-3-(4-fluoro-phényl)-thiéno[2,3-*b*]pyridine,
la 2-(3-cyano-benzènesulfonyl)-3-(4-fluoro-phényl)-6-chloro-thiéno[2,3-*b*]pyridine,
la 2-(3-cyano-5-fluoro-benzènesulfonyl)-3'-(4-fluoro-phényl)-thiéno[2,3-*b*]pyridine,
la 2-(3-cyano-benzènesulfonyl)-3-(4-fluoro-phényl)-6-fluoro-thiéno[2,3-*b*]pyridine,
la 2-(4-bromo-benzènesulfonyl)-3-(4-chloro-phényl)-thiéno[2,3-*b*]pyridine,
la 5-amino-2-(4-chloro-benzènesulfonyl)-3-(4-chloro-phényl)-thiéno[2,3*-b*]pyridine,
la 2-(3,4-dichloro-benzènesulfonyl)-3-(4-chloro-phényl)-thiéno[2,3-*b*]pyridine,
la 2-(3-fluoro-4-méthyl-benzènesulfonyl)-3-(4-fluoro-phényl)-thiéno[2,3-*b*]pyridine,
la 2-(3-cyano-benzènesulfonyl)-3-(2-chloro-phényl)-thiéno[2,3-*b*]pyridine,
la 2-(3-cyano-5-fluoro-benzènesulfonyl)-3-(4-chloro-phényl)-6-chloro-thiéno[2,3-*b*]pyridine,
la 2-(3-fluoro-4-méthoxy-benzènesulfonyl)-3-(4-chloro-phényl)-thiéno[2,3-*b*]pyridine,
la 2-(4-chloro-benzènesulfonyl)-3-(4-méthyl-pipéridinyl)-thiéno[2,3-*b*]pyridine,
la 2-(3-cyano-5-fluoro-benzènesulfonyl)-3-(4-méthyl-pipéridinyl)-thiéno[2,3-*b*]pyridine,
la 2-(4-chloro-benzènesulfonyl)-3-(4-chloro-phényl)-5-fluoro-thiéno[2,3-*b*]pyridine,
la 2-(4-chloro-benzènesulfonyl)-3-(4-chloro-phényl)-5-chloro-thiéno[2,3-*b*]pyridine,
la 2-(4-chloro-benzènesulfonyl)-3-(4-chloro-phényl)-4-chloro-thiéno[2,3-*b*]pyridine,
la 2-(4-chloro-benzènesulfonyl)-3-(4-chloro-phényl)-4-fluoro-thiéno[2,3-*b*]pyridine,
la 5-amino-2-(4-chloro-benzènesulfonyl)-3-(4-méthyl-pipéridinyl)-thiéno[2,3-*b*]pyridine,
la 2-(3-cyano-5-fluoro-benzènesulfonyl)-3-(3-fluoro-phényl)-thiéno[2,3-*b*]pyridine,
la 5-amino-2-(4-chloro-benzènesulfonyl)-3-(3-fluoro-phényl)-thiéno[2,3-*b*]pyridin,
la 2-(4-chloro-benzènesulfonyl)-3-(4-méthyl-pipéridinyl)-6-chloro-thiéno[2,3-*b*]pyridine,
la 2-(3-fluoro-4-méthoxy-benzènesulfonyl)-3-(4-fluoro-phényl)-thiéno[2,3-*b*]pyridine,
la 2-(3-fluoro-4-méthoxy-benzènesulfonyl)-3-(3-fluoro-phényl)-thiéno[2,3-*b*]pyridine,
la 2-(3-cyano-5-fluoro-benzènesulfonyl)-3-(2-fluoro-phényl)-thiéno[2,3-*b*]pyridine,
la 2-(3-cyano-5-fluoro-benzènesulfonyl)-3-(3-chloro-phényl)-thiéno[2,3-*b*]pyridine.

4. Procédé de préparation d'un composé de formule (Ia) 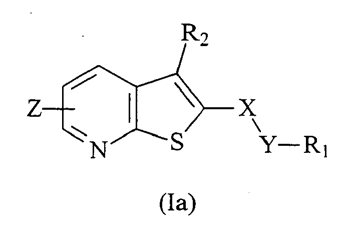 dans laquelle
X représente un groupe choisi parmi SO, SO₂ ;
Y représente un groupe choisi parmi (CH₂)ₙ, NH, NHCH₂ ;
n est un entier de 0 à 1 ;
Z est H ou monosubstitué par un groupe alkyle ou nitro,
R₁ est un groupe alkyle, cycloalkyle, phényle, biphényle, hétérocyclyle facultativement substitué ;
R₂ est un groupe phényle, facultativement substitué par un groupe alkyle ou un atome d'halogène ;
en faisant réagir un composé de formule (III) : 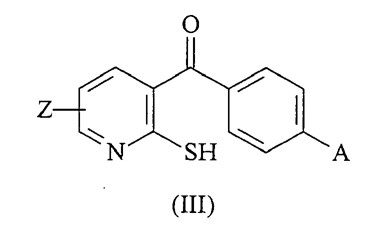 dans laquelle A est un substituant comme R₂ défini dans la formule (Ia) et Z est H ou monosubstitué par un groupe alkyle, ou monosubstitué par un groupe nitro avec un composé de formule (VII) :
HlgCH₂SOYR₁ (VII)
dans laquelle Hlg est un groupe chloro ou bromo, Y et R₁ sont comme décrits ci-dessus pour la formule (Ia), ou
avec un composé de formule (VIII) :
HlgCH₂SO₂YR₁ (VIII)
dans laquelle Hlg est un groupe chloro ou bromo, Y et R₁ sont comme décrits ci-dessus pour la formule (Ia) en présence de méthoxyde de sodium dans du diméthylformamide en tant que solvant pour obtenir un composé de formule (Ia), et facultativement en formant ensuite des sels et/ou hydrates et/ou solvates de composés de formule (Ia).

5. Procédé de préparation d'un composé de formule (Ib) 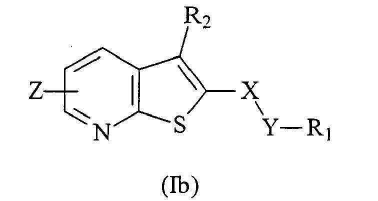 dans laquelle
X représente SO, SO₂ ;
Y représente un groupe choisi parmi (CH₂)ₙ, NH, NHCH₂ ;
n est un entier de 0 à 1 ;
Z est H ou un groupe alkyle ;
R₁ est un groupe alkyle, cycloalkyle, phényle, biphényle, hétérocyclyle facultativement substitué ;
R₂ est un groupe phényle, ou hétérocyclyle aromatique, facultativement substitué par un ou plusieurs substituants choisis parmi les groupes alkyle, alcoxy, trifluorométhyle, amino, alkylamino, dialkylamino, aminométhyle, alkylaminométhyle, dialkylaminométhyle, acylamino, cyano, fluoro, chloro, bromo, hydroxyle, méthylsulfonyle,
en faisant réagir un composé de formule (XI) : 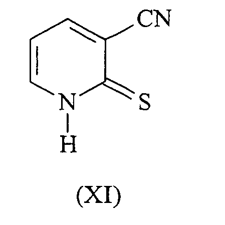 avec un composé de formule (VII) :
HlgCH₂SOYR₁ (VII)
dans laquelle Hlg est un groupe chloro ou bromo, Y et R₁ sont comme définis ci-dessus pour la formule (Ib), ou
avec un composé de formule (VIII) :
HlgCH₂SO₂YR₁ (VIII)
dans laquelle Hlg est un groupe chloro ou bromo, Y et R₁ sont comme décrits ci-dessus pour la formule (Ib) en présence de méthoxyde de sodium dans du diméthylformamide en tant que solvant et
en faisant réagir le composé obtenu de formule (IV) : 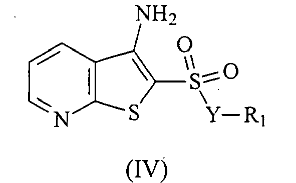 dans laquelle R₁ est comme décrit ci-dessus pour la formule (Ib) avec du bromure de cuivre (II) et du nitrite de tert-butyle dans de l'acétonitrile ; et en faisant réagir le composé obtenu de formule (V) : 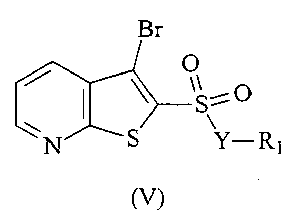 dans laquelle R₁ est comme décrit ci-dessus pour la formule (Ib) avec un composé de formule (IX) :
R₂-B(OH)₂ (IX)
dans laquelle R₂ est comme défini ci-dessus pour la formule (Ib), en présence d'une base et d'un catalyseur dans un solvant pour obtenir un composé de formule (Ib), et facultativement en formant ensuite des sels et/ou hydrates et/ou solvates de composés de formule (Ib).

6. Procédé de préparation d'un composé de formule (Ic) : 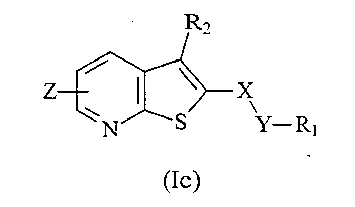 dans laquelle
X représente SO₂ ;
Y représente un groupe choisi parmi (CH₂)ₙ, NH, NHCH ;
n est un entier de 0 à 1 ;
Z est H ou un groupe alkyle ;
R₁ est un groupe alkyle, cycloalkyle, phényle, biphényle, hétérocyclyle facultativement substitué ;
R₂ est NR₃R₄ où R₃ et R₄ sont indépendamment choisis dans le groupe consistant en un atome d'hydrogène et un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes,
en faisant réagir un composé de formule (V) : 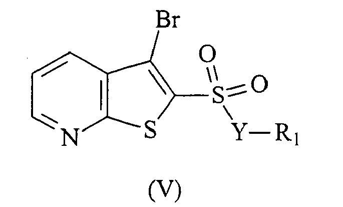 dans laquelle R₁ est comme décrit ci-dessus pour la formule (Ic),
avec un composé de formule (XVI) :
HNR₃R₄ (XVI)
dans laquelle R₃ et R₄ sont comme décrits ci-dessus pour la formule (Ic) pour obtenir un composé de formule (Ic), et facultativement en formant ensuite des sels et/ou hydrates et/ou solvates de composés de formule (Ic).

7. Procédé de préparation d'un composé de formule (Id) : 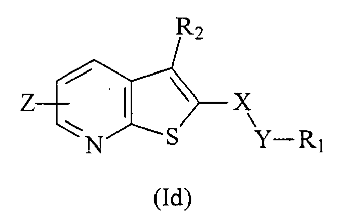 dans laquelle
X représente SO₂ ;
Y représente un groupe choisi parmi (CH₂)ₙ, NH, NHCH₂ ;
n est un entier de 0 à 1 ;
Z est H ou un groupe alkyle ;
R₁ est un groupe alkyle, cycloalkyle, phényle, biphényle, hétérocyclyle facultativement substitué ;
R₂ est un groupe NH-CO-NR₃R₄, où R₃ et R₄ sont indépendamment choisis dans le groupe consistant en un atome d'hydrogène ou un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes,
en faisant réagir un composé de formule (IV) : 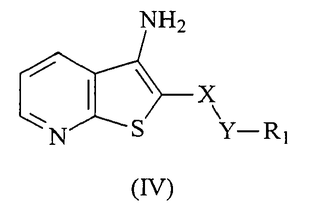 dans laquelle R₁ est comme décrit ci-dessus pour la formule (Id), avec un composé de formule (XIII) :
HNR₃R₄ (XIII)
dans laquelle R₃ et R₄ sont comme décrits ci-dessus pour la formule (Id), en présence de phosgène ou de triphosgène et d'une base pour obtenir un composé de formule (Id), et facultativement en formant ensuite des sels et/ou hydrates et/ou solvates de composés de formule (Id).

8. Procédé de préparation d'un composé de formule (Ie) : 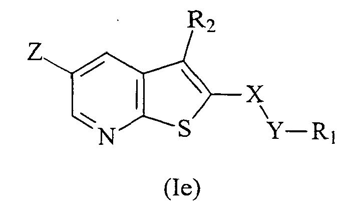 dans laquelle
X représente un groupe choisi parmi SO, SO₂ ;
Y représente un groupe choisi parmi (CH₂)ₙ, NH, NHCH₂ ;
n est un entier de 0 à 1 ;
Z est un groupe amino, alkylsulfonylamino, un atome d'halogène ;
R₁ est un groupe alkyle, cycloalkyle, phényle, biphényle, hétérocyclyle facultativement substitué ;
R₂ est un groupe phényle, facultativement substitué par un groupe alkyle ou un atome d'halogène ;
en faisant réagir un composé de formule (Ia) : 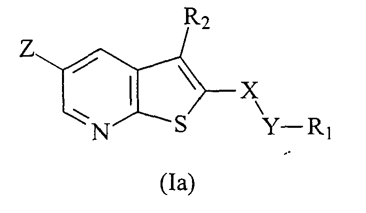 dans laquelle
X représente un groupe choisi parmi SO, SO₂ ;
Y représente un groupe choisi parmi (CH₂)ₙ, NH, NHCH₂;
n est un entier de 0 à 1 ;
Z est un groupe nitro ;
R₁ est un groupe alkyle, cycloalkyle, phényle, biphényle, hétérocyclyle facultativement substitué ;
R₂ est un groupe phényle, facultativement substitué par un groupe alkyle ou un atome d'halogène,
avec un agent réducteur approprié pour obtenir un composé de formule (Ie) 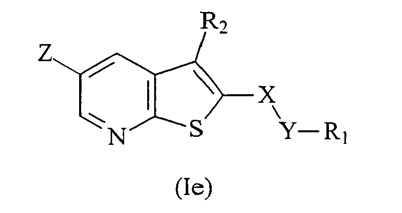 dans laquelle Z est un groupe amino ; X, Y, n, R₁ et R₂ sont comme décrits ci-dessus pour la formule (Ie) et facultativement en formant ensuite des sels et/ou hydrates et/ou solvates des dérivés amino obtenus, ou
a. en faisant réagir facultativement les dérivés amino obtenus de formule (Ie), où Z est un groupe amino ; X, Y, n, R₁ et R₂ sont comme décrits ci-dessus pour la formule (Ie) avec du nitrite de sodium en présence d'acide chlorhydrique et d'iodure de cuivre (I) ou de chlorure de cuivre (I) ou de bromure de cuivre (I) ou de tétrafluoroborate de sodium pour obtenir un composé de formule (Ie), où Z est un atome d'halogène ; X, Y, n, R₁ et R₂ sont comme décrits ci-dessus pour la formule (Ie), et facultativement en formant ensuite des sels et/ou hydrates et/ou solvates des dérivés halogénés obtenus de formule Ie ou
b. en faisant réagir facultativement les dérivés amino obtenus de formule (Ie), où Z est un groupe amino ; X, Y, n, R₁ et R₂ sont comme décrits ci-dessus pour la formule (Ie) avec des dérivés chlorure d'alkylsulfonyle pour obtenir des composés de formule (Ie), où Z est un groupe alkylsulfonylamino ; X, Y, n, R₁ et R₂ sont comme décrits ci-dessus pour la formule (Ie), et facultativement en formant ensuite des sels et/ou hydrates et/ou solvates des dérivés alkylsulfonylamino obtenus de formule (Ie).

9. Procédé de préparation d'un composé de formule (If) : 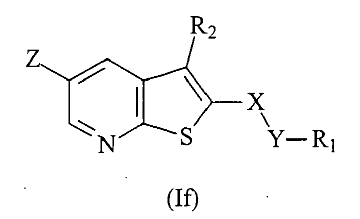 dans laquelle
X représente SO₂ ;
Y représente un groupe choisi parmi (CH₂)ₙ, NH, NHCH₂ ;
n est un entier de 0 à 1 ;
Z est un groupe amino, alkylsulfonylamino, un atome d'halogène ;
R₁ est un groupe alkyle, cycloalkyle, phényle, biphényle, hétérocyclyle facultativement substitué ;
R₂ est un groupe phényle, hétérocyclyle facultativement substitué, ou un groupe NR₃R₄ où R₃ et R₄ sont indépendamment choisis dans le groupe consistant en un atome d'hydrogène ou un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes,
en faisant réagir un composé de formule (V) : 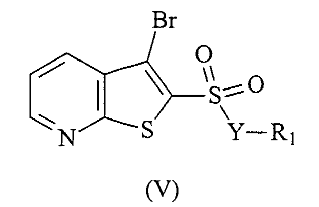 dans laquelle R₁ et Y sont comme décrits ci-dessus pour la formule (If), avec un agent oxydant approprié et en faisant réagir le dérivé N-oxyde obtenu de formule (X) : 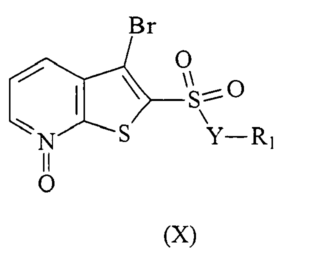 dans laquelle R₁ et Y sont comme décrits ci-dessus pour la formule (If), avec de l'acide nitrique aqueux dans de l'acide acétique, en faisant réagir ensuite le dérivé nitro obtenu de formule (XI) : 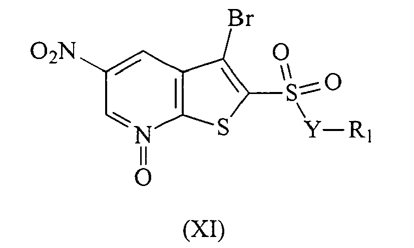 dans laquelle R₁ et Y sont comme décrits ci-dessus pour la formule (If), avec un agent réducteur approprié, et en faisant réagir le dérivé amino obtenu de formule (XII) : 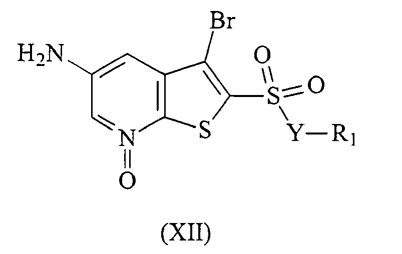 dans laquelle R₁ et Y sont comme décrits ci-dessus pour la formule (If),
a. avec un composé de formule (IX) :
R₂-B(OH)₂ (IX)
dans laquelle R₂ est un groupe phényle, hétérocyclyle facultativement substitué, en présence d'une base et d'un catalyseur dans un solvant pour obtenir un composé de formule (If), où Z est un groupe amino ; R₂ est un groupe phényle, hétérocyclyle facultativement substitué, Y et R₁ sont comme décrits ci-dessus pour la formule (If) et facultativement en formant ensuite des sels et/ou hydrates et/ou solvates des dérivés amino obtenus de formule (If), et
(i) en faisant réagir facultativement les dérivés amino obtenus de formule (If), où Z est un groupe amino ; R₂ est un groupe phényle, hétérocyclyle facultativement substitué, Y et R₁ sont comme décrits ci-dessus pour la formule (If), avec du nitrite de sodium en présence d'acide chlorhydrique et d'iodure de cuivre (I) ou de chlorure de cuivre (I) ou de bromure de cuivre (I) ou de tétrafluoroborate de sodium pour obtenir un composé de formule (If), où Z est un atome d'halogène ; R₂ est un groupe phényle, hétérocyclyle facultativement substitué, Y et R₁ sont comme décrits ci-dessus pour la formule (If), et facultativement en formant ensuite des sels et/ou hydrates et/ou solvates des dérivés halogénés obtenus de formule (If), ou
(ii) en faisant réagir facultativement le dérivé amino obtenu de formule (If), où Z est un groupe amino ; R₂ est un groupe phényle, hétérocyclyle facultativement substitué, Y et R₁ sont comme décrits ci-dessus pour la formule (If), avec des dérivés chlorure d'alkylsulfonyle pour obtenir un composé de formule (If), et facultativement en formant ensuite des sels et/ou hydrates et/ou solvates des dérivés alkylsulfonylamino obtenus de formule (If), ou
b. avec un composé de formule (XIII) :
NHR₃R₄ (XIII)
dans laquelle R₃ et R₄ sont comme décrits ci-dessus pour la formule (If), à 70 à 200 °C pour obtenir un composé de formule (If), où Z est un groupe amino ; Y, R₁ et R₂ qui est un groupe NR₃R₄, sont comme décrits ci-dessus pour la formule (If) et facultativement en formant ensuite des sels et/ou hydrates et/ou solvates des dérivés amino obtenus de formule (If), ou
(i) en faisant réagir facultativement les dérivés amino obtenus de formule (If), où Z est un groupe amino ; Y, R₁ et R₂ qui est un groupe NR₃R₄, sont comme décrits ci-dessus pour la formule (If) avec du nitrite de sodium en présence d'acide chlorhydrique et d'iodure de cuivre (I) ou de chlorure de cuivre (I) ou de bromure de cuivre (I) ou de tétrafluoroborate de sodium pour obtenir un composé de formule (If), où Z est un atome d'halogène, Y, R₁ et R₂ qui est un groupe NR₃R₄, sont comme décrits ci-dessus pour la formule (If) et facultativement en formant ensuite des sels et/ou hydrates et/ou solvates des dérivés halogénés obtenus de formule (If), ou
(ii) en faisant réagir facultativement les dérivés amino obtenus où Z est un groupe amino ; Y, R₁ et R₂ qui est un groupe NR₃R₄, sont comme décrits ci-dessus pour la formule (If) avec des dérivés chlorure d'alkylsulfonyle pour obtenir un composé de formule (If), où Z est un groupe alkylsulfonylamino; Y, R₁ et R₂ qui est un groupe NR₃R₄, sont comme décrits ci-dessus pour la formule (If), et facultativement en formant ensuite des sels et/ou hydrates et/ou solvates des dérivés alkylsulfonylamino obtenus de formule (If).

10. Procédé de préparation d'un composé de formule (Ig) : 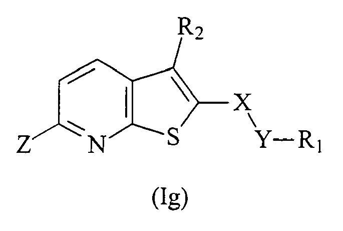 dans laquelle
X représente SO₂ ;
Y représente un groupe choisi parmi (CH₂)ₙ, NH, NHCH₂ ;
n est un entier de 0 à 1 ;
Z est un groupe amino, bromo, chloro, iodo, méthoxy, mono- ou dialkyamino ;
R₁ est un groupe alkyle, cycloalkyle, phényle, biphényle, hétérocyclyle facultativement substitué ;
R₂ est un groupe phényle, hétérocyclyle facultativement substitué, ou
un groupe NR₃R₄ où R₃ et R₄ sont indépendamment choisis dans le groupe consistant en un atome d'hydrogène ou un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes,
en faisant réagir un composé de formule (X) : 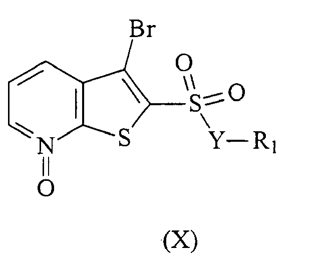 dans laquelle R₁ et Y sont comme décrits ci-dessus pour la formule (Ig), avec un composé de formule (IX) :
R₂-B(OH)₂ (IX)
dans laquelle R₂ est un groupe phényle, hétérocyclyle facultativement substitué, en présence d'une base et d'un catalyseur dans un solvant, pour obtenir des composés de formule (XIV) : 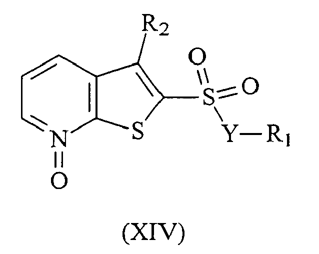 dans laquelle R₂ est un groupe phényle, hétérocyclyle facultativement substitué, R₁ et Y sont comme décrits ci-dessus pour les composés de formule (Ig), ou
avec un composé de formule (XIII) :
NHR₃R₄ (XIII)
dans laquelle R₃ et R₄ sont comme décrits ci-dessus pour la formule (Ig) pour obtenir des composés de formule (XIV), où R₂ est un groupe NR₃R₄, où R₃ et R₄ sont indépendamment choisis ans le groupe consistant en un atome d'hydrogène ou un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes ; et
en faisant réagir des composés de formule (XIV), où R₁, R₂ et Y sont comme décrits ci-dessus pour la formule (Ig), avec de l'anhydride trifluoroacétique dans un solvant pour obtenir des composés de formule (XV) : 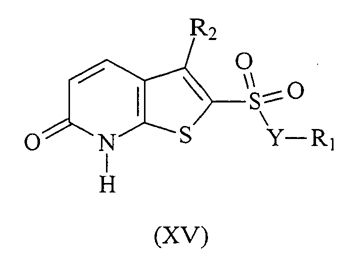 dans laquelle R₁, R₂ et Y sont comme décrits ci-dessus pour la formule (Ig) ; et en faisant réagir des composés de formule (XV) (où R₁, R₂ et Y sont comme décrits ci-dessus pour la formule (Ig),
a. avec de l'oxybromure de phosphore (III) pour obtenir des composés de formule (Ig), où Z est un groupe bromo, X, Y, n, R₁ et R₂ sont comme décrits ci-dessus pour la formule (Ig), et facultativement en formant ensuite des sels et/ou hydrates et/ou solvates des dérivés bromo obtenus de formule (Ig) ; ou
(i) en faisant réagir les dérivés bromo de formule (Ig), où Z est un groupe bromo ; X, Y, n, R₁ et R₂ sont comme décrits ci-dessus pour la formule (Ig) avec du fluorure de potassium dans du DMSO pour obtenir des composés de formule (Ig), où Z est un groupe fluoro, X, Y, n, R₁ et R₂ sont comme décrits ci-dessus pour la formule (Ig), et facultativement en formant ensuite des sels et/ou hydrates et/ou solvates des dérivés fluoro obtenus de formule (Ig) ; ou
b. avec de l'oxychlorure de phosphore (III) pour obtenir des composés de formule (Ig), où Z est un groupe chloro, X, Y, n, R₁ et R₂ sont comme décrits ci-dessus pour la formule (Ig), et facultativement en formant ensuite des sels et/ou hydrates et/ou solvates des dérivés chloro obtenus de formule (Ig) ; ou
(i) en faisant réagir les dérivés chloro de formule (Ig), où Z est un groupe chloro ; X, Y, n, R₁ et R₂ sont comme décrits ci-dessus pour la formule (Ig) avec le fluorure de potassium dans du DMSO pour obtenir des composés de formule (Ig), où Z est un groupe fluoro, X, Y, n, R₁ et R₂ sont comme décrits ci-dessus pour la formule (Ig), et facultativement en formant ensuite des sels et/ou hydrates et/ou solvates des dérivés fluoro obtenus de formule (Ig) ; ou
c. avec de l'iodométhane en présence de carbonate d'argent dans un solvant pour obtenir des composés de formule (Ig), où Z est un groupe méthoxy, X, Y, n, R₁ et R₂ sont comme décrits ci-dessus pour la formule (Ig), et facultativement en formant ensuite des sels et/ou hydrates et/ou solvates des dérivés méthoxy obtenus de formule (Ig) ; ou
d. avec de l'anhydride trifluorométhanesulfonique dans un solvant puis avec de l'ammoniac ou des mono- et dialkylamines pour obtenir des composés de formule (Ig), où Z est respectivement un groupe amino ou monoalkylamino ou dialkylamino, X, Y, n, R₁ et R₂ sont comme décrits ci-dessus pour la formule (Ig), et facultativement en formant ensuite des sels et/ou hydrates et/ou solvates des dérivés méthoxy obtenus de formule (Ig).

11. Procédé de préparation d'un composé de formule (Ih) : 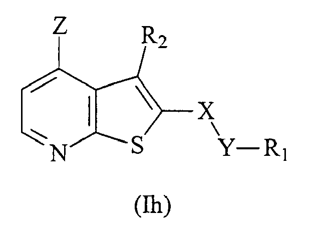 dans laquelle
X représente SO₂ ;
Y représente un groupe choisi parmi (CH₂)ₙ, NH, NHCH₂ ;
n est un entier de 0 à 1 ;
Z est un groupe amino, hydroxy, bromo, chloro, fluoro, méthoxy, mono- ou dialkyamino ;
R₁ est un groupe alkyle, cycloalkyle, phényle, biphényle, hétérocyclyle facultativement substitué ;
R₂ est un groupe phényle, hétérocyclyle facultativement substitué, ou
un groupe NR₃R₄ où R₃ et R₄ sont indépendamment choisis dans le groupe consistant en un atome d'hydrogène ou un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes,
en faisant réagir un composé de formule (XIV) : 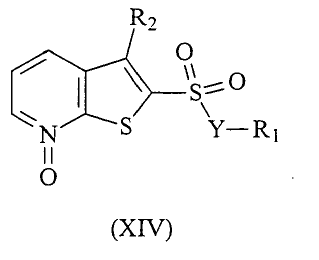 dans laquelle R₁, R₂ et Y sont comme décrits ci-dessus pour la formule (Ih),
a. avec de l'oxychlorure de phosphore (III) ou du pentachlorure de phosphore pour obtenir des composés de formule (Ih), où Z est un groupe chloro, X, Y, n, R₁ et R₂ sont comme décrits ci-dessus pour la formule (Ih), ou
b. avec de l'oxybromure de phosphore (III) pour obtenir des composés de formule (Ih), où Z est un groupe chloro, X, Y, n, R₁ et R₂ sont comme décrits ci-dessus pour la formule (Ih), et facultativement en formant ensuite des sels et/ou hydrates et/ou solvates des dérivés chloro ou bromo obtenus de formule (Ih), où Z est un groupe chloro ou bromo ; X, Y, n, R₁ et R₂ sont comme décrits ci-dessus pour la formule (Ih) ; ou
(i) en faisant réagir les dérivés chloro ou bromo de formule (Ih), où Z est un groupe chloro ou bromo ; X, Y, n, R₁ et R₂ sont comme décrits ci-dessus pour la formule (Ih) avec du fluorure de potassium dans du DMSO pour obtenir des composés de formule (Ih), où Z est un groupe fluoro, X, Y, n, R₁ et R₂ sont comme décrits ci-dessus pour la formule (Ih), et facultativement en formant ensuite des sels et/ou hydrates et/ou solvates des dérivés fluoro obtenus de formule (Ih) ; ou
(ii) en faisant réagir les dérivés chloro ou bromo de formule (Ih), où Z est un groupe chloro ou bromo ; X, Y, n, R₁ et R₂ sont comme décrits ci-dessus pour la formule (Ih) avec du méthylate de sodium dans du méthanol pour obtenir des composés de formule (Ih), où Z est un groupe méthoxy, X, Y, n, R₁ et R₂ sont comme décrits ci-dessus pour la formule (Ih), et facultativement en formant ensuite des sels et/ou hydrates et/ou solvates des dérivés méthoxy obtenus de formule (Ih), ou
(iii) en faisant réagir les dérivés chloro ou bromo de formule (Ih), où Z est un groupe chloro ou bromo ; X, Y, n, R₁ et R₂ sont comme décrits ci-dessus pour la formule (Ih) avec de l'acétate de sodium dans de l'acide acétique pour obtenir des composés de formule (Ih), où Z est un groupe hydroxy, X, Y, n, R₁ et R₂ sont comme décrits ci-dessus pour la formule (Ih), et facultativement en formant ensuite des sels et/ou hydrates et/ou solvates des dérivés d'hydroxy obtenus de formule (Ih), ou
(iv) en faisant réagir les dérivés chloro ou bromo de formule (Ih), où Z est un groupe chloro ou bromo ; X, Y, n, R₁ et R₂ sont comme décrits ci-dessus pour la formule (Ih) avec de l'anhydride trifluorométhanesulfonique dans un solvant puis avec de l'ammoniac ou des mono- et dialkylamines pour obtenir des composés de formule (Ih), où Z est respectivement un groupe amino ou monoalkylamino ou dialkylamino, X, Y, n, R₁ et R₂ sont comme décrits ci-dessus pour la formule (Ih), et facultativement en formant ensuite des sels et/ou hydrates et/ou solvates des dérivés méthoxy obtenus de formule (Ih), où Z est respectivement un groupe amino ou monoalkylamino ou dialkylamino ; ou
(v) en faisant réagir les dérivés chloro ou bromo de formule (Ih), où Z est un groupe chloro ou bromo ; X, Y, n, R₁ et R₂ sont comme décrits ci-dessus pour la formule (Ih)) avec de l'azoture de sodium dans du DMF et de l'eau pour obtenir des composés de formule (Ih), où Z est un groupe azido, X, Y, n, R₁ et R₂ sont comme décrits ci-dessus pour la formule (Ih), puis en faisant réagir les dérivés azido obtenus avec du borohydrure de sodium dans un solvant pour obtenir des composés de formule (Ih), où Z est un groupe amino, X, Y, n, R₁ et R₂ sont comme décrits ci-dessus pour la formule (Ih), et facultativement en formant ensuite des sels et/ou hydrates et/ou solvates des dérivés amino obtenus de formule (Ih).

12. Formulation pharmaceutique comprenant une quantité thérapeutiquement efficace d'un composé de formule (I) : 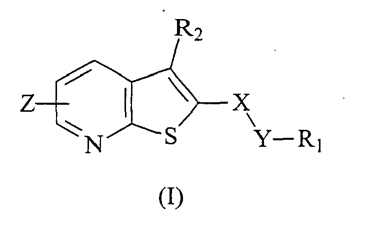 dans laquelle
X représente un groupe choisi parmi SO, SO₂ ;
Y représente un groupe choisi parmi (CH₂)ₙ, NH, NHCH₂ ;
n est un entier de 0 à 1 ;
Z est H ou monosubstitué par un groupe alkyle, nitro, un atome d'halogène, un groupe alcoxy, trifluorométhyle, cyano, amino, alkylamino, dialkylamino, aminométhyle, alkylaminométhyle, dialkylaminométhyle, hydroxyle, alkylsulfonylamino ;
R₁ est un groupe alkyle, cycloalkyle, phényle, biphényle, hétérocyclyle facultativement substitué ;
R₂ est un groupe phényle, hétérocyclyle facultativement substitué, ou
un groupe NR₃R₄ où R₃ et R₄ sont indépendamment choisis dans le groupe consistant en un atome d'hydrogène et un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes, ou
un groupe NH-CO-NR₅R₆, où R₅ et R₆ sont indépendamment choisis dans le groupe consistant en un atome d'hydrogène et un groupe alkyle facultativement substitué, ou bien R₅ et R₆ conjointement avec l'atome N auquel ils sont attachés forment un groupe hétérocyclyle en C_{5 à 7} facultativement substitué, contenant un ou plusieurs hétéroatomes ; et/ou des hydrates et/ou solvates et/ou sels pharmaceutiquement acceptables de ceux-ci formés avec des acides ou des bases en association avec un ou plusieurs diluants, excipients et/ou supports inertes physiologiquement acceptables.

13. Composition pharmaceutique selon la revendication 12, à utiliser dans la prévention et/ou le traitement de troubles véhiculés par les récepteurs mGluR1 et mGluR5.

14. Utilisation d'un composé de formule (I) : 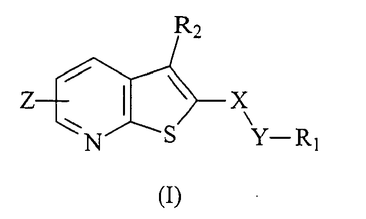 dans laquelle
X représente un groupe choisi parmi SO, SO₂ ;
Y représente un groupe choisi parmi (CH₂)ₙ, NH, NHCH₂ ;
n est un entier de 0 à 1 ;
Z est H ou monosubstitué par un groupe alkyle, nitro, un atome d'halogène, un groupe alcoxy, trifluorométhyle, cyano, amino, alkylamino, dialkylamino, aminométhyle, alkylaminométhyle, dialkylaminométhyle, hydroxyle, alkylsulfonylamino ;
R₁ est un groupe alkyle, cycloalkyle, phényle, biphényle, hétérocyclyle facultativement substitué ;
R₂ est un groupe phényle, hétérocyclyle facultativement substitué, ou
un groupe NR₃R₄ où R₃ et R₄ sont indépendamment choisis dans le groupe consistant en un atome d'hydrogène et un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes, ou
un groupe NH-CO-NR₅R₆, où R₅ et R₆ sont indépendamment choisis dans le groupe consistant en un atome d'hydrogène et un groupe alkyle facultativement substitué, ou bien R₅ et R₆ conjointement avec l'atome N auquel ils sont attachés forment un groupe hétérocyclyle en C_{5 à 7} facultativement substitué, contenant un ou plusieurs hétéroatomes ; et/ou des hydrates et/ou solvates et/ou sels pharmaceutiquement acceptables de celui-ci formés avec des acides ou des bases, pour la fabrication d'un médicament destiné au traitement et/ou à la prévention de troubles véhiculés par mGluR1 et mGluR5.

15. Utilisation d'un composé selon la revendication 14, dans laquelle lesdits troubles véhiculés par les récepteurs mGluR1 et mGluR5 sont des troubles psychiatriques.

16. Utilisation d'un composé selon la revendication 14, dans laquelle lesdits troubles véhiculés par les récepteurs mGluR1 et mGluR5 sont des troubles neurologiques.

17. Utilisation d'un composé selon la revendication 14, dans laquelle lesdits troubles véhiculés par les récepteurs mGluR1 et mGluR5 sont une douleur chronique et aigue.

18. Utilisation d'un composé selon la revendication 14, dans laquelle lesdits troubles véhiculés par les récepteurs mGluR1 et mGluR5 sont un dysfonctionnement neuromusculaire du tractus urinaire bas et des troubles gastro-intestinaux.

19. Composé de formule (I) : 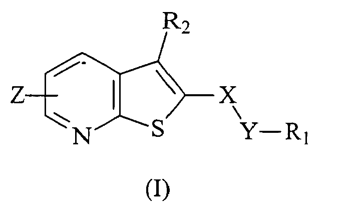 dans laquelle
X représente un groupe choisi parmi SO, SO₂ ;
Y représente un groupe choisi parmi (CH₂)ₙ, NH, NHCH₂ ;
n est un entier de 0 à 1 ;
Z est H ou monosubstitué par un groupe alkyle, nitro, un atome d'halogène, un groupe alcoxy, trifluorométhyle, cyano, amino, alkylamino, dialkylamino, aminométhyle, alkylaminométhyle, dialkylaminométhyle, hydroxyle, alkylsulfonylamino ;
R₁ est un groupe alkyle, cycloalkyle, phényle, biphényle, hétérocyclyle facultativement substitué ;
R₂ est un groupe phényle, hétérocyclyle facultativement substitué, ou
un groupe NR₃R₄ où R₃ et R₄ sont indépendamment choisis dans le groupe consistant en un atome d'hydrogène et un groupe alkyle facultativement substitué, ou bien R₃ et R₄ conjointement avec l'atome N auquel ils sont attachés forment un groupe hétérocyclyle en C_{5 à 7} facultativement substitué, contenant un ou plusieurs hétéroatomes, ou
un groupe NH-CO-NR₅R₆, où R₅ et R₆ sont indépendamment choisis dans le groupe consistant en un atome d'hydrogène et un groupe alkyle facultativement substitué, ou bien R₅ et R₆ conjointement avec l'atome N auquel ils sont attachés forment un groupe hétérocyclyle en C_{5 à 7} facultativement substitué, contenant un ou plusieurs hétéroatomes ; et/ou des hydrates et/ou solvates et/ou sels pharmaceutiquement acceptables de celui-ci formés avec des acides ou des bases,
à utiliser dans un procédé de prévention et/ou de traitement de troubles véhiculés par les récepteurs mGluR1 et mGluR5.

20. Composé selon la revendication 19, dans lequel lesdits troubles véhiculés par les récepteurs mGluR1 et mGluR5 sont des troubles psychiatriques.

21. Composé selon la revendication 19, dans lequel lesdits troubles véhiculés par les récepteurs mGluR1 et mGluR5 sont des troubles neurologiques.

22. Composé selon la revendication 19, dans lequel lesdits troubles véhiculés par les récepteurs mGluR1 et mGluR5 sont des troubles liés à une douleur chronique et aigue.

23. Composé selon la revendication 19, dans lequel lesdits troubles véhiculés par les récepteurs mGluR1 et mGluR5 sont un dysfonctionnement neuromusculaire du tractus urinaire bas et des troubles gastro-intestinaux.
